# EUROPEAN PATENT APPLICATION

(11) **EP 3 698 776 A1**
(43) Date of publication of application: **26.08.2020**
(21) Application number: 19157987.9
(22) Date of filing: 19.02.2019
(51) Int. Cl.: A61K 9/14, A61K 9/20, A61K 31/00

(54) **TAMPER-RESISTANT DOSAGE FORM WITH IMMEDIATE RELEASE AND RESISTANCE AGAINST SOLVENT EXTRACTION**

(71) Applicant: Grünenthal GmbH, 52078 Aachen (DE)
(72) Inventor: IMMOHR, Isabell, 41352 Korschenbroich (DE); WENING. Klaus, 41470 Neuss (DE)
(74) Representative: Kutzenberger Wolff & Partner

(57) **Abstract**

The invention relates to a tamper-resistant pharmaceutical dosage form comprising a multitude of particles which comprise a pharmacologically active compound, preferably selected from opioids; a polyalkylene oxide having a weight average molecular weight within the range of from 200,000 g/mol to 2,000,000 g/mol, and a disintegrant; wherein the pharmacologically active compound is dispersed in a matrix comprising the polyalkylene oxide and the disintegrant; wherein the content of the disintegrant is more than 20 wt.-%, based on the total weight of the particles; wherein the content of the polyalkylene oxide is at least 25 wt.-%, based on the total weight of the particles; and wherein the dosage form provides under *in vitro* conditions immediate release of the pharmacologically active compound in accordance with Ph. Eur.

## Description

The invention relates to a tamper-resistant pharmaceutical dosage form comprising a multitude of particles which comprise a pharmacologically active compound, preferably an opioid; a polyalkylene oxide having a weight average molecular weight within the range of from 200,000 g/mol to 2,000,000 g/mol; and a disintegrant; wherein the pharmacologically active compound is dispersed in a matrix comprising the polyalkylene oxide and the disintegrant; wherein the content of the disintegrant is more than 20 wt.-%, based on the total weight of the particles; wherein the content of the polyalkylene oxide is at least 25 wt.-%, based on the total weight of the particles; and wherein the dosage form provides under *in vitro* conditions immediate release of the pharmacologically active compound in accordance with Ph. Eur.

A large number of pharmacologically active substances have a potential for being abused or misused, i.e. they can be used to produce effects which are not consistent with their intended use. Thus, e.g. opioids which exhibit an excellent efficacy in controlling severe to extremely severe pain, are frequently abused to induce euphoric states similar to being intoxicated. In particular, active substances which have a psychotropic effect are abused accordingly.

To enable abuse, the corresponding dosage forms, such as tablets or capsules are crushed, for example ground by the abuser, the active substance is extracted from the thus obtained powder using a preferably aqueous liquid and after being optionally filtered through cotton wool or cellulose wadding, the resultant solution is administered parenterally, in particular intravenously. This type of dosage results in an even faster diffusion of the active substance compared to the oral abuse, with the result desired by the abuser, namely the kick. This kick or these intoxication-like, euphoric states are also reached if the powdered dosage form is administered nasally, i.e. is sniffed.

Various concepts for the avoidance of drug abuse have been developed.

It has been proposed to incorporate in dosage forms aversive agents and/or antagonists in a manner so that they only produce their aversive and/or antagonizing effects when the dosage forms are tampered with. However, the presence of such aversive agents is principally not desirable and there is a need to provide sufficient tamper-resistance without relying on aversive agents and/or antagonists.

Another concept to prevent abuse relies on the mechanical properties of the pharmaceutical dosage forms, particularly an increased breaking strength (resistance to crushing). The major advantage of such pharmaceutical dosage forms is that comminuting, particularly pulverization, by conventional means, such as grinding in a mortar or fracturing by means of a hammer, is impossible or at least substantially impeded. Thus, the pulverization, necessary for abuse, of the dosage forms by the means usually available to a potential abuser is prevented or at least complicated.

Such pharmaceutical dosage forms are useful for avoiding drug abuse of the pharmacologically active compound contained therein, as they may not be powdered by conventional means and thus, cannot be administered in powdered form, e.g. nasally. The mechanical properties, particularly the high breaking strength of these pharmaceutical dosage forms renders them tamper-resistant. In the context of such tamper-resistant pharmaceutical dosage forms it can be referred to, e.g., WO 2005/016313, WO 2005/016314, WO 2005/ 063214, WO 2005/102286, WO 2006/002883, WO 2006/002884, WO 2006/002886, WO 2006/082097, WO 2006/082099, and WO2009/092601.

WO 2018/024709 relates to a pharmaceutical dosage form having a breaking strength of at least 300 N, said dosage form comprising an opioid (A) selected from the group consisting of Oxymorphone, Oxycodone, Tapentadol, Hydromorphone, Hydrocodone, Morphine, and the physiologically acceptable salts thereof; wherein the weight content of the opioid (A) is within the range of from 5.0 to 35 wt.-%, based on the total weight of the pharmaceutical dosage form; an anionic polysaccharide (B) selected from the group consisting of croscarmellose, carmellose, crosslinked carboxymethyl starch, carboxymethyl starch, and the physiologically acceptable salts thereof; wherein the weight content of the anionic polysaccharide (B) is within the range of from 5.0 to 35 wt.-%, based on the total weight of the pharmaceutical dosage form; and a polyalkylene oxide (C) having a weight average molecular weight of at least 200,000 g/mol; wherein the weight content of the polyalkylene oxide (C) is within the range of from 20 to 80 wt.-%, based on the total weight of the pharmaceutical dosage form; wherein the opioid (A) is present in a controlled-release matrix comprising the anionic polysaccharide (B) and the polyalkylene oxide (C).

These dosage forms secured against abuse are distinguished by a controlled, preferably retarded release of the active substance which has abuse potential. However, a rapid release of the active substance is necessary for numerous therapeutic applications, for example pain relief using active substances with abuse potential.

WO 2008/033523 discloses a pharmaceutical composition that may include a granulate which may at least include one active pharmaceutical ingredient susceptible to abuse. The particle contains both an alcohol soluble and alcohol insoluble and at least partially water soluble material. Both materials are granulated in the presence of alcohol and water. The granulate may also include a coating on the granulate exhibiting crush resistance. Material deposition on the granule is performed using an alcohol based solvent.

WO 2008/107149 (US 2009/004267) discloses multiparticulate dosage forms with impeded abuse containing, one or more active substances having abuse potential, at least one synthetic or natural polymer, and at least one disintegrant, with the individual particles of the pharmaceutical dosage form having a breaking strength of at least 500 N and a release of the active substance of at least 75% after 45 minutes. The exemplified capsules provide rapid release of the pharmacologically active compound. The disintegrant is preferably not contained in the particles. When it is contained in the particles, its content is rather low. The reference does not contain any information that besides its disintegrating effect a disintegrant may have any beneficial effect with respect to tamper-resistance such as resistance against solvent extraction.

WO 2010/140007 discloses dosage forms comprising melt extruded particles comprising a drug, wherein said melt extruded particles are present as a discontinuous phase in a matrix. The dosage forms provide prolonged release of the drug.

WO 2013/017242 and WO 2013/017234 disclose a tamper-resistant tablet comprising a matrix material in an amount of more than one third of the total weight of the tablet; and a plurality of particles in an amount of less than two thirds of the total weight of the tablet; wherein said particles comprise a pharmacologically active compound and a polyalkylene oxide; and form a discontinuous phase within the matrix material. The matrix material may comprise a disintegrant. The reference does not contain any information that besides its disintegrating effect a disintegrant may have any beneficial effect with respect to tamper-resistance such as resistance against solvent extraction.

WO 2014/190440 relates to an immediate release orally administrable abuse-deterrent pharmaceutical formulation comprising: at least one pharmaceutically active ingredient susceptible to abuse; at least one gelling polymeric compound selected from the group consisting of: polysaccharides, sugars, sugar derived alcohols, starches, starch derivatives, cellulose derivatives, Carrageenan, pectin, sodium alginate, gellan gum, xanthan gum, poloxamer, carbopol, polyox, povidone, hydroxypropyl methylcellulose, hypermellose, and combinations thereof; at least one disintegrant and optionally at least one surfactant, wherein said formulation exhibit properties related to deterring the abuse, via injection or nasal inhalation when being tampered and exposed to aqueous, alcoholic, acidic and basic media.

WO 2016/170097 relates to a tamper-resistant pharmaceutical dosage form comprising a multitude of particles which comprise a pharmacologically active compound, a polyalkylene oxide, and a disintegrant; wherein the pharmacologically active compound is dispersed in a matrix comprising the polyalkylene oxide and the disintegrant; wherein the content of the disintegrant is more than 5.0 wt.-%, based on the total weight of the pharmaceutical dosage form and/or based on the total weight of the particles; wherein the content of the polyalkylene oxide is at least 25 wt.-%, based on the total weight of the pharmaceutical dosage form and/or based on the total weight of the particles; and wherein the dosage form provides under in vitroconditions immediate release of the pharmacologically active compound in accordance with Ph. Eur.

US 2010/0092553 discloses solid multiparticle oral pharmaceutical forms whose composition and structure make it possible to avoid misuse. The microparticles have an extremely thick coating layer which assures the modified release of the drug and simultaneously imparts crushing resistance to the coated microparticles so as to avoid misuse.

The properties of these tamper-resistant dosage forms, however, are not satisfactory in every respect.

Due to the increased breaking strength of some commercially available tamper-resistant dosage forms there has been a shift in the route of abuse from nasal to injection. Injection abuse, however, has been associated with cases of thrombotic microangiopathy (see R. Hunt et al., Blood, 2017, 129(7), 896-905).

There is a demand for tamper-resistant dosage forms that possess crush resistance and release the pharmacologically active compound as quick as possible (immediate release), i.e. should show a gradual increase reaching 85% to 100% at 30 to 45 minutes or earlier.

The pharmacokinetic properties of a suitable tamper-resistant dosage form, such as tₘₐₓ, should essentially correspond to those of commercially available dosage forms of the same pharmacologically active compound with the same release characteristics (immediate release). This is important because subjects used to take these conventional dosage forms expect to achieve the desired therapeutic effect (e.g. pain relief) within a certain time window after administration. When a subject changes medication from these conventional dosage forms with early tₘₐₓ to a modified dosage form (e.g. a tamper resistant dosage form) with a later tₘₐₓ, due to the absence of early therapeutic effect (e.g. pain relief), the subject may erroneously conclude that the administered dose was too low, leading the patient to take multiple modified dosage forms at once. If patients who do not see immediate relief take additional doses, there are clear safety concerns as the resultant unintended overdose may cause significant harm or even death to the subject.

Likewise, it would be desirable for a suitable tamper-resistant dosage form to show no clinically relevant food effect, thus to fulfill these pharmacokinetic properties independent of the stomach condition (fed or fasted state).

Further, when trying to tamper the dosage form in order to prepare a formulation suitable for abuse by intravenous administration, the liquid part of the formulation that can be separated from the remainder by means of a syringe should be as less as possible, e.g. should contain no more than 10 wt.-% of the pharmacologically active compound originally contained in the dosage form. This should impede both routes of abuse nasal and injection. Further, even if for some reasons an abuser should be capable of rendering the tamper-resistant dosage form or a part thereof into a form suitable for intravenous abuse, the galenic formulation should not cause additional significant harm to the abuser beyond the effect of the pharmacologically active compound being abused.

It is an object according to the invention to provide tamper-resistant pharmaceutical dosage forms that provide rapid release of the pharmacologically active compound and that have advantages compared to the tamper-resistant pharmaceutical dosage forms of the prior art.

This object has been achieved by the patent claims.

The invention relates to a tamper-resistant pharmaceutical dosage form, preferably for oral administration, comprising a multitude of particles which comprise a pharmacologically active compound, preferably an opioid; a polyalkylene oxide having a weight average molecular weight within the range of from 200,000 g/mol to 2,000,000 g/mol; and a disintegrant; wherein the pharmacologically active compound is dispersed in a matrix comprising the polyalkylene oxide and the disintegrant; wherein the content of the disintegrant is more than 20 wt.-%, based on the total weight of the particles; wherein the content of the polyalkylene oxide is at least 25 wt.-%, based on the total weight of the particles; and wherein the dosage form provides under *in vitro* conditions immediate release of the pharmacologically active compound in accordance with Ph. Eur.

The resistance against mechanical disruption of some known tamper-resistant dosage forms with immediate release of the pharmacologically active compounds contained therein essentially relies upon the presence of high molecular weight polyethylene oxide, whereas the resistance against solvent extraction essentially relies upon the presence of certain disintegrants in amounts of up to 20 wt.-%. Higher contents of disintegrants have not been suggested *inter alia* for reasons of processability.

It has now been surprisingly found that tamper-resistant dosage forms with immediate release of the pharmacologically active compounds contained therein can be provided that on the one hand still exhibit excellent resistance against mechanical disruption thereby avoiding nasal abuse, and on the other hand exhibit improved resistance against solvent extraction thereby also avoiding abuse by injection.

In particular, it has now been surprisingly found that the molecular weight of the polyalkylene oxide may be reduced without significantly deteriorating the resistance against mechanical disruption thereby still making nasal abuse difficult if not impossible, whereas under these conditions the content of disintegrants may be further increased to unusually high amounts significantly improving the resistance against solvent extraction thereby making abuse by injection more difficult if not impossible.

Furthermore, it has been surprisingly found that certain particle shapes have significant advantages when the particles are blended with suitable matrix materials and are compressed into tablets.
Figure 1 illustrates the behavior of the particles contained in the pharmaceutical dosage form according to the invention when being subjected to a breaking strength test, in particular their deformability.
Figure 2 illustrates the behavior of conventional particles when being subjected to a breaking strength test.
Figure 3 shows the trial design of the human abuse liability study according to Inventive Example 3.
Figure 4 shows the results of the primary measure of the human abuse liability study according to Inventive Example 3.
Figure 5 illustrates further subjective parameters of the human abuse liability study according to Inventive Example 3.
Figure 6 shows the objective PD measure - pulpillometry, mean pupil diameter of the human abuse liability study according to Inventive Example 3.
Figure 7 shows the pharmacokinetic results of the human abuse liability study according to Inventive Example 3.
Figure 8 shows the results of solvent extraction tests according to Example 4.

As used herein, the term "pharmaceutical dosage form" refers to a pharmaceutical entity comprising a pharmacologically active compound, preferably opioid, and which is actually administered to, or taken by, a patient, preferably orally.

Preferably, the pharmaceutical dosage from according to the invention is a capsule or a tablet. The particles that are contained in the pharmaceutical dosage form and/or the pharmaceutical dosage form as such may be film-coated.

The pharmaceutical dosage form may be compressed or molded in its manufacture, and it may be of almost any size, shape, weight, and color. Most pharmaceutical dosage forms are intended to be swallowed as a whole and accordingly, preferred pharmaceutical dosage forms according to the invention are designed for oral administration. However, alternatively pharmaceutical dosage forms may be dissolved in the mouth, chewed, or dissolved or dispersed in liquid or meal before swallowing, and some may be placed in a body cavity. Thus, the pharmaceutical dosage form according to the invention may alternatively be adapted for buccal, lingual, rectal or vaginal administration. Implants are also possible.

In a preferred embodiment, the pharmaceutical dosage form according to the invention preferably can be regarded as a MUPS formulation (multiple unit pellet system). In a preferred embodiment, the pharmaceutical dosage form according to the invention is monolithic. In another preferred embodiment, the pharmaceutical dosage form according to the invention is not monolithic. In this regard, monolithic preferably means that the pharmaceutical dosage form is formed or composed of material without joints or seams or consists of or constitutes a single unit.

In a preferred embodiment, the pharmaceutical dosage form according to the invention contains all ingredients in a dense compact unit which in comparison to capsules has a comparatively high density. In another preferred embodiment, the pharmaceutical dosage form according to the invention contains all ingredients in a capsule which in comparison to dense compact unit has a comparatively low density.

An advantage of the pharmaceutical dosage forms according to the invention is that the same particles may be mixed with excipients in different amounts to thereby produce pharmaceutical dosage forms of different strengths. Another advantage of the pharmaceutical dosage forms according to the invention is that the different particles may be mixed with one another to thereby produce pharmaceutical dosage forms of different properties, e.g. different release rates, different pharmacologically active ingredients, and the like.

The pharmaceutical dosage form according to the invention has preferably a total weight in the range of 0.01 to 1.5 g, more preferably in the range of 0.05 to 1.2 g, still more preferably in the range of 0.1 g to 1.0 g, yet more preferably in the range of 0.2 g to 0.9 g, and most preferably in the range of 0.3 g to 0.8 g. In a preferred embodiment, the total weight of the pharmaceutical dosage form is within the range of 500±450 mg, more preferably 500±300 mg, still more preferably 500±200 mg, yet more preferably 500±150 mg, most preferably 500±100 mg, and in particular 500±50 mg. In another preferred embodiment, the total weight of the pharmaceutical dosage form is within the range of 600±450 mg, more preferably 600±300 mg, still more preferably 600±200 mg, yet more preferably 600±150 mg, most preferably 600±100 mg, and in particular 600±50 mg. In still another preferred embodiment, the total weight of the pharmaceutical dosage form is within the range of 700±450 mg, more preferably 700±300 mg, still more preferably 700±200 mg, yet more preferably 700±150 mg, most preferably 700±100 mg, and in particular 700±50 mg. In yet another preferred embodiment, the total weight of the pharmaceutical dosage form is within the range of 800±450 mg, more preferably 800±300 mg, still more preferably 800±200 mg, yet more preferably 800±150 mg, most preferably 800±100 mg, and in particular 800±50 mg.

In a preferred embodiment, the pharmaceutical dosage form according to the invention is a round pharmaceutical dosage form, preferably having a diameter of e.g. 11 mm or 13 mm. Pharmaceutical dosage forms of this embodiment preferably have a diameter in the range of 1 mm to 30 mm, in particular in the range of 2 mm to 25 mm, more in particular 5 mm to 23 mm, even more in particular 7 mm to 13 mm; and a thickness in the range of 1.0 mm to 12 mm, in particular in the range of 2.0 mm to 10 mm, even more in particular from 3.0 mm to 9.0 mm, even further in particular from 4.0 mm to 8.0 mm.

In another preferred embodiment, the pharmaceutical dosage form according to the invention is an oblong pharmaceutical dosage form, preferably having a length of e.g. 17 mm and a width of e.g. 7 mm. In preferred embodiments, the pharmaceutical dosage form according to the invention has a length of e.g. 22 mm and a width of e.g. 7 mm; or a length of 23 mm and a width of 7 mm; whereas these embodiments are particularly preferred for capsules. Pharmaceutical dosage forms of this embodiment preferably have a lengthwise extension (longitudinal extension) of 1 mm to 30 mm, in particular in the range of 2 mm to 25 mm, more in particular 5 mm to 23 mm, even more in particular 7 mm to 20 mm; a width in the range of 1 mm to 30 mm, in particular in the range of 2 mm to 25 mm, more in particular 5 mm to 23 mm, even more in particular 7 mm to 13 mm; and a thickness in the range of 1.0 mm to 12 mm, in particular in the range of 2.0 mm to 10 mm, even more in particular from 3.0 mm to 9.0 mm, even further in particular from 4.0 mm to 8.0 mm.

The pharmaceutical dosage forms according to the invention can optionally be provided, partially or completely, with a conventional coating. The pharmaceutical dosage forms according to the invention are preferably film coated with conventional film coating compositions. Suitable coating materials are commercially available, e.g. under the trademarks Opadry® and Eudragit®.

Examples of suitable materials include cellulose esters and cellulose ethers, such as methylcellulose (MC), hydroxypropylmethylcellulose (HPMC), hydroxypropylcellulose (HPC), hydroxyethylcellulose (HEC), sodium carboxymethylcellulose (Na-CMC), poly(meth)acrylates, such as aminoalkylmethacrylate copolymers, methacrylic acid methylmethacrylate copolymers, methacrylic acid methylmethacrylate copolymers; vinyl polymers, such as polyvinylpyrrolidone, polyvinyl alcohol, polyvinylacetate; and natural film formers.

In a particularly preferred embodiment, the coating is water-soluble. In a preferred embodiment, the coating is based on polyvinyl alcohol, such as polyvinyl alcohol-partially hydrolyzed, and may additionally contain polyethylene glycol, such as macrogol 3350, and/or pigments. In another preferred embodiment, the coating is based on hydroxypropylmethylcellulose, preferably hypromellose type 2910 having a viscosity of 3 to 15 mPas.

The coating can be resistant to gastric juices and dissolve as a function of the pH value of the release environment. By means of this coating, it is possible to ensure that the pharmaceutical dosage form according to the invention passes through the stomach undissolved and the active compound is only released in the intestines. The coating which is resistant to gastric juices preferably dissolves at a pH value of between 5 and 7.5.

The coating can also be applied e.g. to improve the aesthetic impression and/or the taste of the pharmaceutical dosage forms and the ease with which they can be swallowed. Coating the pharmaceutical dosage forms according to the invention can also serve other purposes, e.g. improving stability and shelf-life. Suitable coating formulations comprise a film forming polymer such as, for example, polyvinyl alcohol or hydroxypropyl methylcellulose, e.g. hypromellose, a plasticizer such as, for example, a glycol, e.g. propylene glycol or polyethylene glycol, an opacifier, such as, for example, titanium dioxide, and a film smoothener, such as, for example, talc. Suitable coating solvents are water as well as organic solvents. Examples of organic solvents are alcohols, e.g. ethanol or isopropanol, ketones, e.g. acetone, or halogenated hydrocarbons, e.g. methylene chloride. Coated pharmaceutical dosage forms according to the invention are preferably prepared by first making the cores and subsequently coating said cores using conventional techniques, such as coating in a coating pan.

The pharmaceutical dosage form according to the invention contains a plurality of particles. The particles comprise a pharmacologically active compound, preferably an opioid; a polyalkylene oxide having a weight average molecular weight within the range of from 200,000 g/mol to 2,000,000 g/mol; and a disintegrant. The pharmacologically active compound is dispersed in the polyalkylene oxide and the disintegrant.

Preferably, the particles are hot-melt extruded.

For the purpose of the specification, the term "particle" refers to a discrete mass of material that is solid, e.g. at 20 °C or at room temperature or ambient temperature. Preferably a particle is solid at 20 °C. Preferably, the particles are monoliths. Preferably, the pharmacologically active compound and the polyalkylene oxide are intimately homogeneously distributed in the particles so that the particles do not contain any macroscopic segments where either pharmacologically active compound is present in the absence of polyalkylene oxide or where polyalkylene oxide is present in the absence of pharmacologically active compound.

When the particles are film coated, the polyalkylene oxide is preferably homogeneously distributed in the core of the pharmaceutical dosage form, i.e. the film coating preferably does not contain polyalkylene oxide, but optionally polyalkylene glycol that differs from polyalkylene oxide in its lower molecular weight. Nonetheless, the film coating as such may of course contain one or more polymers, which however, preferably differ from the polyalkylene oxide contained in the core.

The particles are of macroscopic size, typically the average diameter is within the range of from 100 µm to 1500 µm, preferably 200 µm to 1500 µm, more preferably 300 µm to 1500 µm, still more preferably 400 µm to 1500 µm, most preferably 500 µm to 1500 µm, and in particular 600 µm to 1500 µm.

Preferably, the content of the particles in the pharmaceutical dosage forms according to the invention is at most 85 wt.-%, or at most 80 wt.-%, or at most 75 wt.-%, or at most 70 wt.-%, or at most 65 wt.-%, more preferably at most 60 wt.-%, still more preferably at most 55 wt.-%, yet more preferably at most 50 wt.-%, most preferably at most 45 wt.-% and in particular at most 40 wt.-%, in each case based on the total weight of the pharmaceutical dosage form.

Preferably, the content of the particles in the pharmaceutical dosage forms according to the invention is at least 2.5 wt.-%, at least 3.0 wt.-%, at least 3.5 wt.-% or at least 4.0 wt.-%; more preferably at least 4.5 wt.-%, at least 5.0 wt.-%, at least 5.5 wt.-% or at least 6.0 wt.-%; most preferably at least 6.5 wt.-%, at least 7.0 wt.-%, at least 7.5 wt.-% or at least 8.0 wt.-%; and in particular at least 8.5 wt.-%, at least 9.0 wt.-%, at least 9.5 wt.-% or at least 10 wt.-%; or at least 15 wt.-%, or at least 20 wt.-%, or at least 25 wt.-%, or at least 30 wt.-%, or at least 35 wt.-%, or at least 40 wt.-%, or at least 45 wt.-%, or at least 50 wt.-%, or at least 55 wt.-%, in each case based on the total weight of the pharmaceutical dosage form.

In a preferred embodiment, the content of the particles in the pharmaceutical dosage forms according to the invention is within the range of 10±7.5 wt.-%, more preferably 10±5.0 wt.-%, still more preferably 10±4.0 wt.-%, yet more preferably 10±3.0 wt.-%, most preferably 10±2.0 wt.-%, and in particular 10±1.0 wt.-%, based on the total weight of the pharmaceutical dosage form.

In another preferred embodiment, the content of the particles in the pharmaceutical dosage forms according to the invention is within the range of 15±12.5 wt.-%, more preferably 15±10 wt.-%, still more preferably 15±8.0 wt.-%, yet more preferably 15±6.0 wt.-%, most preferably 15±4.0 wt.-%, and in particular 15±2.0 wt.-%, based on the total weight of the pharmaceutical dosage form.

In still another preferred embodiment, the content of the particles in the pharmaceutical dosage forms according to the invention is within the range of 20±17.5 wt.-%, more preferably 20±15 wt.-%, still more preferably 20±12.5 wt.-%, yet more preferably 20±10wt.-%, most preferably 20±7.5 wt.-%, and in particular 20±5 wt.-%, based on the total weight of the pharmaceutical dosage form.

In yet another preferred embodiment, the content of the particles in the pharmaceutical dosage forms according to the invention is within the range of 25±17.5 wt.-%, more preferably 25±15 wt.-%, still more preferably 25±12.5 wt.-%, yet more preferably 25±10 wt.-%, most preferably 25±7.5 wt.-%, and in particular 25±5 wt.-%, based on the total weight of the pharmaceutical dosage form.

In another preferred embodiment, the content of the particles in the pharmaceutical dosage forms according to the invention is within the range of 30±17.5 wt.-%, more preferably 30±15 wt.-%, still more preferably 30±12.5 wt.-%, yet more preferably 30±10 wt.-%, most preferably 30±7.5 wt.-%, and in particular 30±5 wt.-%, based on the total weight of the pharmaceutical dosage form.

In still another preferred embodiment, the content of the particles in the pharmaceutical dosage forms according to the invention is within the range of 35±17.5 wt.-%, more preferably 35±15 wt.-%, still more preferably 35±12.5 wt.-%, yet more preferably 35±10 wt.-%, most preferably 35±7.5 wt.-%, and in particular 35±5 wt.-%, based on the total weight of the pharmaceutical dosage form.

In yet another preferred embodiment, the content of the particles in the pharmaceutical dosage forms according to the invention is within the range of 40±17.5 wt.-%, more preferably 40±15 wt.-%, still more preferably 40±12.5 wt.-%, yet more preferably 40±10 wt.-%, most preferably 40±7.5 wt.-%, and in particular 40±5 wt.-%, based on the total weight of the pharmaceutical dosage form.

In another preferred embodiment, the content of the particles in the pharmaceutical dosage forms according to the invention is within the range of 45±17.5 wt.-%, more preferably 45±15 wt.-%, still more preferably 45±12.5 wt.-%, yet more preferably 45±10 wt.-%, most preferably 45±7.5 wt.-%, and in particular 45±5 wt.-%, based on the total weight of the pharmaceutical dosage form.

In yet another preferred embodiment, the content of the particles in the pharmaceutical dosage forms according to the invention is within the range of 50±17.5 wt.-%, more preferably 50±15 wt.-%, still more preferably 50±12.5 wt.-%, yet more preferably 50±10 wt.-%, most preferably 50±7.5 wt.-%, and in particular 50±5 wt.-%, based on the total weight of the pharmaceutical dosage form.

In another preferred embodiment, the content of the particles in the pharmaceutical dosage forms according to the invention is within the range of 55±17.5 wt.-%, more preferably 55±15 wt.-%, still more preferably 55±12.5 wt.-%, yet more preferably 55±10 wt.-%, most preferably 55±7.5 wt.-%, and in particular 55±5 wt.-%, based on the total weight of the pharmaceutical dosage form.

The shape of the particles is not particularly limited. As the particles are preferably manufactured by hot-melt extrusion, preferred particles present in the pharmaceutical dosage forms according to the invention are generally cylindrical in shape. The diameter of such particles is therefore the diameter of their circular cross section. The cylindrical shape is caused by the extrusion process according to which the diameter of the circular cross section is a function of the extrusion die and the length of the cylinders is a function of the cutting length according to which the extruded strand of material is cut into pieces of preferably more or less predetermined length.

The suitability of cylindrical, i.e. aspherical particles for the manufacture of the pharmaceutical dosage forms according to the invention is unexpected. Typically, the aspect ratio is regarded as an important measure of the spherical shape. The aspect ratio is defined as the ratio of the maximal diameter (diₘₐₓ) and its orthogonal Feret-diameter. For aspherical particles, the aspect ratio has values above 1. The smaller the value the more spherical is the particle. Aspect ratios below 1.1 are typically considered satisfactory, aspect ratios above 1.2, however, are typically considered not suitable for the manufacture of conventional pharmaceutical dosage forms.

The inventors have surprisingly found that when manufacturing the pharmaceutical dosage forms according to the invention, even particles having aspect ratios above 1.2 can be processed without difficulties and that it is not necessary to provide spherical particles. In a preferred embodiment, the aspect ratio of the particles is at most 1.40, more preferably at most 1.35, still more preferably at most 1.30, yet more preferably at most 1.25, even more preferably at most 1.20, most preferably at most 1.15 and in particular at most 1.10. In another preferred embodiment, the aspect ratio of the particles is at least 1.10, more preferably at least 1.15, still more preferably at least 1.20, yet more preferably at least 1.25, even more preferably at least 1.30, most preferably at least 1.35 and in particular at least 1.40.

The particles in the pharmaceutical dosage forms according to the invention are of macroscopic size, i.e. typically have an average particle size of at least 50 µm, more preferably at least 100 µm, still more preferably at least 150 µm or at least 200 µm, yet more preferably at least 250 µm or at least 300 µm, most preferably at least 400 µm or at least 500 µm, and in particular at least 550 µm or at least 600 µm.

Preferred particles have an average length and average diameter of 1000 µm or less. When the particles are manufactured by extrusion technology, the "length" of particles is the dimension of the particles that is parallel to the direction of extrusion. The "diameter" of particles is the largest dimension that is perpendicular to the direction of extrusion.

Particularly preferred particles have an average diameter of less than 1000 µm, more preferably less than 800 µm, still more preferably of less than 650 µm. Especially preferred particles have an average diameter of less than 700 µm, particularly less than 600 µm, still more particularly less than 500 µm, e.g. less than 400 µm. Particularly preferred particles have an average diameter in the range 200 to 1000 µm, more preferably 400 to 800 µm, still more preferably 450 to 700 µm, yet more preferably 500 to 650 µm, e.g. 500 to 600 µm. Further preferred particles have an average diameter of between 300 µm and 400 µm, of between 400 µm and 500 µm, or of between 500 µm and 600 µm, or of between 600 µm and 700 µm or of between 700 µm and 800 µm.

Preferred particles that are present in the pharmaceutical dosage forms according to the invention have an average length of less than 1000 µm, preferably an average length of less than 800 µm, still more preferably an average length of less than 650 µm, e.g. a length of 800 µm, 700 µm 600 µm, 500 µm, 400 µm or 300 µm. Especially preferred particles have an average length of less than 700 µm, particularly less than 650 µm, still more particularly less than 550 µm, e.g. less than 450 µm. Particularly preferred particles therefore have an average length in the range 200-1000 µm, more preferably 400-800 µm, still more preferably 450-700 µm, yet more preferably 500-650 µm, e.g. 500-600 µm. The minimum average length of the microparticles is determined by the cutting step and may be, e.g. 500 µm, 400 µm, 300 µm or 200 µm.

In a preferred embodiment, the particles have (i) an average diameter of 1000±300 µm, more preferably 1000±250 µm, still more preferably 1000±200 µm, yet more preferably 1000±150 µm, most preferably 1000±100 µm, and in particular 1000±50 µm; and/or (ii) an average length of 1000±300 µm, more preferably 1000±250 µm, still more preferably 1000±200 µm, yet more preferably 1000±150 µm, most preferably 1000±100 µm, and in particular 1000±50 µm.

The size of particles may be determined by any conventional procedure known in the art, e.g. laser light scattering, sieve analysis, light microscopy or image analysis.

Preferably, the plurality of particles that is contained in the pharmaceutical dosage form according to the invention has an arithmetic average weight, in the following referred to as "aaw", wherein at least 70%, more preferably at least 75%, still more preferably at least 80%, yet more preferably at least 85%, most preferably at least 90% and in particular at least 95% of the individual particles contained in said plurality of particles has an individual weight within the range of aaw±30%, more preferably aaw±25%, still more preferably aaw±20%, yet more preferably aaw±15%, most preferably aaw±10%, and in particular aaw±5%. For example, if the pharmaceutical dosage form according to the invention contains a plurality of 100 particles and aaw of said plurality of particles is 1.00 mg, at least 75 individual particles (i.e. 75%) have an individual weight within the range of from 0.70 to 1.30 mg (1.00 mg ±30%).

Preferably, the particles have an individual weight of at lost 5.0 mg, more preferably at most 4.5 mg, still more preferably at most 4.0 mg, yet more preferably at most 3.5 mg, even more preferably at most 3.0 mg, most preferably at most 2.5 mg, and in particular at most 2.0 mg.

In a preferred embodiment, the particles are not film coated.

In another preferred embodiment, the particles are film coated. It has been surprisingly found that when the particles are film coated, the disintegration time and/or the drug release from the pharmaceutical dosage forms can be further accelerated, which is particularly significant for pharmaceutical dosage forms with immediate drug release.

The particles according to the invention can optionally be provided, partially or completely, with a conventional coating. The particles according to the invention are preferably film coated with conventional film coating compositions. Suitable coating materials are commercially available, e.g. under the trademarks Opadry® and Eudragit®.

When the particles are film coated, the content of the dried film coating is preferably at most 5 wt.-%, more preferably at most 4 wt.-%, still more preferably at most 3.5 wt.-%, yet more preferably at most 3 wt.-%, most preferably at most 2.5 wt.-%, and in particular at most 2 wt.-%, based on the total weight of the particles. In a particularly preferred embodiment, the weight increase based on the total weight of the particles (uncoated starting material) is within the range of from 3.0 to 4.7 wt.-%, more preferably 3.1 to 4.6 wt.-%, still more preferably 3.2 to 4.5 wt.-%, yet more preferably 3.3 to 4.4 wt.-%, most preferably 3.4 to 4.3 wt.-%, and in particular 3.5 to 4.2 wt.-%.

The tamper-resistant pharmaceutical dosage form according to the invention comprises a multitude of particles which comprise a pharmacologically active compound. The particles contain at least a pharmacologically active compound, preferably an opioid; a polyalkylene oxide having a weight average molecular weight within the range of from 200,000 g/mol to 2,000,000 g/mol; and a disintegrant. Preferably, however, the particles contain additional pharmaceutical excipients such as gelling agents, acids, antioxidants and plasticizers.

The pharmacologically active compound is dispersed in a matrix comprising the polyalkylene oxide and the disintegrant. In other words, the polyalkylene oxide and the disintegrant form a matrix in which the pharmacologically active compound is embedded.

The pharmacologically active compound is not particularly limited. Preferably, the pharmacologically active compound is an opioid.

In a preferred embodiment, the particles and the pharmaceutical dosage form, respectively, contain only a single pharmacologically active compound, preferably only a single opioid. In another preferred embodiment, the particles and the pharmaceutical dosage form, respectively, contain a combination of two or more pharmacologically active compounds.

Preferably, pharmacologically active compound is an active ingredient with potential for being abused. Active ingredients with potential for being abused are known to the person skilled in the art and comprise e.g. tranquillizers, stimulants, barbiturates, narcotics, opioids or opioid derivatives.

Preferably, the pharmacologically active compound exhibits psychotropic action.

Preferably, the pharmacologically active compound is selected from the group consisting of opiates, opioids, stimulants, tranquilizers, and other narcotics.

In a preferred embodiment, the pharmacologically active compound is an opioid. According to the ATC index, opioids are divided into natural opium alkaloids, phenylpiperidine derivatives, diphenylpropylamine derivatives, benzomorphan derivatives, oripavine derivatives, morphinan derivatives and others.

In another preferred embodiment, the pharmacologically active compound is a stimulant. Stimulants are psychoactive drugs that induce temporary improvements in either mental or physical functions or both. Examples of these kinds of effects may include enhanced wakefulness, locomotion, and alertness. Preferred stimulants are phenylethylamine derivatives. According to the ATC index, stimulants are contained in different classes and groups, e.g. psychoanaleptics, especially psychostimulants, agents used for ADHD and nootropics, particularly centrally acting sympathomimetics; and e.g. nasal preparations, especially nasal decongestants for systemic use, particularly sympathomimetics.

The following opiates, opioids, stimulants, tranquillizers or other narcotics are substances with a psychotropic action, i.e. have a potential of abuse, and hence are preferably contained in the pharmaceutical dosage form and the particles, respectively: alfentanil, allobarbital, allylprodine, alphaprodine, alprazolam, amfepramone, amphetamine, amphetaminil, amobarbital, anileridine, apocodeine, axomadol, barbital, bemidone, benzyl-morphine, bezitramide, bromazepam, brotizolam, buprenorphine, butobarbital, butorphanol, camazepam, carfentanil, cathine/D-norpseudoephedrine, cebranopadol, chlordiazepoxide, clobazam clofedanol, clonazepam, clonitazene, clorazepate, clotiazepam, cloxazolam, cocaine, codeine, cyclobarbital, cyclorphan, cyprenorphine, delorazepam, desomorphine, dex-amphetamine, dextromoramide, dextropropoxyphene, dezocine, diampromide, diamorphone, diazepam, dihydrocodeine, dihydromorphine, dihydromorphone, dimenoxadol, dimephetamol, dimethylthiambutene, dioxaphetylbutyrate, dipipanone, dronabinol, eptazocine, estazolam, ethoheptazine, ethylmethylthiambutene, ethyl loflazepate, ethylmorphine, etonitazene, etorphine, faxeladol, fencamfamine, fenethylline, fenpipramide, fenproporex, fentanyl, fludiazepam, flunitrazepam, flurazepam, halazepam, haloxazolam, heroin, hydrocodone, hydromorphone, hydroxypethidine, isomethadone, hydroxymethylmorphinan, ketazolam, ketobemidone, levacetylmethadol (LAAM), levomethadone, levorphanol, levophenacylmorphane, levoxemacin, lisdexamfetamine dimesylate, lofentanil, loprazolam, lorazepam, lormetazepam, mazindol, medazepam, mefenorex, meperidine, meprobamate, metapon, meptazinol, metazocine, methylmorphine, metamphetamine, methadone, methaqualone, 3-methylfentanyl, 4-methylfentanyl, methylphenidate, methyl-phenobarbital, methyprylon, metopon, midazolam, modafinil, morphine, myrophine, nabilone, nalbuphene, nalorphine, narceine, nicomorphine, nimetazepam, nitrazepam, nordazepam, norlevorphanol, normethadone, normorphine, norpipanone, opium, oxazepam, oxazolam, oxycodone, oxymorphone, Papaver somniferum, papaveretum, pernoline, pentazocine, pentobarbital, pethidine, phenadoxone, phenomorphane, phenazocine, phenoperidine, piminodine, pholcodeine, phenmetrazine, phenobarbital, phentermine, pinazepam, pipradrol, piritramide, prazepam, profadol, proheptazine, promedol, properidine, propoxyphene, pseudoephedrine, remifentanil, secbutabarbital, secobarbital, sufentanil, tapentadol, temazepam, tetrazepam, tilidine (cis and trans), tramadol, triazolam, vinylbital, N-(1-methyl-2-piperidinoethyl)-N-(2-pyridyl)propionamide, (1R,2R)-3-(3-dimethylamino-1-ethyl-2-methyl-propyl)phenol, (1R,2R,4S)-2-(dimethylamino)methyl-4-(p-fluorobenzyloxy)-1-(m-methoxyphenyl)cyclohexanol, (1R,2R)-3-(2-dimethylaminomethyl-cyclohexyl)phenol, (1S,2S)-3-(3-dimethylamino-1-ethyl-2-methyl-propyl)phenol, (2R,3R)-1-dimethylamino-3 (3 -methoxyphenyl)-2-methyl-pentan-3 -ol, (1RS,3RS,6RS)-6-dimethylaminomethyl-1-(3 -methoxyphenyl)-cyclohexane-1,3 -diol, preferably as racemate, 3-(2-dimethylaminomethyl-1-hydroxy-cyclohexyl)phenyl 2-(4-isobutyl-phenyl)propionate, 3-(2-dimethylaminomethyl-1-hydroxy-cyclohexyl)phenyl 2-(6-methoxy-naphthalen-2-yl)propionate, 3-(2-dimethylaminomethyl-cyclohex-1-enyl)-phenyl 2-(4-isobutyl-phenyl)propionate, 3-(2-dimethylaminomethyl-cyclohex-1-enyl)-phenyl 2-(6-methoxy-naphthalen-2-yl)propionate, (RR-SS)-2-acetoxy-4-trifluoromethylbenzoic acid 3-(2-dimethylaminomethyl-1-hydroxy-cyclohexyl)-phenyl ester, (RR-SS)-2-hydroxy-4-trifluoromethyl-benzoic acid 3-(2-dimethylaminomethyl-1-hydroxy-cyclohexyl)-phenyl ester, (RR-SS)-4-chloro-2-hydroxy-benzoic acid 3-(2-dimethylaminomethyl-1-hydroxy-cyclohexyl)-phenyl ester, (RR-SS)-2-hydroxy-4-methyl-benzoic acid 3-(2-dimethylaminomethyl-1-hydroxy-cyclohexyl)-phenyl ester, (RR-SS)-2-hydroxy-4-methoxy-benzoic acid 3-(2-dimethylaminomethyl-1-hydroxy-cyclohexyl)-phenyl ester, (RR-SS)-2-hydroxy-5-nitro-benzoic acid 3-(2-dimethylaminomethyl-1-hydroxy-cyclohexyl)-phenyl ester, (RR-SS)-2',4'-difluoro-3-hydroxy-biphenyl-4-carboxylic acid 3-(2-dimethylaminomethyl-1-hydroxy-cyclohexyl)-phenyl ester, and corresponding stereoisomeric compounds, in each case the corresponding derivatives thereof, physiologically acceptable enantiomers, stereoisomers, diastereomers and racemates and the physiologically acceptable derivatives thereof, e.g. ethers, esters or amides, and in each case the physiologically acceptable compounds thereof, in particular the acid or base addition salts thereof and solvates, e.g. hydrochlorides.

In a preferred embodiment, the pharmacologically active compound is selected from the group consisting of DPI-125, M6G (CE-04-410), ADL-5859, CR-665, NRP290 and sebacoyl dinalbuphine ester.

The pharmacologically active compound may be present in form of a physiologically acceptable salt, e.g. physiologically acceptable acid addition salt.

Physiologically acceptable acid addition salts comprise the acid addition salt forms which can conveniently be obtained by treating the base form of the active ingredient with appropriate organic and inorganic acids. Active ingredients containing an acidic proton may be converted into their non-toxic metal or amine addition salt forms by treatment with appropriate organic and inorganic bases. The term addition salt also comprises the hydrates and solvent addition forms which the active ingredients are able to form. Examples of such forms are e.g. hydrates, alcoholates and the like.

In a preferred embodiment, the pharmacologically active compound is an opioid selected from the group consisting of oxycodone, hydrocodone, oxymorphone, hydromorphone, morphine, tramadol, tapentadol, and the physiologically acceptable salts thereof.

In another preferred embodiment, the pharmacologically active compound is a stimulant selected from the group consisting of amphetamine, dex-amphetamine, dex-methylphenidate, atomoxetine, caffeine, ephedrine, phenylpropanolamine, phenylephrine, fencamphamin, fenozolone, fenetylline, methylenedioxymethamphetamine (MDMA), methylenedioxypyrovalerone (MDPV), prolintane, lisdexamfetamine, mephedrone, methamphetamine, methylphenidate, modafinil, nicotine, pemoline, phenylpropanolamine, propylhexedrine, dimethylamylamine, and pseudoephedrine.

In a particularly preferred embodiment, the pharmacologically active compound is methylphenidate.

In another particularly preferred embodiment, the pharmacologically active compound is lisdexamfetamine.

In another particularly preferred embodiment, the pharmacologically active compound is dex-methylphenidate.

In a preferred embodiment, the pharmacologically active compound is amphetamine aspartate monohydrate.

In another preferred embodiment, the pharmacologically active compound is dextroamphetamine saccharate.

In another preferred embodiment, the pharmacologically active compound is dextroamphetamine sulfate.

It has been found that the content of the pharmacologically active compound in the pharmaceutical dosage form and in the particles, respectively, can be optimized in order to provide the best compromise between tamper-resistance, disintegration time and drug release, drug load, processability (especially tablettability) and patient compliance.

The pharmacologically active compound is present in the pharmaceutical dosage form in a therapeutically effective amount. The amount that constitutes a therapeutically effective amount varies according to the active ingredients being used, the condition being treated, the severity of said condition, the patient being treated, and the frequency of administration.

The content of the pharmacologically active compound, preferably of the opioid, in the pharmaceutical dosage form is not limited. The dose of the pharmacologically active compound which is adapted for administration preferably is in the range of 0.1 mg to 500 mg, more preferably in the range of 1.0 mg to 400 mg, even more preferably in the range of 5.0 mg to 300 mg, and most preferably in the range of 10 mg to 250 mg. In a preferred embodiment, the total amount of the pharmacologically active compound, preferably of the opioid, that is contained in the pharmaceutical dosage form is within the range of from 0.01 to 200 mg, more preferably 0.1 to 190 mg, still more preferably 1.0 to 180 mg, yet more preferably 1.5 to 160 mg, most preferably 2.0 to 100 mg and in particular 2.5 to 80 mg.

Preferably, the content of the pharmacologically active compound is at least 0.5 wt.-%, based on the total weight of the pharmaceutical dosage form and/or based on the total weight of the particles.

Preferably, the content of the pharmacologically active compound is within the range of from 0.01 to 80 wt.-%, more preferably 0.1 to 50 wt.-%, still more preferably 1 to 25 wt.-%, based on the total weight of the pharmaceutical dosage form and/or based on the total weight of the particles.

In a preferred embodiment, the content of pharmacologically active compound is within the range of from 0.50±0.45 wt.-%, or 0.75±0.70 wt.-%, or 1.00±0.90 wt.-%, or 1.25±1.20 wt.-%, or 1.50±1.40 wt.-%, or 1.75±1.70 wt.-%, or 2.00±1.90 wt.-%, or 2.25±2.20 wt.-%, or 2.50±2.40 wt.-%; more preferably 0.50±0.40 wt.-%, or 0.75±0.60 wt.-%, or 1.00±0.80 wt.-%, or 1.25±1.10 wt.-%, or 1.50±1.25 wt.-%, or 1.75±1.50 wt.-%, or 2.00±1.75 wt.-%, or 2.25±2.00 wt.-%, or 2.50±2.25 wt.-%; still more preferably 0.50±0.35 wt.-%, or 0.75±0.50 wt.-%, or 1.00±0.70 wt.-%, or 1.25±1.00 wt.-%, or 1.50±1.15 wt.-%, or 1.75±1.30 wt.-%, or 2.00±1.50 wt.-%, or 2.25±1.90 wt.-%, or 2.50±2.10 wt.-%; yet more preferably 0.50±0.30 wt.-%, or 0.75±0.40 wt.-%, or 1.00±0.60 wt.-%, or 1.25±0.80 wt.-%, or 1.50±1.00 wt.-%, or 1.75±1.10 wt.-%, or 2.00±1.40 wt.-%, or 2.25±1.60 wt.-%, or 2.50±1.80 wt.-%; even more preferably 0.50±0.25 wt.-%, or 0.75±0.30 wt.-%, or 1.00±0.50 wt.-%, or 1.25±0.60 wt.-%, or 1.50±0.80 wt.-%, or 1.75±0.90 wt.-%, or 2.00±1.30 wt.-%, or 2.25±1.40 wt.-%, or 2.50±1.50 wt.-%; most preferably 0.50±0.20 wt.-%, or 0.75±0.25 wt.-%, or 1.00±0.40 wt.-%, or 1.25±0.50 wt.-%, or 1.50±0.60 wt.-%, or 1.75±0.70 wt.-%, or 2.00±1.10 wt.-%, or 2.25±1.20 wt.-%, or 2.50±1.30 wt.-%; and in particular 0.50±0.15 wt.-%, or 0.75±0.20 wt.-%, or 1.00±0.30 wt.-%, or 1.25±0.40 wt.-%, or 1.50±0.50 wt.-%, or 1.75±0.60 wt.-%, or 2.00±0.70 wt.-%, or 2.25±0.80 wt.-%, or 2.50±0.90 wt.-%; in each case based on the total weight of the pharmaceutical dosage form.

In a preferred embodiment, the content of pharmacologically active compound is within the range of from 2.0±1.9 wt.-%, or 2.5±2.4 wt.-%, or 3.0±2.9 wt.-%, or 3.5±3.4 wt.-%, or 4.0±3.9 wt.-%, or 4.5±4.4 wt.-%, or 5.0±4.9 wt.-%, or 5.5±5.4 wt.-%, or 6.0±5.9 wt.-%; more preferably 2.0±1.7 wt.-%, or 2.5±2.2 wt.-%, or 3.0±2.6 wt.-%, or 3.5±3.1 wt.-%, or 4.0±3.5 wt.-%, or 4.5±4.0 wt.-%, or 5.0±4.4 wt.-%, or 5.5±4.9 wt.-%, or 6.0±5.3 wt.-%; still more preferably 2.0±1.5 wt.-%, or 2.5±2.0 wt.-%, or 3.0±2.3 wt.-%, or 3.5±2.8 wt.-%, or 4.0±3.1 wt.-%, or 4.5±3.6 wt.-%, or 5.0±3.9 wt.-%, or 5.5±4.4 wt.-%, or 6.0±4.7 wt.-%; yet more preferably 2.0±1.3 wt.-%, or 2.5±1.8 wt.-%, or 3.0±2.0 wt.-%, or 3.5±2.5 wt.-%, or 4.0±2.7 wt.-%, or 4.5±3.2 wt.-%, or 5.0±3.4 wt.-%, or 5.5±3.9 wt.-%, or 6.0±4.1wt.-%; even more preferably 2.0±1.1 wt.-%, or 2.5±1.6 wt.-%, or 3.0±1.7 wt.-%, or 3.5±2.2 wt.-%, or 4.0±2.4 wt.-%, or 4.5±2.8 wt.-%, or 5.0±2.9 wt.-%, or 5.5±3.4 wt.-%, or 6.0±3.5 wt.-%; most preferably 2.0±0.9 wt.-%, or 2.5±1.4 wt.-%, or 3.0±1.4 wt.-%, or 3.5±1.9 wt.-%, or 4.0±2.1 wt.-%, or 4.5±2.4 wt.-%, or 5.0±2.4wt.-%, or 5.5±2.9 wt.-%, or 6.0±2.9 wt.-%; and in particular 2.0±0.7 wt.-%, or 2.5±1.2 wt.-%, or 3.0±1.1 wt.-%, or 3.5±1.6 wt.-%, or 4.0±1.8 wt.-%, or 4.5±2.0 wt.-%, or 5.0±1.9 wt.-%, or 5.5±2.4 wt.-%, or 6.0±2.3 wt.-%; in each case based on the total weight of the particles.

In a preferred embodiment, the content of pharmacologically active compound is within the range of from 10±6 wt.-%, more preferably 10±5 wt.-%, still more preferably 10±4 wt.-%, most preferably 10±3 wt.-%, and in particular 10±2 wt.-%, based on the total weight of the pharmaceutical dosage form and/or based on the total weight of the particles. In another preferred embodiment, the content of pharmacologically active compound is within the range of from 15±6 wt.-%, more preferably 15±5 wt.-%, still more preferably 15±4 wt.-%, most preferably 15±3 wt.-%, and in particular 15±2 wt.-%, based on the total weight of the pharmaceutical dosage form and/or based on the total weight of the particles. In a further preferred embodiment, the content of pharmacologically active compound is within the range of from 20±6 wt.-%, more preferably 20±5 wt.-%, still more preferably 20±4 wt.-%, most preferably 20±3 wt.-%, and in particular 20±2 wt.-%, based on the total weight of the pharmaceutical dosage form and/or based on the total weight of the particles. In another preferred embodiment, the content of pharmacologically active compound is within the range of from 25±6 wt.-%, more preferably 25±5 wt.-%, still more preferably 25±4 wt.-%, most preferably 25±3 wt.-%, and in particular 25±2 wt.-%, based on the total weight of the pharmaceutical dosage form and/or based on the total weight of the particles.

The skilled person may readily determine an appropriate amount of pharmacologically active compound to include in a pharmaceutical dosage form. For instance, in the case of analgesics, e.g. opioids, the total amount of pharmacologically active compound present in the pharmaceutical dosage form is that sufficient to provide analgesia. The total amount of pharmacologically active compound administered to a patient in a dose will vary depending on numerous factors including the nature of the pharmacologically active compound, the weight of the patient, the severity of the pain, the nature of other therapeutic agents being administered etc.

In a preferred embodiment, the pharmacologically active compound is contained in the pharmaceutical dosage form in an amount of 2.5±1 mg, 5.0±2.5 mg, 7.5±5 mg, 10±5 mg, 20±5 mg, 30±5 mg, 40±5 mg, 50±5 mg, 60±5 mg, 70±5 mg, 80±5 mg, 90±5 mg, 100±5 mg, 110±5 mg, 120±5 mg, 130±5, 140±5 mg, 150±5 mg, 160±5 mg, 170±5 mg, 180±5 mg, 190±5 mg, 200±5 mg, 210±5 mg, 220±5 mg, 230±5 mg, 240±5 mg, 250±5 mg, 260±5 mg, 270±5 mg, 280±5 mg, 290±5 mg, or 300±5 mg.

In another preferred embodiment, the pharmacologically active compound is contained in the pharmaceutical dosage form in an amount of 2.5±1 mg, 5.0±2.5 mg, 7.5±2.5 mg, 10±2.5 mg, 15±2.5 mg, 20±2.5 mg, 25±2.5 mg, 30±2.5 mg, 35±2.5 mg, 40±2.5 mg, 45±2.5 mg, 50±2.5 mg, 55±2.5 mg, 60±2.5 mg, 65±2.5 mg, 70±2.5 mg, 75±2.5 mg, 80±2.5 mg, 85±2.5 mg, 90±2.5 mg, 95±2.5 mg, 100±2.5 mg, 105±2.5 mg, 110±2.5 mg, 115±2.5 mg, 120±2.5 mg, 125±2.5 mg, 130±2.5 mg, 135±2.5 mg, 140±2.5 mg, 145±2.5 mg, 150±2.5 mg, 155±2.5 mg, 160±2.5 mg, 165±2.5 mg, 170±2.5 mg, 175±2.5 mg, 180±2.5 mg, 185±2.5 mg, 190±2.5 mg, 195±2.5 mg, 200±2.5 mg, 205±2.5 mg, 210±2.5 mg, 215±2.5 mg, 220±2.5 mg, 225±2.5 mg, 230±2.5 mg, 235±2.5 mg, 240±2.5 mg, 245±2.5 mg, 250±2.5 mg, 255±2.5 mg, 260±2.5 mg, or 265±2.5 mg.

In a particularly preferred embodiment, the pharmacologically active compound is tapentadol, preferably its HCl salt, and the pharmaceutical dosage form is adapted for administration once daily, twice daily, thrice daily or more frequently. In this embodiment, pharmacologically active compound is preferably contained in the pharmaceutical dosage form in an amount of from 25 to 100 mg.

In a particularly preferred embodiment, the pharmacologically active compound is oxymorphone, preferably its HCl salt, and the pharmaceutical dosage form is adapted for administration once daily, twice daily, thrice daily or more frequently. In this embodiment, the pharmacologically active compound is preferably contained in the pharmaceutical dosage form in an amount of from 5 to 40 mg. In another particularly preferred embodiment, the pharmacologically active compound is oxymorphone, preferably its HCl salt, and the pharmaceutical dosage form is adapted for administration once daily. In this embodiment, the pharmacologically active compound is preferably contained in the pharmaceutical dosage form in an amount of from 10 to 80 mg.

In another particularly preferred embodiment, the pharmacologically active compound is oxycodone, preferably its HCl salt, and the pharmaceutical dosage form is adapted for administration once daily, twice daily, thrice daily or more frequently. In this embodiment, the pharmacologically active compound is preferably contained in the pharmaceutical dosage form in an amount of from 5 to 80 mg.

In still another particularly preferred embodiment, the pharmacologically active compound is hydromorphone, preferably its HCl, and the pharmaceutical dosage form is adapted for administration once daily, twice daily, thrice daily or more frequently. In this embodiment, the pharmacologically active compound is preferably contained in the pharmaceutical dosage form in an amount of from 2 to 52 mg. In another particularly preferred embodiment, the pharmacologically active compound is hydromorphone, preferably its HCl, and the pharmaceutical dosage form is adapted for administration once daily, twice daily, thrice daily or more frequently. In this embodiment, the pharmacologically active compound is preferably contained in the pharmaceutical dosage form in an amount of from 4 to 104 mg.

In yet another particularly preferred embodiment, the pharmacologically active compound is hydrocodone, preferably its bitartrate salt, and the pharmaceutical dosage form is adapted for administration once daily, twice daily, thrice daily or more frequently. In this embodiment, the pharmacologically active compound is preferably contained in the pharmaceutical dosage form in an amount of from 2.5 to 10 mg.

The particles present in the pharmaceutical dosage forms according to the invention preferably comprise 3 to 75 wt.-% of pharmacologically active compound, more preferably 5 to 70 wt.-% of pharmacologically active compound, still more preferably 7.5 to 65 wt.-% of pharmacologically active compound, based on the total weight of the pharmaceutical dosage form and/or based on the total weight of the particles.

Preferably, the content of the pharmacologically active compound is at least 25 wt.-%, more preferably at least 30 wt.-%, still more preferably at least 35 wt.-%, yet more preferably at least 40 wt.-%, most preferably at least 45 wt.-%, based on the total weight of the pharmaceutical dosage form and/or based on the total weight of the particles.

Preferably, the content of the pharmacologically active compound is at most 70 wt.-%, more preferably at most 65 wt.-%, still more preferably at most 60 wt.-%, yet more preferably at most 55 wt.-%, most preferably at most 50 wt.-%, based on the total weight of the pharmaceutical dosage form and/or based on the total weight of the particles.

In a preferred embodiment, the content of the pharmacologically active compound is within the range of 35±30 wt.-%, more preferably 35±25 wt.-%, still more preferably 35±20 wt.-%, yet more preferably 35±15 wt.-%, most preferably 35±10 wt.-%, and in particular 35±5 wt.-%, based on the total weight of the pharmaceutical dosage form and/or based on the total weight of the particles. In another preferred embodiment, the content of the pharmacologically active compound is within the range of 45±30 wt.-%, more preferably 45±25 wt.-%, still more preferably 45±20 wt.-%, yet more preferably 45±15 wt.-%, most preferably 45±10 wt.-%, and in particular 45±5 wt.-%, based on the total weight of the pharmaceutical dosage form and/or based on the total weight of the particles. In still another preferred embodiment, the content of the pharmacologically active compound is within the range of 55±30 wt.-%, more preferably 55±25 wt.-%, still more preferably 55±20 wt.-%, yet more preferably 55±15 wt.-%, most preferably 55±10 wt.-%, and in particular 55±5 wt.-%, based on the total weight of the pharmaceutical dosage form and/or based on the total weight of the particles.

The pharmacologically active compound that is included in the preparation of the pharmaceutical dosage forms according to the invention preferably has an average particle size of less than 500 microns, still more preferably less than 300 microns, yet more preferably less than 200 or 100 microns. There is no lower limit on the average particle size and it may be, for example, 50 microns. The particle size of pharmacologically active compounds may be determined by any technique conventional in the art, e.g. laser light scattering, sieve analysis, light microscopy or image analysis. Generally speaking it is preferable that the largest dimension of the pharmacologically active compound particle be less than the size of the particles (e.g. less than the smallest dimension of the particles).

A skilled person knows how to determine pharmacokinetic parameters such as t_{1/2}, Tₘₐₓ, Cₘₐₓ, AUC and bioavailability. For the purposes of the description, the pharmacokinetic parameters, which may be determined from the blood plasma concentrations of 3-(2-dimethylaminomethylcyclohexyl)phenol, are defined as follows:

| | |
|---|---|
| Cₘₐₓ | maximum measured plasma concentration of the active ingredient after single administration (≡ average *peak plasma level*) |
| tₘₐₓ | interval of time from administration of the active ingredient until Cₘₐₓ is reached |
| AUC | total area of the plasma concentration/time curve including the subarea from the final measured value extrapolated to infinity |
| t_{1/2} | half-life |

The above parameters are in each case stated as mean values of the individual values for all investigated patients/test subjects.

A person skilled in the art knows how the pharmacokinetic parameters of the active ingredient may be calculated from the measured concentrations of the active ingredient in the blood plasma. In this connection, reference may be made, for example, to Willi Cawello (ed.) Parameters for Compartment-free Pharmacokinetics, Shaker Verlag Aachen (1999).

In a preferred embodiment, the pharmacologically active compound is tapentadol or a physiologically acceptable salt thereof, e.g. the hydrochloride. Preferably, the pharmaceutical dosage form according to the invention provides a mean absolute bioavailability of tapentadol of at least 22%, more preferably at least 24%, still more preferably at least 26%, yet more preferably at least 28%, most preferably at least 30%, and in particular at least 32%. Tₘₐₓ of tapentadol is preferably within the range of 1.25±1.20 h, more preferably 1.25±1.00 h, still more preferably 1.25±0.80 h, yet more preferably 1.25±0.60 h, most preferably 1.25±0.40 h, and in particular 1.25±0.20 h. t_{1/2} of tapentadol is preferably within the range of 4.0±2.8 h, more preferably 4.0±2.4 h, still more preferably 4.0±2.0 h, yet more preferably 4.0±1.6 h, most preferably 4.0±1.2 h, and in particular 4.0±0.8 h. Preferably, when normalized to a dose of 100 mg tapentadol, Cₘₐₓ of tapentadol is preferably within the range of 90±85 ng/mL, more preferably 90±75 ng/mL, still more preferably 90±65 ng/mL, yet more preferably 90±55 ng/mL, most preferably 90±45 ng/mL, and in particular 90±35 ng/mL; and/or AUC of tapentadol is preferably within the range of 420±400 ng/mL-h, more preferably 420±350 ng/mL-h, still more preferably 420±300 ng/mL-h, yet more preferably 420±250 ng/mL-h, most preferably 420±200 ng/mL-h, and in particular 420±150 ng/mL-h.

In another preferred embodiment, the pharmacologically active compound is oxycodone or a physiologically acceptable salt thereof, e.g. the hydrochloride. Preferably, the pharmaceutical dosage form according to the invention provides a mean absolute bioavailability of oxycodone of at least 40%, more preferably at least 45%, still more preferably at least 50%, yet more preferably at least 55%, most preferably at least 60%, and in particular at least 70%. Tₘₐₓ of oxycodone is preferably within the range of 2.6±2.5 h, more preferably 2.6±2.0 h, still more preferably 2.6±1.8 h, yet more preferably 2.6±0.1.6 h, most preferably 2.6±1.4 h, and in particular 2.6±1.2 h. t_{1/2} of oxycodone is preferably within the range of 3.8±3.5 h, more preferably 3.8±3.0 h, still more preferably 3.8±2.5 h, yet more preferably 3.8±2.0 h, most preferably 3.8±1.5 h, and in particular 3.8±1.0 h. Preferably, when normalized to a dose of 30 mg oxycodone, Cₘₐₓ of oxycodone is preferably within the range of 40±35 ng/mL, more preferably 40±30 ng/mL, still more preferably 40±25 ng/mL, yet more preferably 40±20 ng/mL, most preferably 40±15 ng/mL, and in particular 40±10 ng/mL; and/or AUC of oxycodone is preferably within the range of 270±250 ng/mL·h, more preferably 270±200 ng/mL·h, still more preferably 270±150 ng/mL·h, yet more preferably 270±100 ng/mL·h, most preferably 270±75 ng/mL·h, and in particular 270±50 ng/mL·h.

In still another preferred embodiment, the pharmacologically active compound is hydrocodone or a physiologically acceptable salt thereof, e.g. the bitartrate. Tₘₐₓ of hydrocodone is preferably within the range of 1.3±1.2 h, more preferably 1.3±1.0 h, still more preferably 1.3±0.8 h, yet more preferably 1.3±0.6 h, most preferably 1.3±0.4 h, and in particular 1.3±0.2 h. t_{1/2} of hydrocodone is preferably within the range of 3.8±3.5 h, more preferably 3.8±3.0 h, still more preferably 3.8±2.5 h, yet more preferably 3.8±2.0 h, most preferably 3.8±1.5 h, and in particular 3.8±1.0h.

In yet another preferred embodiment, the pharmacologically active compound is morphine or a physiologically acceptable salt thereof, e.g. the sulfate. Preferably, the pharmaceutical dosage form according to the invention provides a mean absolute bioavailability of morphine of at least 15%, more preferably at least 20%, still more preferably at least 25%, yet more preferably at least 30%, most preferably at least 35%, and in particular at least 40%. Tₘₐₓ of morphine is preferably within the range of 0.625±0.60 h, more preferably 0.625±0.50 h, still more preferably 0.625±0.40 h, yet more preferably 0.625±0.30 h, most preferably 0.625±0.20 h, and in particular 0.625±0.15 h. Preferably, when normalized to a dose of 30 mg morphine sulfate, Cₘₐₓ of morphine is preferably within the range of 25±20 ng/mL, more preferably 25±15 ng/mL, still more preferably 25±10 ng/mL, yet more preferably 25±5 ng/mL; and/or AUC of morphine is preferably within the range of 50±45 ng/mL·h, more preferably 50±40 ng/mL·h, still more preferably 50±35 ng/mL·h, yet more preferably 50±30 ng/mL·h, most preferably 50±25 ng/mL·h, and in particular 50±20 ng/mL·h.

In still another preferred embodiment, the pharmacologically active compound is amphetamine or a physiologically acceptable salt thereof. Tₘₐₓ of amphetamine is preferably within the range of 1.7±1.2 h, more preferably 1.7±1.0 h, still more preferably 1.7±0.8 h, yet more preferably 1.7±0.6 h, most preferably 1.7±0.4 h, and in particular 1.7±0.2 h.

In still another preferred embodiment, the pharmacologically active compound is dex-amphetamine or a physiologically acceptable salt thereof, e.g. the sulfate. Tₘₐₓ of dex-amphetamine is preferably within the range of 3.0±2.9 h, more preferably 3.0±2.5 h, still more preferably 3.0±2.1 h, yet more preferably 3.0±1.7 h, most preferably 3.0±1.3 h, and in particular 3.0±0.9 h. t_{1/2} of dex-amphetamine is preferably within the range of 10±6.0 h, more preferably 10±5.0 h, still more preferably 10±4.0 h, yet more preferably 10±3.0 h, most preferably 10±2.0 h, and in particular 10±1.0 h.

The pharmaceutical dosage forms according to the invention may also comprise one or more additional pharmacologically active compounds. The additional pharmacologically active compound may be susceptible to abuse or another pharmaceutical. Additional pharmacologically active compounds may be present within the particles ("intragranular") or within the matrix ("extragranular"). Where an additional pharmacologically active compound is present intragranularly, it may be present either in combination with one or more pharmacologically active compounds within the same particles or in a discrete population of particles alone and separate from any other pharmacologically active compounds present in the pharmaceutical dosage form.

In a preferred embodiment, the pharmaceutical dosage form according to the invention, preferably the particles, comprise an opioid (agonist) as well as an opioid antagonist.

Any conventional opioid antagonist may be present, e.g. naltrexone or naloxone or their pharmaceutically acceptable salts. Naloxone, including its salts, is particularly preferred. The opioid antagonist may be present within the particles or within the matrix. Alternatively, opioid antagonist may be provided in separate particles to the pharmacologically active compounds. The preferred composition of such particles is the same as that described for pharmacologically active compound-containing particles.

The ratio of opioid agonist to opioid antagonist in the pharmaceutical dosage forms according to the invention is preferably 1 :1 to 3:1 by weight, for example, 2:1 by weight.

In another preferred embodiment, neither the particles nor the pharmaceutical dosage form comprise any opioid antagonist.

The tamper-resistant pharmaceutical dosage form according to the invention comprises a multitude of particles which comprise a polyalkylene oxide having a weight average molecular weight within the range of from 200,000 g/mol to 2,000,000 g/mol, wherein the content of the polyalkylene oxide is at least 25 wt.-%, based on the total weight of the pharmaceutical dosage form and/or based on the total weight of the particles.

Preferably, the polyalkylene oxide is selected from polymethylene oxide, polyethylene oxide and polypropylene oxide, or copolymers thereof. Polyethylene oxide is preferred.

Preferably, the polyalkylene oxide has a weight average molecular weight of at least 300,000 g/mol, more preferably at least 400,000 g/mol, still more preferably at least 500,000 g/mol, yet more preferably at least 600,000 g/mol, even more preferably at least 700,000 g/mol, most preferably at least 800,000 g/mol, and in particular at least 900,000 g/mol.

Preferably, the polyalkylene oxide has a weight average molecular weight of at most 1,700,000 g/mol, more preferably at most 1,600,000 g/mol, still more preferably at most 1,500,000 g/mol, yet more preferably at most 1,400,000 g/mol, even more preferably at most 1,300,000 g/mol, most preferably at most 1,200,000 g/mol, and in particular at most 1,100,000 g/mol.

Preferably, the polyalkylene oxide has a weight average molecular weight within the range of 1,000,000±750,000 g/mol; more preferably 1,000,000±500,000 g/mol; in particular 1,000,000±250,000 g/mol.

Suitable methods to determine M_{W} are known to a person skilled in the art. Mw can be determined e.g. by gel permeation chromatography (GPC).

Polyalkylene oxide may comprise a single polyalkylene oxide having a particular average molecular weight, or a mixture (blend) of different polymers, such as two, three, four or five polymers, e.g., polymers of the same chemical nature but different average molecular weight, polymers of different chemical nature but same average molecular weight, or polymers of different chemical nature as well as different molecular weight.

For the purpose of the specification, a polyalkylene glycol has a molecular weight of up to 20,000 g/mol whereas a polyalkylene oxide has a molecular weight of more than 20,000 g/mol. In a preferred embodiment, the weight average over all molecular weights of all polyalkylene oxides that are contained in the pharmaceutical dosage form is at least 200,000 g/mol. Thus, polyalkylene glycols, if any, are preferably not taken into consideration when determining the weight average molecular weight of polyalkylene oxide.

In a preferred embodiment, polyalkylene oxide is homogeneously distributed in the particles according to the invention. Preferably, the pharmacologically active compound and polyalkylene oxide are intimately homogeneously distributed in the particles so that the particles do not contain any segments where either pharmacologically active compound is present in the absence of polyalkylene oxide or where polyalkylene oxide is present in the absence of pharmacologically active compound.

When the particles are film coated, the polyalkylene oxide is preferably homogeneously distributed in the core of the particles, i.e. the film coating preferably does not contain polyalkylene oxide. Nonetheless, the film coating as such may of course contain one or more polymers, which however, preferably differ from the polyalkylene oxide contained in the core.

Preferably, the molecular weight dispersity M_{w}/Mₙ of polyalkylene oxide is within the range of 2.5±2.0, more preferably 2.5±1.5, still more preferably 2.5±1.0, yet more preferably 2.5±0.8, most preferably 2.5±0.6, and in particular 2.5±0.4.

The polyalkylene oxide preferably has a viscosity at 25°C of 200 to 4,000 cP, more preferably 300 to 2,000 cP, still more preferably 350 to 1,000 cP, in each case measured with a Brookfield viscosimeter, model RVF, on a 2 wt.-% aqueous solution using the stated viscosimeter (spindle no. 1 or 3 / rotational speed 10 rpm).

Polyethylene oxide that is suitable for use in the pharmaceutical dosage forms according to the invention is commercially available from Dow. For example, Polyox WSR N-750 NF (∼300,000 g/mol), Polyox WSR-205 NF (∼600,000 g/mol), Polyox WSR1105 NF (∼900,000 g/mol), Polyox WSR N-12K NF (∼1,000,000 g/mol), or Polyox WSR N-60K NF (∼2,000,000 g/mol) may be used in the pharmaceutical dosage forms according to the invention. Mixtures of different products are also contemplated. For details concerning the properties of these products, it can be referred to e.g. the product specification.

The polyalkylene oxide may be combined with one or more different polymers selected from the group consisting of polyalkylene oxide, preferably polymethylene oxide, polyethylene oxide, polypropylene oxide; polyethylene, polypropylene, polyvinyl chloride, polycarbonate, polystyrene, polyvinylpyrrolidone, poly(alk)acrylate, poly(hydroxy fatty acids), such as for example poly(3-hydroxybutyrate-co-3-hydroxyvalerate) (Biopol®), poly(hydroxyvaleric acid); polycaprolactone, polyvinyl alcohol, polyesteramide, polyethylene succinate, polylactone, polyglycolide, polyurethane, polyamide, polylactide, polyacetal (for example polysaccharides optionally with modified side chains), polylactide/glycolide, polylactone, polyglycolide, polyorthoester, polyanhydride, block polymers of polyethylene glycol and polybutylene terephthalate (Polyactive®), polyanhydride (Polifeprosan), copolymers thereof, block-copolymers thereof (e.g., Poloxamer®), and mixtures of at least two of the stated polymers, or other polymers with the above characteristics.

Preferably, the content of the polyalkylene oxide is at least at least 20 wt.-%, more preferably at least 25 wt.-%, still more preferably at least 30 wt.-%, yet more preferably at least 35 wt.-%, even more preferably at least 40 wt.-%, most preferably at least 45 wt.-%, and in particular at least 50 wt.-%, based on the total weight of the pharmaceutical dosage form and/or based on the total weight of the particles.

Preferably, the content of the polyalkylene oxide is at most at most 85 wt.-%, more preferably at most 80 wt.-%, still more preferably at most 75 wt.-%, yet more preferably at most 70 wt.-%, even more preferably at most 65 wt.-%, most preferably at most 60 wt.-%, and in particular at most 55 wt.-%, based on the total weight of the pharmaceutical dosage form and/or based on the total weight of the particles.

Preferably, the content of the polyalkylene oxide is within the range of from 25 to 80 wt.-%, more preferably 25 to 75 wt.-%, still more preferably 25 to 70 wt.-%, yet more preferably 25 to 65 wt.-%, most preferably 30 to 65 wt.-% and in particular 35 to 65 wt.-%, based on the total weight of the pharmaceutical dosage form and/or based on the total weight of the particles.

In a preferred embodiment, the overall content of polyalkylene oxide is within the range of 35±8 wt.-%, more preferably 35±6 wt.-%, most preferably 35±4 wt.-%, and in particular 35±2 wt.-%, based on the total weight of the pharmaceutical dosage form and/or based on the total weight of the particles.

In another preferred embodiment, the overall content of polyalkylene oxide is within the range of 40±12 wt.-%, more preferably 40±10 wt.-%, most preferably 40±7 wt.-%, and in particular 40±3 wt.-%, based on the total weight of the pharmaceutical dosage form and/or based on the total weight of the particles.

In still another preferred embodiment, the overall content of polyalkylene oxide is within the range of 45±16 wt.-%, more preferably 45±12 wt.-%, most preferably 45±8 wt.-%, and in particular 45±4 wt.-%, based on the total weight of the pharmaceutical dosage form and/or based on the total weight of the particles.

In yet another preferred embodiment, the overall content of polyalkylene oxide is within the range of 50±20 wt.-%, more preferably 50±15 wt.-%, most preferably 50±10 wt.-%, and in particular 50±5 wt.-%, based on the total weight of the pharmaceutical dosage form and/or based on the total weight of the particles.

In a further preferred embodiment, the overall content of polyalkylene oxide is within the range of 55±20 wt.-%, more preferably 55±15 wt.-%, most preferably 55±10 wt.-%, and in particular 55±5 wt.-%, based on the total weight of the pharmaceutical dosage form and/or based on the total weight of the particles.

In still a further a preferred embodiment, the overall content of polyalkylene oxide is within the range of 60±20 wt.-%, more preferably 60±15 wt.-%, most preferably 60±10 wt.-%, and in particular 60±5 wt.-%.

In a still further a preferred embodiment, the overall content of polyalkylene oxide is within the range of 65±20 wt.-%, more preferably 65±15 wt.-%, and most preferably 65±10 wt.-%, and in particular 65±5 wt.-%, based on the total weight of the pharmaceutical dosage form and/or based on the total weight of the particles.

Preferably, the relative weight ratio of the polyalkylene oxide to the pharmacologically active compound is within the range of 30:1 to 1:10, more preferably 20:1 to 1:1, still more preferably 15:1 to 5:1, yet more preferably 14:1 to 6:1, most preferably 13:1 to 7:1, and in particular 12:1 to 8:1.

The tamper-resistant pharmaceutical dosage form according to the invention comprises a multitude of particles which comprise a disintegrant, wherein the content of the disintegrant is more than 20 wt.-%, based on the total weight of the particles.

In a preferred embodiment, particularly when the pharmaceutical dosage form is a capsule, the pharmaceutical dosage form contains the entire amount of disintegrant within the particles, i.e. outside the particles there is preferably no disintegrant. Furthermore, the disintegrant is preferably homogeneously distributed in the particles. Preferably, when the particles are coated, the coating does not contain disintegrant.

In another preferred embodiment, particularly when the pharmaceutical dosage form is a tablet, the pharmaceutical dosage form contains the disintegrant within the particles as well as outside the particles. In a preferred embodiment, the nature of disintegrant within the particle is identical with the nature of disintegrant outside the particles. However, different disintegrants inside the particles and outside the particles are also possible in accordance with the invention. Furthermore, the disintegrant is preferably homogeneously distributed in the particles. Preferably, when the particles are coated, the coating does not contain disintegrant.

In still another preferred embodiment, particularly when the pharmaceutical dosage form is a tablet, the pharmaceutical dosage form contains the entire amount of disintegrant within the particles, i.e. outside the particles there is preferably no disintegrant.

Suitable disintegrants are known to the skilled person and are preferably selected from the group consisting of polysaccharides, starches, starch derivatives, cellulose derivatives, polyvinylpyrrolidones, acrylates, gas releasing substances, and the mixtures of any of the foregoing.

Preferred starches include but are not limited to "standard starch" (e.g. native maize starch) and pregelatinized starch (e.g. starch 1500).

Preferred starch derivatives include but are not limited to sodium starch glycolate (carboxymethyl starch sodium, e.g. Vivastar®).

Preferred cellulose derivatives include but are not limited to croscarmellose sodium (=crosslinked sodium carboxymethylcellulose; e.g. Vivasol®), carmellose calcium (calcium carboxymethylcellulose), carmellose sodium (sodium carboxymethylcellulose), low substituted carmellose sodium (low substituted sodium carboxymethylcellulose; average degree of substitution (DS) 0.20 to 0.40, Mr 80,000 to 600,000 g/mol, CAS 9004-32-4, E 466), low substituted hydroxypropylcellulose (having a content of propyl groups within the range of from 5 to 16%; CAS 9004-64-2).

Preferred acrylates include but are not limited to carbopol.

Preferred polyvinylpyrrolidones include but are not limited to crospovidone (PVP Cl).

Preferred gas releasing substances include but are not limited to sodium bicarbonate.

Preferred disintegrants include but are not limited to crosslinked sodium carboxymethylcellulose (Na-CMC) (e.g. Crosscarmellose, Vivasol® ,Ac-Di-Sol®); crosslinked casein (e.g. Esma-Spreng®); polysaccharide mixtures obtained from soybeans (e.g. Emcosoy®); maize starch or pretreated maize starch (e.g. Amijel®); alginic acid, sodium alginate, calcium alginate; polyvinylpyrrolidone (PVP) (e.g. Kollidone®, Polyplasdone®, Polydone®); crosslinked polyvinylpyrrolidone (PVP CI) (e.g. Polyplasdone® XL); starch and pretreated starch such as sodium carboxymethyl starch (= sodium starch glycolate, e.g. Explotab®, Prejel®, Primotab® ET, Starch® 1500, Ulmatryl®), and the mixtures thereof. Crosslinked polymers are particularly preferred disintegrants, especially crosslinked sodium carboxymethylcellulose (Na-CMC) or crosslinked polyvinylpyrrolidone (PVP CI).

Particularly preferred disintegrants are selected from the group consisting of
- crosslinked sodium carboxymethylcellulose (Na-CMC) (e.g. Crosscarmellose, Vivasol®, Ac-Di-Sol®);
- crosslinked casein (e.g. Esma-Spreng®);
- alginic acid, sodium alginate, calcium alginate;
- polysaccharide mixtures obtained from soybeans (e.g. Emcosoy®);
- starch and pretreated starch such as sodium carboxymethyl starch (= sodium starch glycolate, e.g. Explotab®, Prejel®, Primotab® ET, Starch® 1500, Ulmatryl®);
- maize starch or pretreated maize starch (e.g. Amijel®);
- and mixtures of any of the foregoing.

Preferably, the content of the disintegrant is at least 21 wt.-%, at least 22 wt.-%, at least 23 wt.-%, at least 24 wt.-%, or at least 25 wt.-%, or at least 26 wt.-%, or at least 27 wt.-%, or at least 28 wt.-%, more preferably at least 29 wt.-%, still more preferably at least 30 wt.-%, yet more preferably at least 31 wt.-%, even more preferably at least 32 wt.-%, most preferably at least 33 wt.-%, and in particular at least 34 wt.-%, based on the total weight of the pharmaceutical dosage form and/or based on the total weight of the particles.

Preferably, the content of the disintegrant is at least 25 wt.-%; preferably at least 30 wt.-%; in each case based on the total weight of the particles.

Preferably, the content of the disintegrant is at most 49 wt.-%, at most 48 wt.-%, at most 47 wt.-%, at most 46 wt.-%, or at most 45 wt.-%, or at most 44 wt.-%, or at most 43 wt.-%, or at most 42 wt.-%, more preferably at most 41 wt.-%, still more preferably at most 40 wt.-%, yet more preferably at most 39 wt.-%, even more preferably at most 38 wt.-%, most preferably at most 37 wt.-%, and in particular at most 36 wt.-%, based on the total weight of the pharmaceutical dosage form and/or based on the total weight of the particles.

It has been surprisingly found that when the weight average molecular weight of the polyalkylene oxide is not more than 2,000,000 g/mol, the content of disintegrant may be increased to values beyond 20 wt.-%, relative to the total weight of the particles, thereby achieving excellent resistance against solvent extraction as well as excellent resistance against mechanical disruption.

In a preferred embodiment, the content of the disintegrant in the pharmaceutical dosage form is within the range of 35±15 wt.-%, more preferably 35±14 wt.-%, still more preferably 35±13 wt.-%, yet more preferably 35±12 wt.-%, most preferably 35±11 wt.-%, and in particular 35±10 wt.-%, based on the total weight of the pharmaceutical dosage form and/or based on the total weight of the particles. In another preferred embodiment, the content of the disintegrant in the pharmaceutical dosage form is within the range of 35±9.0 wt.-%, more preferably 35±8.5 wt.-%, still more preferably 35±8.0 wt.-%, yet more preferably 35±7.5 wt.-%, most preferably 35±7.0 wt.-%, and in particular 35±6.5 wt.-%, based on the total weight of the pharmaceutical dosage form and/or based on the total weight of the particles. In still another preferred embodiment, the content of the disintegrant in the pharmaceutical dosage form is within the range of 35±6.0 wt.-%, more preferably 35±5.5 wt.-%, still more preferably 35±5.0 wt.-%, yet more preferably 35±4.5 wt.-%, most preferably 35±4.0 wt.-%, and in particular 35±3.5 wt.-%, based on the total weight of the pharmaceutical dosage form and/or based on the total weight of the particles. In another preferred embodiment, the content of the disintegrant in the pharmaceutical dosage form is within the range of 35±3.0 wt.-%, more preferably 35±2.5 wt.-%, still more preferably 35±2.0 wt.-%, yet more preferably 35±1.5 wt.-%, most preferably 35±1.0 wt.-%, and in particular 35±0.5 wt.-%, based on the total weight of the pharmaceutical dosage form and/or based on the total weight of the particles.

Preferably, the content of the disintegrant is within the range of 35±15 wt.-%; preferably 35±10 wt.-%; more preferably 35±5.0 wt.-%; in each case based on the total weight of the particles.

When the pharmaceutical dosage form according to the invention contains more than a single disintegrant, e.g. a mixture of two different disintegrants, the above percentages preferably refer to the total content of disintegrants.

Preferably, the relative weight ratio of the polyalkylene oxide to the disintegrant is within the range of 4.5:1 to 1:3.5, more preferably 4:1 to 1:3, still more preferably 3.5:1 to 1:2.5, yet more preferably 3:1 to 1:2, most preferably 2.5:1 to 1:1.5, and in particular 2:1 to 1:1.

Preferably, the relative weight ratio of the pharmacologically active ingredient to the disintegrant is within the range of 1:4 to 1:10, more preferably 1:4.5 to 1:9.5, still more preferably 1:5 to 1:9, yet more preferably 1:.5.5 to 1:8.5, most preferably 1:6 to 1:8, and in particular 1:6.5 to 1:7.5.

The pharmaceutical dosage form may contain a single disintegrant or a mixture of different disintegrants. Preferably, the pharmaceutical dosage form contains a single disintegrant.

Preferably, the pharmaceutical dosage form and/or the particles according to the invention additionally comprise a gelling agent, which is preferably a polysaccharide, more preferably a cellulose ether, in particular hydroxyethylcellulose.

While the gelling agent may principally contribute to the overall resistance against solvent extraction of the pharmaceutical dosage form according to the invention, it has been unexpectedly found that one or more disintegrants in comparatively high amounts in combination with one or more gelling agents are of particular advantage in this regard. It has been surprisingly found that the combination of one or more disintegrants in comparatively high amounts with one or more gelling agent is robust against variation of the pharmacologically active ingredient. Thus, according to the present invention exchanging a given pharmacologically active ingredient by another pharmacologically active ingredient does preferably not substantially alter the overall resistance against solvent extraction of the pharmaceutical dosage form according to the invention

As used herein the term "gelling agent" is used to refer to a compound that, upon contact with a solvent (e.g. water), absorbs the solvent and swells, thereby forming a viscous or semi-viscous substance. Preferred gelling agents are not cross-linked. This substance may moderate pharmacologically active compound release from the particles in both aqueous and aqueous alcoholic media. Upon full hydration, a thick viscous solution or dispersion is typically produced that significantly reduces and/or minimizes the amount of free solvent which can contain an amount of solubilized pharmacologically active compound, and which can be drawn into a syringe. The gel that is formed may also reduce the overall amount of pharmacologically active compound extractable with the solvent by entrapping the pharmacologically active compound within a gel structure. Thus the gelling agent may play an important role in conferring tamper-resistance to the pharmaceutical dosage forms according to the invention.

Gelling agents include pharmaceutically acceptable polymers, typically hydrophilic polymers, such as hydrogels. Representative examples of gelling agents include gums like xanthan gum, carrageenan, locust bean gum, guar, tragacanth, acaica (gum arabic), karaya, tara and gellan gum; polyethylene oxide, polyvinyl alcohol, cellulose ethers, carbomers, poly(uronic) acids and mixtures thereof.

Preferably, the gelling agent is a polysaccharide; preferably a cellulose ether; more preferably a cellulose ether selected from the group consisting of methylcellulose, hydroxyethylcellulose, hydroxy-propylcellulose, and hydroxypropylmethylcellulose; in particular hydroxyethylcellulose.

Preferably, the content of the gelling agent, preferably hydroxyethylcellulose, is at least 1.0 wt.-%, more preferably at least 2.0 wt.-%, still more preferably at least 2.5 wt.-%, yet more preferably at least 3.0 wt.-%, even more preferably at least 3.5 wt.-%, most preferably at least 4.0 wt.-%, and in particular at least 4.5 wt.-%, based on the total weight of the pharmaceutical dosage form and/or based on the total weight of the particles.

Preferably, the content of the gelling agent, preferably hydroxyethylcellulose, is at most 16 wt.-%, more preferably at most 15 wt.-%, still more preferably at most 14 wt.-%, even more preferably at most 13 wt.-%, most preferably at most 12 wt.-%, and in particular at most 11 wt.-%, based on the total weight of the pharmaceutical dosage form and/or based on the total weight of the particles.

Preferably, the content of the gelling agent, preferably hydroxyethylcellulose, is within the range of 7.5±7.0 wt.-%, more preferably 7.5±6.0 wt.-%, still more preferably 7.5±5.0 wt.-%, yet more preferably 7.5±4.5 wt.-%, even more preferably 7.5±4.0 wt.-%, most preferably 7.5±3.5 wt.-%, and in particular 7.5±3.0 wt.-%, based on the total weight of the pharmaceutical dosage form and/or based on the total weight of the particles. Preferably, the content of the gelling agent is within the range of 7.5±7.0 wt.-%, preferably 7.5±5.0 wt.-%, in each case based on the total weight of the particles.

Preferably, the relative weight ratio of disintegrant : gelling agent is within the range of from 10.5:1 to 3.5:1, more preferably 10:1 to 4:1, still more preferably 9.5:1 to 4.5:1, yet more preferably 9:1 to 5:1, even more preferably 8.5:1 to 5.5:1, most preferably 8:1 to 6:1, and in particular 7.5:1 to 6.5:1.

The pharmaceutical dosage form and/or the particles according to the invention may contain additional pharmaceutical excipients conventionally contained in pharmaceutical dosage forms in conventional amounts, such as antioxidants, preservatives, lubricants, plasticizer, fillers, binders, and the like.

The skilled person will readily be able to determine appropriate further excipients as well as the quantities of each of these excipients. Specific examples of pharmaceutically acceptable carriers and excipients that may be used to formulate the pharmaceutical dosage forms according to the invention are described in the Handbook of Pharmaceutical Excipients, American Pharmaceutical Association (1986).

Preferably, the pharmaceutical dosage form and/or the particles according to the invention further comprise an antioxidant. Suitable antioxidants include ascorbic acid, butylated hydroxyanisole (BHA), butylated hydroxytoluene (BHT), salts of ascorbic acid, monothioglycerol, phosphorous acid, vitamin E and the derivatives thereof, coniferyl benzoate, nordihydroguajaretic acid, gallus acid esters, sodium bisulfite, particularly preferably butylated hydroxytoluene, butylated hydroxyanisole or alpha-tocopherol.

The antioxidant is preferably present in quantities of 0.01 wt.-% to 10 wt.-%, more preferably of 0.03 wt.-% to 5 wt.-%, most preferably of 0.05 wt.-% to 2.5 wt.-%, based on the total weight of the pharmaceutical dosage form and/or based on the total weight of the particles. Preferably, the content of the antioxidant is within the range of 0.20±0.15 wt.-%, preferably 0.20±0.10 wt.-%, in each case on the total weight of the particles.

In a preferred embodiment, the pharmaceutical dosage form and/or the particles according to the invention further comprise an acid, preferably an organic carboxylic acid, more preferably an organic carboxylic acid containing more than 1 carboxylic acid group, in particular citric acid.

Preferred organic carboxylic acids include but are not limited to dicarboxylic acids and tricarboxylic acids, and more preferably hydroxy dicarboxylic acids or hydroxy tricarboxylic acids such as citric acid, tartaric acid, fumaric acid, maleic acid and other similar acids. Citric acid is most preferred. Preferably, the acid is an organic carboxylic acid; more preferably a dicarboxylic acid or a tricarboxylic acid; still more preferably a hydroxy dicarboxylic acid or a hydroxy tricarboxylic acid; in particular citric acid.

The amount of acid is preferably in the range of 0.01 wt.-% to 20 wt.-%, more preferably in the range of 0.02 wt.-% to 10 wt.-%, and still more preferably in the range of 0.05 wt.-% to 5 wt.-%, and most preferably in the range of 0.1 wt.-% to 1.0 wt.-%, based on the total weight of the pharmaceutical dosage form and/or based on the total weight of the particles. Preferably, the content of the acid is within the range of 0.8±0.7 wt.-%, preferably 0.8±0.5 wt.-%, in each case based on the total weight of the particles.

Preferably, the pharmaceutical dosage form and/or the particles according to the invention further comprise a plasticizer. The plasticizer improves the processability of the polyalkylene oxide. A preferred plasticizer is polyalkylene glycol, like polyethylene glycol, triacetin, fatty acids, fatty acid esters, waxes and/or microcrystalline waxes. Particularly preferred plasticizers are polyethylene glycols having a weight average molecular weight within the range of from 4000 to 10,000 g/mol, preferably from 5400 to 6600 g/mol, such as PEG 6000 (Macrogol 6000).

Preferably, the content of the plasticizer is within the range of from 0.1 to 30 wt.-%, more preferably 0.2 to 25 wt.-%, still more preferably 0.3 wt.-% to 22.5 wt.-%, yet more preferably 0.4 wt.-% to 20 wt.-%, most preferably 0.5 to 20 wt.-% and in particular 0.6 wt.-% to 17.5 wt.-%, based on the total weight of the pharmaceutical dosage form and/or based on the total weight of the particles. Preferably, the content of the plasticizer is within the range of 2.0±1.5 wt.-%, preferably 2.0±1.0 wt.-%, in each case based on the total weight of the particles.

In a preferred embodiment, the relative weight ratio of the polyalkylene oxide to the polyalkylene glycol is within the range of 25±20 : 1, more preferably 25±15 : 1, still more preferably 25±10 : 1, yet more preferably 25±7.5 : 1, most preferably 25±5 : 1, and in particular 25±2.5 : 1. This ratio still satisfies the requirements of relative high polyalkylene oxide content and good extrudability.

Plasticizers can sometimes act as a lubricant, and lubricants can sometimes act as a plasticizer.

In a preferred embodiment, the pharmaceutical dosage form contains at least one lubricant. Preferably, the lubricant is contained in the pharmaceutical dosage form outside the particles, i.e. the particles as such preferably do not contain lubricant. In another preferred embodiment, the pharmaceutical dosage form contains no lubricant. Especially preferred lubricants are selected from
- magnesium stearate and stearic acid;
- glycerides of fatty acids, including monoglycerides, diglycerides, triglycerides, and mixtures thereof; preferably of C₆ to C₂₂ fatty acids; especially preferred are partial glycerides of the C₁₆ to C₂₂ fatty acids such as glycerol behenat, glycerol palmitostearate and glycerol monostearate;
- polyoxyethylene glycerol fatty acid esters, such as mixtures of mono-, di- and triesters of glycerol and di- and monoesters of macrogols having molecular weights within the range of from 200 to 4000 g/mol, e.g., macrogolglycerolcaprylocaprate, macrogolglycerollaurate, macrogolglycerolococoate, macrogolglycerollinoleate, macrogol-20-glycerolmonostearate, macrogol-6-glycerolcaprylocaprate, macrogolglycerololeate; macrogolglycerolstearate, macrogolglycerolhydroxystearate, and macrogolglycerolrizinoleate;
- polyglycolyzed glycerides, such as the one known and commercially available under the trade name "Labrasol";
- fatty alcohols that may be linear or branched, such as cetylalcohol, stearylalcohol, cetylstearyl alcohol, 2-octyldodecane-1-ol and 2-hexyldecane-1-ol;
- polyethylene glycols having a molecular weight between 10.000 and 60.000 g/mol; and
- natural semi-synthetic or synthetic waxes, preferably waxes with a softening point of at least 50 °C, more preferably 60 °C, and in particular carnauba wax and bees wax.

Preferably, the amount of the lubricant ranges from 0.01 wt.-% to 10 wt.-%, more preferably in the range of 0.05 wt.-% to 7.5 wt.-%, most preferably in the range of 0.1 wt.-% to 5 wt.-%, and in particular in the range of 0.1 wt.-% to 1 wt.-%, based on the total weight of the pharmaceutical dosage form and/or based on the total weight of the particles.

In preferred compositions of the particles that are preferably hot-melt extruded and that are contained in the pharmaceutical dosage form according to the invention, the pharmacologically active ingredient is an opioid, preferably oxycodone hydrochloride, the polyalkylene oxide is a polyethylene oxide with a weight average molecular weight within the range of from 200,000 to 2,000,000 g/mol, , preferably from 500,000 to 1,500,000 g/mol, and the disintegrant is preferably crosslinked sodium carboxymethylcellulose (Na-CMC). Particularly preferred embodiments A¹ to A³² are summarized in the tables here below (all percentages relative to the total weight of the particles):

| [wt.-%] | A¹ | A² | A³ | A⁴ | A⁵ | A⁶ | A⁷ | A⁸ |
|---|---|---|---|---|---|---|---|---|
| opioid | 5.5±5.0 | 5.5±5.0 | 5.5±4.5 | 5.5±4.5 | 5.5±4.0 | 5.5±4.0 | 5.5±3.5 | 5.5±3.5 |
| polyethylene oxide | 52±25 | 52±25 | 52±20 | 52±20 | 52±15 | 52±15 | 52±10 | 52±10 |
| disintegrant | 35±15 | 35±13 | 35±11 | 35±9 | 35±7 | 35±5 | 35±4 | 35±3 |
| | | | | | | | | |

| [wt.-%] | A⁹ | A¹⁰ | A¹¹ | A¹² | A¹³ | A¹⁴ | A¹⁵ | A¹⁶ |
|---|---|---|---|---|---|---|---|---|
| oxycodone HCl | 5.5±5.0 | 5.5±5.0 | 5.5±4.5 | 5.5±4.5 | 5.5±4.0 | 5.5±4.0 | 5.5±3.5 | 5.5±3.5 |
| polyethylene oxide | 52±25 | 52±25 | 52±20 | 52±20 | 52±15 | 52±15 | 52±10 | 52±10 |
| disintegrant | 35±15 | 35±13 | 35±11 | 35±9 | 35±7 | 35±5 | 35±4 | 35±3 |
| | | | | | | | | |

| [wt.-%] | A¹⁷ | A¹⁸ | A¹⁹ | A²⁰ | A²¹ | A²² | A²³ | A²⁴ |
|---|---|---|---|---|---|---|---|---|
| opioid | 5.5±5.0 | 5.5±5.0 | 5.5±4.5 | 5.5±4.5 | 5.5±4.0 | 5.5±4.0 | 5.5±3.5 | 5.5±3.5 |
| polyethylene oxide | 52±25 | 52±25 | 52±20 | 52±20 | 52±15 | 52±15 | 52±10 | 52±10 |
| Na-CMC | 35±15 | 35±13 | 35±11 | 35±9 | 35±7 | 35±5 | 35±4 | 35±3 |

| [wt.-%] | A²⁵ | A²⁶ | A²⁷ | A²⁸ | A²⁹ | A³⁰ | A³¹ | A³² |
|---|---|---|---|---|---|---|---|---|
| oxycodone HCl | 5.5±5.0 | 5.5±5.0 | 5.5±4.5 | 5.5±4.5 | 5.5±4.0 | 5.5±4.0 | 5.5±3.5 | 5.5±3.5 |
| polyethylene oxide | 52±25 | 52±25 | 52±20 | 52±20 | 52±15 | 52±15 | 52±10 | 52±10 |
| Na-CMC | 35±15 | 35±13 | 35±11 | 35±9 | 35±7 | 35±5 | 35±4 | 35±3 |

In preferred compositions of the particles that are preferably hot-melt extruded and that are contained in the pharmaceutical dosage form according to the invention, the pharmacologically active ingredient is an opioid, preferably oxycodone hydrochloride, the polyalkylene oxide is a polyethylene oxide with a weight average molecular weight within the range of from 200,000 to 2,000,000 g/mol, preferably from 500,000 to 1,500,000 g/mol, the disintegrant is preferably crosslinked sodium carboxymethylcellulose (Na-CMC), and a gelling agent is present, preferably hydroxyethylcellulose (HEC). Particularly preferred embodiments B¹ to B⁴⁰ are summarized in the tables here below (all percentages relative to the total weight of the particles):

| [wt.-%] | B¹ | B² | B³ | B⁴ | B⁵ | B⁶ | B⁷ | B⁸ |
|---|---|---|---|---|---|---|---|---|
| opioid | 5.5±5.0 | 5.5±5.0 | 5.5±4.5 | 5.5±4.5 | 5.5±4.0 | 5.5±4.0 | 5.5±3.5 | 5.5±3.5 |
| polyethylene oxide | 52±25 | 52±25 | 52±20 | 52±20 | 52±15 | 52±15 | 52±10 | 52±10 |
| disintegrant | 35±15 | 35±13 | 35±11 | 35±9 | 35±7 | 35±5 | 35±4 | 35±3 |
| gelling agent | 7.5±7.0 | 7.5±7.0 | 7.5±6.0 | 7.5±6.0 | 7.5±5.0 | 7.5±5.0 | 7.5±4.0 | 7.5±4.0 |
| | | | | | | | | |

| [wt.-%] | B⁹ | B¹⁰ | B¹¹ | B¹² | B¹³ | B¹⁴ | B¹⁵ | B¹⁶ |
|---|---|---|---|---|---|---|---|---|
| oxycodone HCl | 5.5±5.0 | 5.5±5.0 | 5.5±4.5 | 5.5±4.5 | 5.5±4.0 | 5.5±4.0 | 5.5±3.5 | 5.5±3.5 |
| polyethylene oxide | 52±25 | 52±25 | 52±20 | 52±20 | 52±15 | 52±15 | 52±10 | 52±10 |
| disintegrant | 35±15 | 35±13 | 35±11 | 35±9 | 35±7 | 35±5 | 35±4 | 35±3 |
| gelling agent | 7.5±7.0 | 7.5±7.0 | 7.5±6.0 | 7.5±6.0 | 7.5±5.0 | 7.5±5.0 | 7.5±4.0 | 7.5±4.0 |
| | | | | | | | | |

| [wt.-%] | B¹⁷ | B¹⁸ | B¹⁹ | B²⁰ | B²¹ | B²² | B²³ | B²⁴ |
|---|---|---|---|---|---|---|---|---|
| opioid | 5.5±5.0 | 5.5±5.0 | 5.5±4.5 | 5.5±4.5 | 5.5±4.0 | 5.5±4.0 | 5.5±3.5 | 5.5±3.5 |
| polyethylene oxide | 52±25 | 52±25 | 52±20 | 52±20 | 52±15 | 52±15 | 52±10 | 52±10 |
| Na-CMC | 35±15 | 35±13 | 35±11 | 35±9 | 35±7 | 35±5 | 35±4 | 35±3 |
| gelling agent | 7.5±7.0 | 7.5±7.0 | 7.5±6.0 | 7.5±6.0 | 7.5±5.0 | 7.5±5.0 | 7.5±3.0 | 7.5±3.0 |
| | | | | | | | | |

| [wt.-%] | B²⁵ | B²⁶ | B²⁷ | B²⁸ | B²⁹ | B³⁰ | B³¹ | B³² |
|---|---|---|---|---|---|---|---|---|
| oxycodone HCl | 5.5±5.0 | 5.5±5.0 | 5.5±4.5 | 5.5±4.5 | 5.5±4.0 | 5.5±4.0 | 5.5±3.5 | 5.5±3.5 |
| polyethylene oxide | 52±25 | 52±25 | 52±20 | 52±20 | 52±15 | 52±15 | 52±10 | 52±10 |
| Na-CMC | 35±15 | 35±13 | 35±11 | 35±9 | 35±7 | 35±5 | 35±4 | 35±3 |
| gelling agent | 7.5±7.0 | 7.5±7.0 | 7.5±6.0 | 7.5±6.0 | 7.5±5.0 | 7.5±5.0 | 7.5±4.0 | 7.5±4.0 |
| | | | | | | | | |

| [wt.-%] | B³³ | B³⁴ | B³⁵ | B³⁶ | B³⁷ | B³⁸ | B³⁹ | B⁴⁰ |
|---|---|---|---|---|---|---|---|---|
| oxycodone HCl | 5.5±5.0 | 5.5±5.0 | 5.5±4.5 | 5.5±4.5 | 5.5±4.0 | 5.5±4.0 | 5.5±3.5 | 5.5±3.5 |
| polyethylene oxide | 52±25 | 52±25 | 52±20 | 52±20 | 52±15 | 52±15 | 52±10 | 52±10 |
| Na-CMC | 35±15 | 35±13 | 35±11 | 35±9 | 35±7 | 35±5 | 35±4 | 35±3 |
| HEC | 7.5±4.5 | 7.5±4.5 | 7.5±4.0 | 7.5±4.0 | 7.5±3.5 | 7.5±3.5 | 7.5±4.0 | 7.5±4.0 |

The pharmaceutical dosage form according to the invention preferably contains no antagonists for the pharmacologically active compound, preferably no antagonists against psychotropic substances, in particular no antagonists against opioids. Antagonists suitable for a given pharmacologically active compound are known to the person skilled in the art and may be present as such or in the form of corresponding derivatives, in particular esters or ethers, or in each case in the form of corresponding physiologically acceptable compounds, in particular in the form of the salts or solvates thereof. The pharmaceutical dosage form according to the invention preferably contains no antagonists selected from among the group comprising naloxone, naltrexone, nalmefene, nalide, nalmexone, nalorphine or naluphine, in each case optionally in the form of a corresponding physiologically acceptable compound, in particular in the form of a base, a salt or solvate; and no neuroleptics, for example a compound selected from among the group comprising haloperidol, promethacine, fluphenazine, perphenazine, levomepromazine, thioridazine, perazine, chlorpromazine, chlorprothixine, zuclopenthixol, flupentixol, prothipendyl, zotepine, benperidol, pipamperone, melperone and bromperidol.

Further, the pharmaceutical dosage form according to the invention preferably also contains no bitter substance. Bitter substances and the quantities effective for use may be found in US-2003/0064099 A1, the corresponding disclosure of which should be deemed to be the disclosure of the present application and is hereby introduced as a reference. Examples of bitter substances are aromatic oils, such as peppermint oil, eucalyptus oil, bitter almond oil, menthol, fruit aroma substances, aroma substances from lemons, oranges, limes, grapefruit or mixtures thereof, and/or denatonium benzoate.

The pharmaceutical dosage form according to the invention accordingly preferably contains neither antagonists for the pharmacologically active compound nor bitter substances.

In a preferred embodiment, the pharmaceutical dosage form according to the invention is a tablet, wherein the particles are contained in a matrix of a matrix material. In the following, this preferred embodiment is referred to as the "preferred tablet according to the invention".

The preferred tablet according to the invention comprises subunits having different morphology and properties, namely drug-containing particles and matrix material, wherein the particles form a discontinuous phase within the matrix material. The particles typically have mechanical properties that differ from the mechanical properties of the matrix material. Preferably, the particles have a higher mechanical strength than the matrix material. The particles within the preferred tablet according to the invention can be visualized by conventional means such as solid state nuclear magnetic resonance spectroscopy, raster electron microscopy, terahertz spectroscopy and the like.

In the preferred tablet according to the invention, the particles are incorporated in a matrix material. From a macroscopic perspective, the matrix material preferably forms a continuous phase in which the particles are embedded as discontinuous phase.

Preferably, the matrix material is a homogenous coherent mass, preferably a homogeneous mixture of solid constituents, in which the particles are embedded thereby spatially separating the particles from one another. While it is possible that the surfaces of particles are in contact or at least in very close proximity with one another, the plurality of particles preferably cannot be regarded as a single continuous coherent mass within the preferred tablet according to the invention.

In other words, the preferred tablet according to the invention comprises the particles as volume element(s) of a first type in which the pharmacologically active compound, the polyalkylene oxide and the disintegrant are contained, preferably homogeneously, and the matrix material as volume element of a second type differing from the material that forms the particles, preferably containing neither pharmacologically active compound nor polyalkylene oxide, but optionally polyethylene glycol which differs from polyethylene oxide in its molecular weight.

A purpose of the matrix material in the preferred tablet according to the invention is to ensure rapid disintegration and subsequent release of the pharmacologically active compound from the disintegrated preferred tablet according to the invention, i.e. from the particles. Thus, the matrix material preferably does not contain any excipient that might have a retardant effect on disintegration and drug release, respectively. Thus, the matrix material preferably does not contain any polymer that is typically employed as matrix material in prolonged release formulations.

The preferred tablet according to the invention preferably comprises the matrix material in an amount of more than one third of the total weight of the preferred tablet according to the invention. Thus, the polyalkylene oxide that is contained in the particles of the preferred tablet according to the invention is preferably not also contained in the matrix material.

Preferably, the pharmacologically active compound which is contained in the particles of the preferred tablet according to the invention is preferably not also contained in the matrix material. Thus, in a preferred embodiment, the total amount of pharmacologically active compound contained in the preferred tablet according to the invention is present in the particles which form a discontinuous phase within the matrix material; and the matrix material forming a continuous phase does not contain any pharmacologically active compound.

Suitable matrix materials are known to the skilled person and include but are not limited to

Preferably, the matrix material comprises a disintegrant in combination with one or more water insoluble pharmaceutical excipients, preferably fillers/binders and/or lubricants and/or disintegrants.

Preferably, the matrix material comprises a filler or a binder. As many fillers can be regarded as binders and vice versa, for the purpose of the specification "filler/binder" refers to any excipient that is suitable as filler, binder or both. Thus, the matrix material preferably comprises a filler/binder.

Preferred fillers (=filler/binders) are selected from the group consisting of silicium dioxide (e.g. Aerosil®), microcrystalline cellulose (e.g. Avicel®, Elcema®, Emocel®, ExCel®, Vitacell®); cellulose ether (e.g. Natrosol®, Klucel®, Methocel®, Blanose®, Pharmacoat®, Viscontran®); mannitol; dextrines; dextrose; calciumhydrogen phosphate (e.g. Emcompress®); maltodextrine (e.g. Emdex®); lactose (e.g. Fast-Flow Lactose®; Ludipress®' Tablettose®, Zeparox®); polyvinylpyrrolidone (PVP) (e.g. Kollidone®, Polyplasdone®, Polydone®); saccharose (e.g. Nu-Tab®, Sugar Tab®); magnesium salts (e.g. MgCO₃, MgO, MgSiO₃); starches and pretreated starches (e.g. Prejel®, Primotab® ET, Starch® 1500). Preferred binders are selected from the group consisting of alginates; chitosanes; and any of the fillers mentioned above (= fillers/binders).

Some fillers/binders may also serve other purposes. It is known, for example, that silicium dioxide exhibits excellent function as a glidant. Thus, preferably, the matrix material comprises a glidant such as silicium dioxide. In a preferred embodiment, the content of the filler/binder or mixture of fillers/binders in the matrix material is within the range of 50±25 wt.-%, more preferably 50±20 wt.-%, still more preferably 50±15 wt.-%, yet more preferably 50±10 wt.-%, most preferably 50±7.5 wt.-%, and in particular 50±5 wt.-%, based on the total weight of matrix material. In another preferred embodiment, the content of the filler/binder or mixture of fillers/binders in the matrix material is within the range of 65±25 wt.-%, more preferably 65±20 wt.-%, still more preferably 65±15 wt.-%, yet more preferably 65±10 wt.-%, most preferably 65±7.5 wt.-%, and in particular 65±5 wt.-%, based on the total weight of matrix material. In still another preferred embodiment, the content of the filler/binder or mixture of fillers/binders in the matrix material is within the range of 80±19 wt.-%, more preferably 80±17.5 wt.-%, still more preferably 80±15 wt.-%, yet more preferably 80±10 wt.-%, most preferably 80±7.5 wt.-%, and in particular 80±5 wt.-%, based on the total weight of matrix material. In another preferred embodiment, the content of the filler/binder or mixture of fillers/binders in the matrix material is within the range of 90±9 wt.-%, more preferably 90±8 wt.-%, still more preferably 90±7 wt.-%, yet more preferably 90±6 wt.-%, most preferably 90±5 wt.-%, and in particular 90±4 wt.-%, based on the total weight of matrix material.

In a preferred embodiment, the content of the filler/binder or mixture of fillers/binders in the tablet is within the range of 25±24 wt.-%, more preferably 25±20 wt.-%, still more preferably 25±16 wt.-%, yet more preferably 25±12 wt.-%, most preferably 25±8 wt.-%, and in particular 25±4 wt.-%, based on the total weight of tablet. In another preferred embodiment, the content of the filler/binder or mixture of fillers/binders in the tablet is within the range of 30±29 wt.-%, more preferably 30±25 wt.-%, still more preferably 30±20 wt.-%, yet more preferably 30±15 wt.-%, most preferably 30±10 wt.-%, and in particular 30±5 wt.-%, based on the total weight of tablet. In still another preferred embodiment, the content of the filler/binder or mixture of fillers/binders in the tablet is within the range of 35±34 wt.-%, more preferably 35±28 wt.-%, still more preferably 35±22 wt.-%, yet more preferably 35±16 wt.-%, most preferably 35±10 wt.-%, and in particular 35±4 wt.-%, based on the total weight of tablet. In another preferred embodiment, the content of the filler/binder or mixture of fillers/binders in the tablet is within the range of 40±39 wt.-%, more preferably 40±32 wt.-%, still more preferably 40±25 wt.-%, yet more preferably 40±18 wt.-%, most preferably 40±11 wt.-%, and in particular 40±4 wt.-%, based on the total weight of tablet.

Preferably, the filler/binder is contained in the matrix material but not in the particles of the tablet according to the invention. In a preferred embodiment, a portion (e.g. 10% of the total tablet mass) of the matrix is granulated on the particles (preferably by non-aqueous wet granulation, e.g. with isopropylic alcohol) and the remaining matrix material is added to the thus granulated particles and blended prior to compression / processing to tablets. Thus, according to this embodiment, the particles are coated by a portion of the matrix material, whereas the remainder of the matrix material is preferably employed in non-granulated form.

Preferably, the matrix material comprises a diluent or lubricant, preferably selected from the group consisting of calcium stearate; magnesium stearate; glycerol monobehenate (e.g. Compritol®); Myvatex®; Precirol®; Precirol® Ato5; sodium stearylfumarate (e.g. Pruv®); and talcum. Magnesium stearate is particularly preferred. Preferably, the content of the lubricant in the matrix material is at most 10.0 wt.-%, more preferably at most 7.5 wt.-%, still more preferably at most 5.0 wt.-%, yet more preferably at most 2.0 wt.-%, even more preferably at most 1.0 wt.-%, and most preferably at most 0.5 wt.-%, based on the total weight of the matrix material and/or based on the total weight of tablet.

In particularly preferred embodiment, the matrix material comprises a combination of disintegrant, filler/binder and lubricant.

The matrix material of the tablets according to the invention may additionally contain other excipients that are conventional in the art, e.g. diluents, binders, granulating aids, colorants, flavourants, pore formers, surfactants, glidants, wet-regulating agents and disintegrants. The skilled person will readily be able to determine appropriate quantities of each of these excipients. Preferred pore formers include, but are not limited to glucose, fructose, mannitol, mannose. galactose, sorbitol, pullulan, dextran, water-soluble hydrophilic polymers, hydroxyalkyl- celluloses, carboxyalkylcelluloses, hydroxypropylmethylcellulose, cellulose ethers, acrylic resins, polyvinylpyrrolidone, cross-linked polyvinylpyrrolidone, polyethylene oxide, carbowaxes, carbopol, diols, polyols, polyhydric alcohols, polyalkylene glycols, polyethylene glycols, polypropylene glycols or block polymers thereof, polyglycols, poly(a-co)alkylenediols; inorganic compounds; alkali metal salts; alkaline earth metal salts, or combinations thereof.

Preferred surfactants are nonionic, anionic, cationic or amphoteric surfactants.

In a preferred embodiment, however, the matrix material of the tablet according to the invention consists of one or more disintegrants, one or more filler/binder's and one or more lubricants, but does not contain any other constituents.

In a particularly preferred embodiment, the matrix material of the tablet according to the invention does not contain one or more gelling agents and/or a silicone.

The polyalkylene oxide that is contained in the particles of the tablets according to the invention is preferably not also contained in the matrix material.

Preferably, the pharmacologically active compound which is contained in the particles of the tablet according to the invention is preferably not also contained in the matrix material. Thus, in a preferred embodiment, the total amount of pharmacologically active compound contained in the tablet according to the invention is present in the particles which form a discontinuous phase within the matrix material; and the matrix material forming a continuous phase does not contain any pharmacologically active compound.

Preferably, the content of the matrix material is at least 10 wt.-%, at least 12.5 wt.-%, at least 15 wt.-%, at least 17.5 wt.-%, at least 20 wt.-%, at least 22.5 wt.-%, at least 25 wt.-%, at least 27.5 wt.-%, at least 30 wt.-%, at least 32.5 wt.-%, at least 35 wt.-%, at least 37.5 wt.-% or at least 40 wt.-%; more preferably at least 42.5 wt.-%, at least 45 wt.-%, at least 47.5 wt.-% or at least 50 wt.-%; still more preferably at least 52.5 wt.-%, at least 55 wt.-%, at least 57.5 wt.-% or at least 60 wt.-%; yet more preferably at least 62.5 wt.-%, at least 65 wt.-%, at least 67.5 wt.-% or at least 60 wt.-%; most preferably at least 72.5 wt.-%, at least 75 wt.-%, at least 77.5 wt.-% or at least 70 wt.-%; and in particular at least 82.5 wt.-%, at least 85 wt.-%, at least 87.5 wt.-% or at least 90 wt.-%; based on the total weight of the preferred tablet according to the invention.

Preferably, the content of the matrix material is at most 90 wt.-%, at most 87.5 wt.-%, at most 85 wt.-%, or at most 82.5 wt.-%; more preferably at most 80 wt.-%, at most 77.5 wt.-%, at most 75 wt.-% or at most 72.5 wt.-%; still more preferably at most 70 wt.-%, at most 67.5 wt.-%, at most 65 wt.-% or at most 62.5 wt.-%; yet more preferably at most 60 wt.-%, at most 57.5 wt.-%, at most 55 wt.-% or at most 52.5 wt.-%; most preferably at most 50 wt.-%, at most 47.5 wt.-%, at most 45 wt.-% or at most 42.5 wt.-%; and in particular at most 40 wt.-%, at most 37.5 wt.-%, or at most 35 wt.-%; based on the total weight of the preferred tablet according to the invention.

In a preferred embodiment, the content of the matrix material is within the range of 20±10 wt.-%, more preferably 20±7.5 wt.-%, still more preferably 20±5 wt.-%, and most preferably 20±2.5 wt.-%,, based on the total weight of the preferred tablet according to the invention.

In another preferred embodiment, the content of the matrix material is within the range of 25±10 wt.-%, more preferably 25±7.5 wt.-%, still more preferably 25±5 wt.-%, and most preferably 25±2.5 wt.-%, based on the total weight of the preferred tablet according to the invention.

In still another preferred embodiment, the content of the matrix material is within the range of 30±10 wt.-%, more preferably 30±7.5 wt.-%, still more preferably 30±5 wt.-%, and most preferably 30±2.5 wt.-%, based on the total weight of the preferred tablet according to the invention.

In yet another preferred embodiment, the content of the matrix material is within the range of 35±10 wt.-%, more preferably 35±7.5 wt.-%, still more preferably 35±5 wt.-%, and most preferably 35±2.5 wt.-%, based on the total weight of the preferred tablet according to the invention.

In a preferred embodiment, the content of the matrix material is within the range of 40±10 wt.-%, more preferably 40±7.5 wt.-%, still more preferably 40±5 wt.-%, and most preferably 40±2.5 wt.-%, based on the total weight of the preferred tablet according to the invention.

In another preferred embodiment, the content of the matrix material is within the range of 45±10 wt.-%, more preferably 45±7.5 wt.-%, still more preferably 45±5 wt.-%, and most preferably 45±2.5 wt.-%, based on the total weight of the preferred tablet according to the invention.

In still another preferred embodiment, the content of the matrix material is within the range of 50±10 wt.-%, more preferably 50±7.5 wt.-%, still more preferably 50±5 wt.-%, and most preferably 50±2.5 wt.-%, based on the total weight of the preferred tablet according to the invention.

In yet another preferred embodiment, the content of the matrix material is within the range of 55±10 wt.-%, more preferably 55±7.5 wt.-%, still more preferably 55±5 wt.-%, and most preferably 55±2.5 wt.-%, based on the total weight of the preferred tablet according to the invention.

Preferably, the matrix material is a mixture, preferably a homogeneous mixture of at least two different constituents, more preferably of at least three different constituents. In a preferred embodiment, all constituents of the matrix material are homogeneously distributed in the continuous phase that is formed by the matrix material.

The pharmaceutical dosage form according to the invention is tamper-resistant.

As used herein, the term "tamper-resistant" refers to pharmaceutical dosage forms that are resistant to conversion into a form suitable for misuse or abuse, particular for nasal and/or intravenous administration, by conventional means such as grinding in a mortar or crushing by means of a hammer. In this regard, the pharmaceutical dosage forms as such may be crushable by conventional means. However, the particles contained in the pharmaceutical dosage forms according to the invention preferably exhibit mechanical properties such that they cannot be pulverized by conventional means any further. As the particles are of macroscopic size and contain the pharmacologically active compound, they cannot be administered nasally thereby rendering the pharmaceutical dosage forms tamper-resistant.

Preferably, when trying to tamper the dosage form in order to prepare a formulation suitable for abuse by intravenous administration, the liquid part of the formulation that can be separated from the remainder by means of a syringe is as less as possible, preferably it contains not more than 20 wt.-%, more preferably not more than 15 wt.-%, still more preferably not more than 10 wt.-%, and most preferably not more than 5 wt.-% of the originally contained pharmacologically active compound. Preferably, this property is tested by (i) dispensing a pharmaceutical dosage form that is either intact or has been manually comminuted by means of two spoons in 5 ml of purified water, (ii) heating the liquid up to its boiling point, (iii) boiling the liquid in a covered vessel for 5 min without the addition of further purified water, (iv) drawing up the hot liquid into a syringe (needle 21G equipped with a cigarette filter), (v) determining the amount of the pharmacologically active compound contained in the liquid within the syringe.

Further, when trying to disrupt the pharmaceutical dosage forms by means of a hammer or mortar, the particles tend to adhere to one another thereby forming aggregates and agglomerates, respectively, which are larger in size than the untreated particles.

Preferably, tamper-resistance is achieved based on the mechanical properties of the particles so that comminution is avoided or at least substantially impeded. According to the invention, the term comminution means the pulverization of the particles using conventional means usually available to an abuser, for example a pestle and mortar, a hammer, a mallet or other conventional means for pulverizing under the action of force. Thus, tamper-resistance preferably means that pulverization of the particles using conventional means is avoided or at least substantially impeded.

Preferably, the mechanical properties of the particles according to the invention, particularly their breaking strength and deformability, substantially rely on the presence and spatial distribution of polyalkylene oxide, although their mere presence does typically not suffice in order to achieve said properties. The advantageous mechanical properties of the particles according to the invention may not automatically be achieved by simply processing pharmacologically active compound, polyalkylene oxide, and optionally further excipients by means of conventional methods for the preparation of pharmaceutical dosage forms. In fact, usually suitable apparatuses must be selected for the preparation and critical processing parameters must be adjusted, particularly pressure/force, temperature and time. Thus, even if conventional apparatuses are used, the process protocols usually must be adapted in order to meet the required criteria.

In general, the particles exhibiting the desired properties may be obtained only if, during preparation of the particles,
- suitable components
- in suitable amounts
   are exposed to
- a sufficient pressure
- at a sufficient temperature
- for a sufficient period of time.

Thus, regardless of the apparatus used, the process protocols must be adapted in order to meet the required criteria. Therefore, the breaking strength and deformability of the particles is separable from the composition.

The particles contained in the pharmaceutical dosage form according to the invention preferably have a breaking strength of at least 300 N, at least 400 N, or at least 500 N, preferably at least 600 N, more preferably at least 700 N, still more preferably at least 800 N, yet more preferably at least 1000 N, most preferably at least 1250 N and in particular at least 1500 N.

In order to verify whether a particle exhibits a particular breaking strength of e.g. 300 N or 500 N it is typically not necessary to subject said particle to forces much higher than 300 N and 500 N, respectively. Thus, the breaking strength test can usually be terminated once the force corresponding to the desired breaking strength has been slightly exceeded, e.g. at forces of e.g. 330 N and 550 N, respectively.

The "breaking strength" (resistance to crushing) of a pharmaceutical dosage form and of a particle is known to the skilled person. In this regard it can be referred to, e.g., W.A. Ritschel, Die Tablette, 2. Auflage, Editio Cantor Verlag Aulendorf, 2002; H Liebermann et al., Pharmaceutical dosage forms: Pharmaceutical dosage forms, Vol. 2, Informa Healthcare; 2 edition, 1990; and Encyclopedia of Pharmaceutical Technology, Informa Healthcare; 1 edition.

For the purpose of the specification, the breaking strength is preferably defined as the amount of force that is necessary in order to fracture the particle (= breaking force). Therefore, for the purpose of the specification a particle does preferably not exhibit the desired breaking strength when it breaks, i.e., is fractured into at least two independent parts that are separated from one another. In another preferred embodiment, however, the particle is regarded as being broken if the force decreases by 50% (threshold value) of the highest force measured during the measurement (see below).

The particles according to the invention are distinguished from conventional particles that can be contained in pharmaceutical dosage forms in that, due to their breaking strength, they cannot be pulverized by the application of force with conventional means, such as for example a pestle and mortar, a hammer, a mallet or other usual means for pulverization, in particular devices developed for this purpose (tablet crushers). In this regard "pulverization" means crumbling into small particles. Avoidance of pulverization virtually rules out oral or parenteral, in particular intravenous or nasal abuse.

Conventional particles typically have a breaking strength well below 200 N.

The breaking strength of conventional round pharmaceutical dosage forms/particles may be estimated according to the following empirical formula: Breaking Strength [in N] = 10 x Diameter Of The Pharmaceutical dosage form/Particle [in mm]. Thus, according to said empirical formula, a round pharmaceutical dosage form/particle having a breaking strength of at least 300 N would require a diameter of at least 30 mm). Such a particle, however, could not be swallowed, let alone a pharmaceutical dosage form containing a plurality of such particles. The above empirical formula preferably does not apply to the particles according to the invention, which are not conventional but rather special.

Further, the actual mean chewing force is 220 N (cf., e.g., P.A. Proeschel et al., J Dent Res, 2002, 81(7), 464-468). This means that conventional particles having a breaking strength well below 200 N may be crushed upon spontaneous chewing, whereas the particles according to the invention may preferably not.

Still further, when applying a gravitational acceleration of 9.81 m/s², 300 N correspond to a gravitational force of more than 30 kg, i.e. the particles according to the invention can preferably withstand a weight of more than 30 kg without being pulverized.

Methods for measuring the breaking strength of a pharmaceutical dosage form are known to the skilled artisan. Suitable devices are commercially available.

For example, the breaking strength (resistance to crushing) can be measured in accordance with the Eur. Ph. 5.0, 2.9.8 or 6.0, 2.09.08 "Resistance to Crushing of Pharmaceutical dosage forms". The test is intended to determine, under defined conditions, the resistance to crushing of pharmaceutical dosage forms and particles, respectively, measured by the force needed to disrupt them by crushing. The apparatus consists of 2 jaws facing each other, one of which moves towards the other. The flat surfaces of the jaws are perpendicular to the direction of movement. The crushing surfaces of the jaws are flat and larger than the zone of contact with the pharmaceutical dosage form and particle, respectively. The apparatus is calibrated using a system with a precision of 1 Newton. The pharmaceutical dosage form and particle, respectively, is placed between the jaws, taking into account, where applicable, the shape, the break-mark and the inscription; for each measurement the pharmaceutical dosage form and particle, respectively, is oriented in the same way with respect to the direction of application of the force (and the direction of extension in which the breaking strength is to be measured). The measurement is carried out on 10 pharmaceutical dosage forms and particles, respectively, taking care that all fragments have been removed before each determination. The result is expressed as the mean, minimum and maximum values of the forces measured, all expressed in Newton.

A similar description of the breaking strength (breaking force) can be found in the USP. The breaking strength can alternatively be measured in accordance with the method described therein where it is stated that the breaking strength is the force required to cause a pharmaceutical dosage form and particle, respectively, to fail (i.e., break) in a specific plane. The pharmaceutical dosage forms and particles, respectively, are generally placed between two platens, one of which moves to apply sufficient force to the pharmaceutical dosage form and particle, respectively, to cause fracture. For conventional, round (circular cross-section) pharmaceutical dosage forms and particles, respectively, loading occurs across their diameter (sometimes referred to as diametral loading), and fracture occurs in the plane. The breaking force of pharmaceutical dosage forms and particles, respectively, is commonly called hardness in the pharmaceutical literature; however, the use of this term is misleading. In material science, the term hardness refers to the resistance of a surface to penetration or indentation by a small probe. The term crushing strength is also frequently used to describe the resistance of pharmaceutical dosage forms and particle, respectively, to the application of a compressive load. Although this term describes the true nature of the test more accurately than does hardness, it implies that pharmaceutical dosage forms and particles, respectively, are actually crushed during the test, which is often not the case.

Alternatively, the breaking strength (resistance to crushing) can be measured in accordance with WO 2008/107149, which can be regarded as a modification of the method described in the Eur. Ph. The apparatus used for the measurement is preferably a "Zwick Z 2.5" materials tester, Fₘₐₓ = 2.5 kN with a maximum draw of 1150 mm, which should be set up with one column and one spindle, a clearance behind of 100 mm and a test speed adjustable between 0.1 and 800 mm/min together with testControl software. A skilled person knows how to properly adjust the test speed, e.g. to 10 mm/min, 20 mm/min, or 40 mm/min, for example. Measurement is performed using a pressure piston with screw-in inserts and a cylinder (diameter 10 mm), a force transducer, Fₘₐₓ. 1 kN, diameter = 8 mm, class 0.5 from 10 N, class 1 from 2 N to ISO 7500-1, with manufacturer's test certificate M according to DIN 55350-18 (Zwick gross force Fₘₐₓ = 1.45 kN) (all apparatus from Zwick GmbH & Co. KG, Ulm, Germany) with Order No BTC-FR 2.5 TH. D09 for the tester, Order No BTC-LC 0050N. P01 for the force transducer, Order No BO 70000 S06 for the centring device.

When using the testControl software (testXpert V10.11), the following exemplified settings and parameters have revealed to be useful: LE-position: clamping length 150 mm. LE-speed: 500 mm/min, clamping length after pre-travel: 195 mm, pre-travel speed: 500 mm/min, no pre-force control - pre-force: pre-force IN, pre-force speed 10 mm/min - sample data: no sample form, measuring length traverse distance 10 mm, no input required prior to testing - testing / end of test; test speed: position-controlled 10 mm/min, delay speed shift: 1, force shut down threshold 50% Fₘₐₓ, no force threshold for break-tests, no max length variation, upper force limit: 600N - expansion compensation: no correction of measuring length - actions after testing: LE to be set after test, no unload of sample - TRS: data memory: TRS distance interval until break 1, TRS time interval 0.1s, TRS force interval 1N - machine; traverse distance controller: upper soft end 358 mm, lower soft end 192 mm - lower test space. Parallel arrangement of the upper plate and the ambos should be ensured - these parts must not touch during or after testing. After testing, a small gap (e.g. 0.1 or 0.2 mm) should still be present between the two brackets in intimated contact with the tested particle, representing the remaining thickness of the deformed particle.

In a preferred embodiment, the particle is regarded as being broken if it is fractured into at least two separate pieces of comparable morphology. Separated matter having a morphology different from that of the deformed particle, e.g. dust, is not considered as pieces qualifying for the definition of breaking.

The particles according to the invention preferably exhibit mechanical strength over a wide temperature range, in addition to the breaking strength (resistance to crushing) optionally also sufficient hardness, yield strength, fatigue strength, impact resistance, impact elasticity, tensile strength, compressive strength and/or modulus of elasticity, optionally also at low temperatures (e.g. below -24 °C, below -40 °C or possibly even in liquid nitrogen), for it to be virtually impossible to pulverize by spontaneous chewing, grinding in a mortar, pounding, etc. Thus, preferably, the comparatively high breaking strength of the particle according to the invention is maintained even at low or very low temperatures, e.g., when the pharmaceutical dosage form is initially chilled to increase its brittleness, for example to temperatures below -25°C, below -40 °C or even in liquid nitrogen.

The particle according to the invention is characterized by a certain degree of breaking strength. This does not mean that the particle must also exhibit a certain degree of hardness. Hardness and breaking strength are different physical properties. Therefore, the tamper-resistance of the pharmaceutical dosage form does not necessarily depend on the hardness of the particles. For instance, due to its breaking strength, impact strength, elasticity modulus and tensile strength, respectively, the particles can preferably be deformed, e.g. plastically, when exerting an external force, for example using a hammer, but cannot be pulverized, i.e., crumbled into a high number of fragments. In other words, the particles according to the invention are characterized by a certain degree of breaking strength, but not necessarily also by a certain degree of form stability.

Therefore, in the meaning of the specification, a particle that is deformed when being exposed to a force in a particular direction of extension but that does not break (plastic deformation or plastic flow) is preferably to be regarded as having the desired breaking strength in said direction of extension.

Preferred particles present in the pharmaceutical dosage forms according to the invention are those having a suitable tensile strength as determined by a test method currently accepted in the art. Further preferred particles are those having a Youngs Modulus as determined by a test method of the art. Still further preferred particles are those having an acceptable elongation at break.

Irrespective of whether the particles according to the invention have an increased breaking strength or nor, the particles according to the invention preferably exhibit a certain degree of deformability. The particles contained in the pharmaceutical dosage form according to the invention preferably have a deformability such that they show an increase, preferably a substantially steady increase of the force at a corresponding decrease of the displacement in the force-displacement-diagram when being subjected to a breaking strength test as described above.

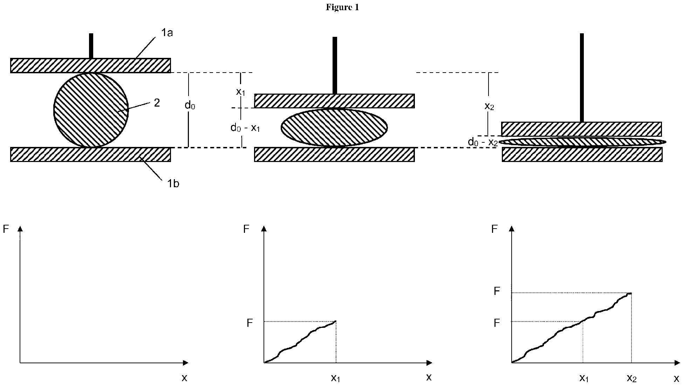

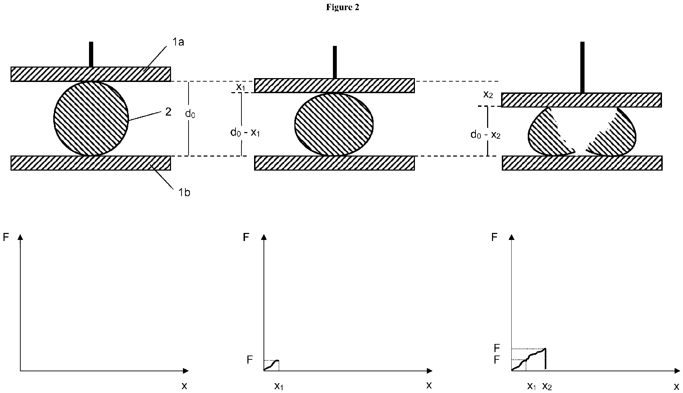

This mechanical property, i.e. the deformability of the individual particles, is illustrated in Figures 1 and 2.

Figure 1 schematically illustrates the measurement and the corresponding force-displacement-diagram. In particular, Figure 1A shows the initial situation at the beginning of the measurement. The sample particle (2) is placed between upper jaw (1a) and lower jaw (1b) which each are in intimate contact with the surface of the particle (2). The initial displacement do between upper jaw (1a) and lower jaw (1b) corresponds to the extension of the particle orthogonal to the surfaces of upper jaw (1a) and lower jaw (1b). At this time, no force is exerted at all and thus, no graph is displayed in the force-displacement-diagram below. When the measurement is commenced, the upper jaw is moved in direction of lower jaw (1b), preferably at a constant speed. Figure 1B shows a situation where due to the movement of upper jaw (1a) towards lower jaw (1b) a force is exerted on particle (2). Because of its deformability, the particle (2) is flattened without being fractured. The force-displacement-diagram indicates that after a reduction of the displacement do of upper jaw (1a) and lower jaw (1b) by distance x₁, i.e. at a displacement of d₁ = d₀ - x₁, a force F₁ is measured. Figure 1C shows a situation where due to the continuous movement of upper jaw (1a) towards lower jaw (1b), the force that is exerted on particle (2) causes further deformation, although the particle (2) does not fracture. The force-displacement-diagram indicates that after a reduction of the displacement do of upper jaw (1a) and lower jaw (1b) by distance x₂, i.e. at a displacement of d₂ = d₀ - x₂, a force F₂ is measured. Under these circumstances, the particle (2) has not been broken (fractured) and a substantially steady increase of the force in the force-displacement-diagram is measured.

In contrast, Figure 2 schematically illustrates the measurement and the corresponding force-displacement-diagram of a conventional comparative particle not having the degree of deformability as the particles according to the invention. Figure 2A shows the initial situation at the beginning of the measurement. The comparative sample particle (2) is placed between upper jaw (1a) and lower jaw (1b) which each are in intimate contact with the surface of the comparative particle (2). The initial displacement do between upper jaw (1a) and lower jaw (1b) corresponds to the extension of the comparative particle orthogonal to the surfaces of upper jaw (1a) and lower jaw (1b). At this time, no force is exerted at all and thus, no graph is displayed in the force-displacement-diagram below. When the measurement is commenced, the upper jaw is moved in direction of lower jaw (1b), preferably at a constant speed. Figure 2B shows a situation where due to the movement of upper jaw (1a) towards lower jaw (1b) a force is exerted on comparative particle (2). Because of some deformability, the comparative particle (2) is slightly flattened without being fractured. The force-displacement-diagram indicates that after a reduction of the displacement do of upper jaw (1a) and lower jaw (1b) by distance x₁, i.e. at a displacement of d₁ = d₀ - x₁, a force F₁ is measured. Figure 2C shows a situation where due to the continuous movement of upper jaw (1a) towards lower jaw (1b), the force that is exerted on particle (2) causes sudden fracture of the comparative particle (2). The force-displacement-diagram indicates that after a reduction of the displacement do of upper jaw (1a) and lower jaw (1b) by distance x₂, i.e. at a displacement of d₂ = d₀ - x₂, a force F₂ is measured that suddenly drops when the particle fractures. Under these circumstances, the particle (2) has been broken (fractured) and no steady increase of the force in the force-displacement-diagram is measured. The sudden drop (decrease) of the force can easily be recognized and does not need to be quantified for the measurement. The steady increase in the force-displacement-diagram ends at displacement d₂ = d₀ - x₂ when the particle breaks.

In a preferred embodiment, the particles contained in the pharmaceutical dosage form according to the invention have a deformability such that they show an increase, preferably a substantially steady increase of the force at a corresponding decrease of the displacement in the force-displacement-diagram when being subjected to a breaking strength test as described above ("Zwick Z 2.5" materials tester, constant speed), preferably at least until the displacement d of upper jaw (1a) and lower jaw (1b) has been reduced to a value of 90% of the original displacement do (i.e. d = 0.9 · d₀), preferably to a displacement d of 80% of the original displacement d₀, more preferably to a displacement d of 70% of the original displacement do, still more preferably to a displacement d of 60% of the original displacement do, yet more preferably to a displacement d of 50% of the original displacement do, even more preferably to a displacement d of 40% of the original displacement do, most preferably to a displacement d of 30% of the original displacement do, and in particular to a displacement d of 20% of the original displacement d₀ or to a displacement d of 15% of the original displacement d₀, to a displacement d of 10% of the original displacement do, or to a displacement d of 5% of the original displacement d₀.

In another preferred embodiment, the particles contained in the pharmaceutical dosage form according to the invention have a deformability such that they show an increase, preferably a substantially steady increase of the force at a corresponding decrease of the displacement in the force-displacement-diagram when being subjected to a breaking strength test as described above ("Zwick Z 2.5" materials tester, constant speed), preferably at least until the displacement d of upper jaw (1a) and lower jaw (1b) has been reduced to 0.80 mm or 0.75 mm, preferably 0.70 mm or 0.65 mm, more preferably 0.60 mm or 0.55 mm, still more preferably 0.50 mm or 0.45 mm, yet more preferably 0.40 mm or 0.35 mm, even more preferably 0.30 mm or 0.25 mm, most preferably 0.20 mm or 0.15 mm and in particular 0.10 or 0.05 mm.

In still another preferred embodiment, the particles contained in the pharmaceutical dosage form according to the invention have a deformability such that they show an increase, preferably a substantially steady increase of the force at a corresponding decrease of the displacement in the force-displacement-diagram when being subjected to a breaking strength test as described above ("Zwick Z 2.5" materials tester, constant speed), at least until the displacement d of upper jaw (1a) and lower jaw (1b) has been reduced to 50% of the original displacement do (i.e. d = d₀/2), whereas the force measured at said displacement (d = d₀/2) is at least 25 N or at least 50 N, preferably at least 75 N or at least 100 N, still more preferably at least 150 N or at least 200 N, yet more preferably at least 250 N or at least 300 N, even more preferably at least 350 N or at least 400 N, most preferably at least 450 N or at least 500 N, and in particular at least 625 N, or at least 750 N, or at least 875 N, or at least 1000 N, or at least 1250 N, or at least 1500 N.

In another preferred embodiment, the particles contained in the pharmaceutical dosage form according to the invention have a deformability such that they show an increase, preferably a substantially steady increase of the force at a corresponding decrease of the displacement in the force-displacement-diagram when being subjected to a breaking strength test as described above ("Zwick Z 2.5" materials tester, constant speed), at least until the displacement d of upper jaw (1a) and lower jaw (1b) has been reduced by at least 0.1 mm, more preferably at least 0.2 mm, still more preferably at least 0.3 mm, yet more preferably at least 0.4 mm, even more preferably at least 0.5 mm, most preferably at least 0.6 mm, and in particular at least 0.7 mm, whereas the force measured at said displacement is within the range of from 5.0 N to 250 N, more preferably from 7.5 N to 225 N, still more preferably from 10 N to 200 N, yet more preferably from 15 N to 175 N, even more preferably from 20 N to 150 N, most preferably from 25 N to 125 N, and in particular from 30 N to 100 N.

In yet another embodiment, the particles contained in the pharmaceutical dosage form according to the invention have a deformability such that they are deformed without being fractured when subjected to a constant force of e.g. 50 N, 100 N, 200 N, 300 N, 400 N, 500 N or 600 N in a breaking strength test as described above ("Zwick Z 2.5" materials tester, constant force), until the displacement d of upper jaw (1a) and lower jaw (1b) is reduced so that no further deformation takes place at said constant force, whereas at this equilibrated state the displacement d of upper jaw (1a) and lower jaw (1b) is at most 90% of the original displacement do (i.e. d ≤ 0.9 · d₀), preferably at most 80% of the original displacement do (i.e. d ≤ 0.8 · d₀), more preferably at most 70% of the original displacement do (i.e. d ≤ 0.7 · d₀), still more preferably at most 60% of the original displacement do (i.e. d ≤ 0.6 · d₀), yet more preferably at most 50% of the original displacement do (i.e. d ≤ 0.5 · d₀), even more preferably at most 40% of the original displacement do (i.e. d ≤ 0.4 · d₀), most preferably at most 30% of the original displacement do (i.e. d ≤ 0.3 · d₀), and in particular at most 20% of the original displacement do (i.e. d ≤ 0.2 · d₀), or at most 15% of the original displacement do (i.e. d ≤ 0.15 · d₀), at most 10% of the original displacement do (i.e. d ≤ 0.1 · d₀), or at most 5% of the original displacement do (i.e. d ≤ 0.05 · d₀).

Preferably, the particles contained in the pharmaceutical dosage form according to the invention have a deformability such that they are deformed without being fractured when subjected to a constant force of e.g. 50 N, 100 N, 200 N, 300 N, 400 N, 500 N or 600 N in a breaking strength test as described above ("Zwick Z 2.5" materials tester, constant force), until the displacement d of upper jaw (1a) and lower jaw (1b) is reduced so that no further deformation takes place at said constant force, whereas at this equilibrated state the displacement d of upper jaw (1a) and lower jaw (1b) is at most 0.80 mm or at most 0.75 mm, preferably at most 0.70 mm or at most 0.65 mm, more preferably at most 0.60 mm or at most 0.55 mm, still more preferably at most 0.50 mm or at most 0.45 mm, yet more preferably at most 0.40 mm or at most 0.35 mm, even more preferably at most 0.30 mm or at most 0.25 mm, most preferably at most 0.20 mm or at most 0.15 mm and in particular at most 0.10 or at most 0.05 mm.

In another embodiment, the particles contained in the pharmaceutical dosage form according to the invention have a deformability such that they are deformed without being fractured when subjected to a constant force of e.g. 50 N, 100 N , 200 N, 300 N, 400 N, 500 N or 600 N in a breaking strength test as described above ("Zwick Z 2.5" materials tester, constant force), until the displacement d of upper jaw (1a) and lower jaw (1b) is reduced so that no further deformation takes place at said constant force, whereas at this equilibrated state the displacement d of upper jaw (1a) and lower jaw (1b) is at least 5% of the original displacement do (i.e. d ≥ 0.05 · d₀), preferably at least 10% of the original displacement do (i.e. d ≥ 0.1 · d₀), more preferably at least 15% of the original displacement do (i.e. d ≥ 0.15 · d₀), still more preferably at least 20% of the original displacement do (i.e. d ≥ 0.2 · d₀), yet more preferably at least 30% of the original displacement do (i.e. d≥ 0.3 · d₀), even more preferably at least 40% of the original displacement do (i.e. d ≥ 0.4 · d₀), most preferably at least 50% of the original displacement do (i.e. d ≥ 0.5 · d₀), and in particular at least 60% of the original displacement do (i.e. d ≥ 0.6 · d₀), or at least 70% of the original displacement do (i.e. d ≥ 0.7 · d₀), at least 80% of the original displacement do (i.e. d ≥ 0.8 · d₀), or at least 90% of the original displacement do (i.e. d ≥ 0.9 · d₀).

Preferably, the particles contained in the pharmaceutical dosage form according to the invention have a deformability such that they are deformed without being fractured when subjected to a constant force of e.g. 50 N, 100 N, 200 N, 300 N, 400 N, 500 N or 600 N in a breaking strength test as described above ("Zwick Z 2.5" materials tester, constant force), until the displacement d of upper jaw (1a) and lower jaw (1b) is reduced so that no further deformation takes place at said constant force, whereas at this equilibrated state the displacement d of upper jaw (1a) and lower jaw (1b) is at least 0.05 mm or at least 0.10 mm, preferably at least 0.15 mm or at least 0.20 mm, more preferably at least 0.25 mm or at least 0.30 mm, still more preferably at least 0.35 mm or at least 0.40 mm, yet more preferably at least 0.45 mm or at least 0.50 mm, even more preferably at least 0.55 mm or at least 0.60 mm, most preferably at least 0.65 mm or at least 0.70 mm and in particular at least 0.75 or at least 0.80 mm.

The pharmaceutical dosage form according to the invention provides under *in vitro* conditions immediate release of the pharmacologically active compound in accordance with Ph. Eur. Preferably, the pharmaceutical dosage form according to the invention provides an release profile such that under *in vitro* conditions in 600 ml 0.1 M HCl (pH 1) at 75 rpm after 30 min (USP apparatus II) at least 80 wt.-%, more preferably at least 90 wt.-% of the pharmacologically active ingredient that was originally contained in the dosage form have been released.

The term "immediate release" as applied to pharmaceutical dosage forms is understood by persons skilled in the art which has structural implications for the respective pharmaceutical dosage forms. The term is defined, for example, in the current issue of the US Pharmacopoeia (USP), General Chapter 1092, "THE DISSOLUTION PROCEDURE: DEVELOPMENT AND VALIDATION", heading "STUDY DESIGN", "Time Points". For immediate-release dosage forms, the duration of the procedure is typically 30 to 60 minutes; in most cases, a single time point specification is adequate for Pharmacopeia purposes. Industrial and regulatory concepts of product comparability and performance may require additional time points, which may also be required for product registration or approval. A sufficient number of time points should be selected to adequately characterize the ascending and plateau phases of the dissolution curve. According to the Biopharmaceutics Classification System referred to in several FDA Guidances, highly soluble, highly permeable drugs formulated with rapidly dissolving products need not be subjected to a profile comparison if they can be shown to release 85% or more of the active drug substance within 15 minutes. For these types of products a one-point test will suffice. However, most products do not fall into this category. Dissolution profiles of immediate-release products typically show a gradual increase reaching 85% to 100% at 30 to 45 minutes. Thus, dissolution time points in the range of 15, 20, 30, 45, and 60 minutes are usual for most immediate-release products.

Preferably, under physiological conditions the pharmaceutical dosage form according to the invention has released after 30 minutes at least 70%, more preferably at least 75%, still more preferably at least 80%, yet more preferably at least 82%, most preferably at least 84% and in particular at least 86% of the pharmacologically active compound originally contained in the pharmaceutical dosage form.

Preferably, under physiological conditions the pharmaceutical dosage form according to the invention has released after 10 minutes at least 70%, more preferably at least 73%, still more preferably at least 76%, yet more preferably at least 78%, most preferably at least 80% and in particular at least 82% of the pharmacologically active compound originally contained in the pharmaceutical dosage form.

Further preferred release profiles C¹ to C¹⁰ are summarized in the table here below [all data in wt.-% of released pharmacologically active compound]:

| time | C¹ | C² | C³ | C⁴ | C⁵ | C⁶ | C⁷ | C⁸ | C⁹ | C¹⁰ |
|---|---|---|---|---|---|---|---|---|---|---|
| 10 min | ≥ 30 | ≥ 35 | ≥ 40 | ≥ 45 | ≥ 50 | ≥ 60 | ≥ 70 | ≥ 80 | ≥ 80 | ≥ 80 |
| 20 min | ≥ 50 | ≥ 55 | ≥ 60 | ≥ 65 | ≥ 70 | ≥ 75 | ≥ 80 | ≥ 85 | ≥ 90 | ≥ 95 |
| 30 min | ≥ 55 | ≥ 60 | ≥ 65 | ≥ 70 | ≥ 75 | ≥ 85 | ≥ 90 | ≥ 95 | ≥ 95 | ≥ 95 |
| 40 min | ≥ 60 | ≥ 65 | ≥ 70 | ≥ 80 | ≥ 85 | ≥ 90 | ≥ 95 | ≥ 95 | ≥ 95 | ≥ 95 |
| 50 min | ≥ 65 | ≥ 70 | ≥ 80 | ≥ 85 | ≥ 88 | ≥ 92 | ≥ 95 | ≥ 95 | ≥ 95 | ≥ 95 |
| 60 min | ≥ 75 | ≥ 80 | ≥ 85 | ≥ 90 | ≥ 92 | ≥ 94 | ≥ 95 | ≥ 95 | ≥ 95 | ≥ 95 |

Preferably, the release profile, the drug and the pharmaceutical excipients of the pharmaceutical dosage form according to the invention are stable upon storage, preferably upon storage at elevated temperature, e.g. 40 °C, for 3 months in sealed containers.

In connection with the release profile "stable" means that when comparing the initial release profile with the release profile after storage, at any given time point the release profiles deviate from one another by not more than 20%, more preferably not more than 15%, still more preferably not more than 10%, yet more preferably not more than 7.5%, most preferably not more than 5.0% and in particular not more than 2.5%.

In connection with the drug and the pharmaceutical excipients "stable" means that the pharmaceutical dosage forms satisfy the requirements of EMEA concerning shelf-life of pharmaceutical products.

Suitable in vitro conditions are known to the skilled artisan. In this regard it can be referred to, e.g., the Eur. Ph. Preferably, the release profile is measured under the following conditions: Paddle apparatus equipped without sinker, 50 rpm, 37±5 °C, 900 mL simulated intestinal fluid pH 6.8 (phosphate buffer) or pH 4.5. In a preferred embodiment, the rotational speed of the paddle is increased to 75 rpm.

In a preferred embodiment, the pharmaceutical dosage form according to the invention is adapted for administration once daily. In another preferred embodiment, the pharmaceutical dosage form according to the invention is adapted for administration twice daily. In still another preferred embodiment, the pharmaceutical dosage form according to the invention is adapted for administration thrice daily. In yet another preferred embodiment, the pharmaceutical dosage form according to the invention is adapted for administration more frequently than thrice daily, for example 4 times daily, 5 times daily, 6 times daily, 7 times daily or 8 times daily.

For the purpose of the specification, "twice daily" means equal or nearly equal time intervals, i.e., every 12 hours, or different time intervals, e.g., 8 and 16 hours or 10 and 14 hours, between the individual administrations.

For the purpose of the specification, "thrice daily" means equal or nearly equal time intervals, i.e., every 8 hours, or different time intervals, e.g., 6, 6 and 12 hours; or 7, 7 and 10 hours, between the individual administrations.

Preferably, the pharmaceutical dosage form according to the invention has under *in vitro* conditions a disintegration time measured in accordance with Ph. Eur. of at most 5 minutes, more preferably at most 4 minutes, still more preferably at most 3 minutes, yet more preferably at most 2.5 minutes, most preferably at most 2 minutes and in particular at most 1.5 minutes.

It has been surprisingly found that oral dosage forms can be designed that provide the best compromise between tamper-resistance, disintegration time and drug release, drug load, processability (especially tablettability) and patient compliance.

Tamper-resistance and drug release antagonize each other. While smaller particles should typically show a faster release of the pharmacologically active compound, tamper-resistance requires some minimal size of the particles in order to effectively prevent abuse, e.g. i.v. administration. The larger the particles are the less they are suitable for being abused nasally. The smaller the particles are the faster gel formation occurs. Thus, drug release on the one hand and tamper-resistance on the other hand can be optimized by finding the best compromise.

In a preferred embodiment of the pharmaceutical dosage form according to the invention, the particles are hot-melt extruded. Thus, the particles according to the invention are preferably prepared by melt extrusion, although also other methods of thermoforming may be used in order to manufacture the particles according to the invention such as press-molding at elevated temperature or heating of particles that were manufactured by conventional compression in a first step and then heated above the softening temperature of the polyalkylene oxide in the particles in a second step to form hard pharmaceutical dosage forms. In this regards, thermoforming means the forming, or molding of a mass after the application of heat. In a preferred embodiment, the particles are thermoformed by hot-melt extrusion.

In a preferred embodiment, the particles are prepared by hot-melt extrusion, preferably by means of a twin-screw-extruder. Melt extrusion preferably provides a melt extruded strand that is preferably cut into monoliths, which are then optionally compressed and formed into particles. Preferably, compression is achieved by means of a die and a punch, preferably from a monolithic mass obtained by melt extrusion. If obtained via melt extrusion, the compressing step is preferably carried out with a monolithic mass exhibiting ambient temperature, that is, a temperature in the range from 20 to 25° C. The strands obtained by way of extrusion can either be subjected to the compression step as such or can be cut prior to the compression step. This cutting can be performed by usual techniques, for example using rotating knives or compressed air, at elevated temperature, e.g. when the extruded stand is still warm due to hot-melt extrusion, or at ambient temperature, i.e. after the extruded strand has been allowed to cool down. When the extruded strand is still warm, singulation of the extruded strand into extruded particles is preferably performed by cutting the extruded strand immediately after it has exited the extrusion die. It is possible to subject the extruded strands to the compression step or to the cutting step when still warm, that is more or less immediately after the extrusion step. The extrusion is preferably carried out by means of a twin-screw extruder.

The particles of the pharmaceutical dosage form according to the invention may be produced by different processes, the particularly preferred of which are explained in greater detail below. Several suitable processes have already been described in the prior art. In this regard it can be referred to, e.g., WO 2005/016313, WO 2005/016314, WO 2005/063214, WO 2005/102286, WO 2006/ 002883, WO 2006/002884, WO 2006/002886, WO 2006/082097, and WO 2006/082099.

In general, the process for the production of the particles according to the invention preferably comprises the following steps:
(a) mixing all ingredients;
(b) optionally pre-forming the mixture obtained from step (a), preferably by applying heat and/or force to the mixture obtained from step (a), the quantity of heat supplied preferably not being sufficient to heat the polyalkylene oxide up to its softening point;
(c) hardening the mixture by applying heat and force, it being possible to supply the heat during and/or before and/or after the application of force and the quantity of heat supplied being sufficient to heat the polyalkylene oxide at least up to its softening point; and thereafter allowing the material to cool and removing the force
(d) optionally singulating the hardened mixture; and
(e) optionally providing a film coating.

Heat may be supplied directly, e.g. by contact or by means of hot gas such as hot air, or with the assistance of ultrasound; or is indirectly supplied by friction and/or shear. Force may be applied and/or the particles may be shaped for example by direct tabletting or with the assistance of a suitable extruder, particularly by means of a screw extruder equipped with one or two screws (single-screw-extruder and twin-screw-extruder, respectively) or by means of a planetary gear extruder.

The final shape of the particles may either be provided during the hardening of the mixture by applying heat and force (step (c)) or in a subsequent step (step (e)). In both cases, the mixture of all components is preferably in the plastified state, i.e. preferably, shaping is performed at a temperature at least above the softening point of the polyalkylene oxide. However, extrusion at lower temperatures, e.g. ambient temperature, is also possible and may be preferred.

In a preferred embodiment, the mixture of ingredients is heated and subsequently compressed under conditions (time, temperature and pressure) sufficient in order to achieve the desired mechanical properties, e.g. in terms of breaking strength and the like. This technique may be achieved e.g. by means of a tabletting tool which is either heated and/or which is filled with the heated mixture that is subsequently compressed without further supply of heat or with simultaneous additional supply of heat.

In another preferred embodiment, the mixture of ingredients is heated and simultaneously compressed under conditions (time, temperature and pressure) sufficient in order to achieve the desired mechanical properties, e.g. in terms of breaking strength and the like. This technique may be achieved e.g. by means of an extruder with one or more heating zones, wherein the mixture is heated and simultaneously subjected to extrusion forces finally resulting in a compression of the heated mixture.

In still another embodiment, the mixture of ingredients is compressed under ambient conditions at sufficient pressure and subsequently heated (cured) under conditions (time, temperature) sufficient in order to achieve the desired mechanical properties, e.g. in terms of breaking strength and the like. This technique may be achieved e.g. by means of a curing oven in which the compressed articles are cured for a sufficient time at a sufficient temperature, preferably without exerting any further pressure. Such process is further described e.g. in US 2009/0081290.

A particularly preferred process for the manufacture of the particles according to the invention involves hot-melt extrusion. In this process, the particles according to the invention are produced by thermoforming with the assistance of an extruder, preferably without there being any observable consequent discoloration of the extrudate.

This process is characterized in that
a) all components are mixed,
b) the resultant mixture is heated in the extruder at least up to the softening point of the polyalkylene oxide and extruded through the outlet orifice of the extruder by application of force,
c) the still plastic extrudate is singulated and formed into the particles or
d) the cooled and optionally reheated singulated extrudate is formed into the particles.

Mixing of the components according to process step a) may also proceed in the extruder.

The components may also be mixed in a mixer known to the person skilled in the art. The mixer may, for example, be a roll mixer, shaking mixer, shear mixer or compulsory mixer.

The, preferably molten, mixture which has been heated in the extruder at least up to the softening point of polyalkylene oxide is extruded from the extruder through a die with at least one bore, preferably a multitude of bores.

The process according to the invention requires the use of suitable extruders, preferably screw extruders. Screw extruders which are equipped with two screws (twin-screw-extruders) are particularly preferred.

Preferably, extrusion is performed in the absence of water, i.e., no water is added. However, traces of water (e.g., caused by atmospheric humidity) may be present.

The extruder preferably comprises at least two temperature zones, with heating of the mixture at least up to the softening point of the polyalkylene oxide proceeding in the first zone, which is downstream from a feed zone and optionally mixing zone. The throughput of the mixture is preferably from 1.0 kg to 15 kg/hour. In a preferred embodiment, the throughput is from 0.5 kg/hour to 3.5 kg/hour. In another preferred embodiment, the throughput is from 4 to 15 kg/hour.

In a preferred embodiment, the die head pressure is within the range of from 25 to 200 bar. The die head pressure can be adjusted inter alia by die geometry, temperature profile, extrusion speed, number of bores in the dies, screw configuration, first feeding steps in the extruder, and the like.

The die geometry or the geometry of the bores is freely selectable. The die or the bores may accordingly exhibit a round, oblong or oval cross-section, wherein the round cross-section preferably has a diameter of 0.1 mm to 2 mm, preferably of 0.5 mm to 0.9 mm. Preferably, the die or the bores have a round cross-section. The casing of the extruder used according to the invention may be heated or cooled. The corresponding temperature control, i.e. heating or cooling, is so arranged that the mixture to be extruded exhibits at least an average temperature (product temperature) corresponding to the softening temperature of the polyalkylene oxide and does not rise above a temperature at which the pharmacologically active compound to be processed may be damaged. Preferably, the temperature of the mixture to be extruded is adjusted to below 180 °C, preferably below 150 °C, but at least to the softening temperature of polyalkylene oxide. Typical extrusion temperatures are 120 °C and 150 °C.

In a preferred embodiment, the extruder torque is within the range of from 30 to 95%. Extruder torque can be adjusted inter alia by die geometry, temperature profile, extrusion speed, number of bores in the dies, screw configuration, first feeding steps in the extruder, and the like.

After extrusion of the molten mixture and optional cooling of the extruded strand or extruded strands, the extrudates are preferably singulated. This singulation may preferably be performed by cutting up the extrudates by means of revolving or rotating knives, wires, blades or with the assistance of laser cutters.

Preferably, intermediate or final storage of the optionally singulated extrudate or the final shape of the particles according to the invention is performed under oxygen-free atmosphere which may be achieved, e.g., by means of oxygen-scavengers.

The singulated extrudate may be press-formed into particles in order to impart the final shape to the particles.

The application of force in the extruder onto the at least plasticized mixture is adjusted by controlling the rotational speed of the conveying device in the extruder and the geometry thereof and by dimensioning the outlet orifice in such a manner that the pressure necessary for extruding the plasticized mixture is built up in the extruder, preferably immediately prior to extrusion. The extrusion parameters which, for each particular composition, are necessary to give rise to a pharmaceutical dosage form with desired mechanical properties, may be established by simple preliminary testing.

For example but not limiting, extrusion may be performed by means of a twin-screw-extruder type ZSE 18 or ZSE27 (Leistritz, Nürnberg, Germany), screw diameters of 18 or 27 mm. Screws having eccentric or blunt ends may be used. A heatable die with a round bore or with a multitude of bores each having a diameter of 0.2, 0.3, 0.4, 0.5, 0.6, 0.7, 0.8, 0.9 or 1.0 mm may be used. For a twin-screw-extruder type ZSE 18, the extrusion parameters may be adjusted e.g. to the following values: rotational speed of the screws: 120 Upm; delivery rate 2 kg/h for a ZSE 18 or 5 kg/h, 10 kg/h, or even 20 kg/h and more for a ZSE27; product temperature: in front of die 125 °C and behind die 135 °C; and jacket temperature: 110 °C. The throughput can generally be increased by increasing the number of dies at the extruder outlet.

Preferably, extrusion is performed by means of twin-screw-extruders or planetary-gear-extruders, twin-screw extruders (co-rotating or contra-rotating) being particularly preferred.

The particles according to the invention are preferably produced by thermoforming with the assistance of an extruder without any observable consequent discoloration of the extrudates. The particles may be produced e.g. by means of a Micro Pelletizer (Leistritz, Nürnberg, Germany).

The process for the preparation of the particles according to the invention is preferably performed continuously. Preferably, the process involves the extrusion of a homogeneous mixture of all components. It is particularly advantageous if the thus obtained intermediate, e.g. the strand obtained by extrusion, exhibits uniform properties. Particularly desirable are uniform density, uniform distribution of the active compound, uniform mechanical properties, uniform porosity, uniform appearance of the surface, etc. Only under these circumstances the uniformity of the pharmacological properties, such as the stability of the release profile, may be ensured and the amount of rejects can be kept low.

Preferably, the particles according to the invention can be regarded as "extruded pellets". The term "extruded pellets" has structural implications which are understood by persons skilled in the art. A person skilled in the art knows that pelletized dosage forms can be prepared by a number of techniques, including:
- drug layering on nonpareil sugar or microcrystalline cellulose beads,
- spray drying,
- spray congealing,
- rotogranulation,
- hot-melt extrusion,
- spheronization of low melting materials, or
- extrusion-spheronization of a wet mass.

Accordingly, "extruded pellets" can be obtained either by hot-melt extrusion or by extrusion-spheronization.

"Extruded pellets" can be distinguished from other types of pellets, as extruded pellets typically have a different shape. The shape of the extruded pellets is typically more cut-rod-like than perfectly globated round.

"Extruded pellets" can be distinguished from other types of pellets because they are structurally different. For example, drug layering on nonpareils yields multilayered pellets having a core, whereas extrusion typically yields a monolithic mass comprising a homogeneous mixture of all ingredients. Similarly, spray drying and spray congealing typically yield spheres, whereas extrusion typically yields cylindrical extrudates which can be subsequently spheronized.

The structural differences between "extruded pellets" and "agglomerated pellets" are significant because they may affect the release of active substances from the pellets and consequently result in different pharmacological profiles. Therefore, a person skilled in the pharmaceutical formulation art would not consider "extruded pellets" to be equivalent to "agglomerated pellets".

The pharmaceutical dosage forms according to the invention may be prepared by any conventional method. Preferably, however, the pharmaceutical dosage forms are prepared by compression. Thus, particles as hereinbefore defined are preferably mixed, e.g. blended and/or granulated (e.g. wet granulated), with matrix material and the resulting mix (e.g. blend or granulate) is then compressed, preferably in moulds, to form pharmaceutical dosage forms. It is also envisaged that the particles herein described may be incorporated into a matrix using other processes, such as by melt granulation (e.g. using fatty alcohols and/or water-soluble waxes and/or water-insoluble waxes) or high shear granulation, followed by compression.

When the pharmaceutical dosage forms according to the invention are manufactured by means of an eccentric press, the compression force is preferably within the range of from 5 to 15 kN. When the pharmaceutical dosage forms according to the invention are manufactured by means of a rotating press, the compression force is preferably within the range of from 5 to 40 kN, in certain embodiments >25 kN, in other embodiments 13 kN.

The pharmaceutical dosage forms according to the invention may optionally comprise a coating, e.g. a cosmetic coating. The coating is preferably applied after formation of the pharmaceutical dosage form. The coating may be applied prior to or after the curing process. Preferred coatings are Opadry® coatings available from Colorcon. Other preferred coating are Opaglos® coatings, also commercially available from Colorcon.

The pharmaceutical dosage form according to the invention is characterized by excellent storage stability. Preferably, after storage for 6 months, 3 months, 2 months, or 4 weeks at 40°C and 75% rel. humidity, the content of pharmacologically active compound amounts to at least 98.0%, more preferably at least 98.5%, still more preferably at least 99.0%, yet more preferably at least 99.2%, most preferably at least 99.4% and in particular at least 99.6%, of its original content before storage. Suitable methods for measuring the content of the pharmacologically active compound in the pharmaceutical dosage form are known to the skilled artisan. In this regard it is referred to the Eur. Ph. or the USP, especially to reversed phase HPLC analysis. Preferably, the pharmaceutical dosage form is stored in closed, preferably sealed containers.

The particles and pharmaceutical dosage forms according to the invention may be used in medicine, e.g. as an analgesic. The particles and pharmaceutical dosage forms are therefore particularly suitable for the treatment or management of pain. In such pharmaceutical dosage forms, the pharmacologically active compound is preferably an analgesic.

A further aspect according to the invention relates to the pharmaceutical dosage form as described above for use in the treatment of pain.

When the pharmacologically active agent is a stimulant, the invention relates to the pharmaceutical dosage form as described above for use in the treatment of attention deficit hyperactivity syndrome (ADHS).

The subjects to which the pharmaceutical dosage forms according to the invention can be administered are not particularly limited. Preferably, the subjects are animals, more preferably human beings.

A further aspect according to the invention relates to the use of a pharmaceutical dosage form as described above for avoiding or hindering the abuse of the pharmacologically active compound contained therein.

A further aspect according to the invention relates to the use of a pharmaceutical dosage form as described above for avoiding or hindering the unintentional overdose of the pharmacologically active compound contained therein.

In this regard, the invention also relates to the use of a pharmacologically active compound as described above and/or a polyalkylene oxide as described above for the manufacture of the pharmaceutical dosage form according to the invention for the prophylaxis and/or the treatment of a disorder, thereby preventing an overdose of the pharmacologically active compound, particularly due to comminution of the pharmaceutical dosage form by mechanical action.

### EXAMPLES

The following examples further illustrate the invention but are not to be construed as limiting its scope.

### General operation procedures

Powder mixtures of various ingredients were manufactured by weighing (10 kg balance), sieving (1.0 mm hand sieve) and blending. The thus obtained powder mixtures were then hot-melt extruded (twin-screw extruder, Leistritz ZSE 18, blunt ends of kneading elements, and extrusion diameter of 8 x 0.8 mm). The extrudates were pelletized (LMP, 900 U/min) and then analyzed.

*In vitro* dissolution was tested in accordance with USP (apparatus II), in 600 ml 0.1 M HCl (pH 1) at 75 rpm (n=3).

Resistance against solvent extraction was tested by dispensing particles in 5 ml of boiling water. After boiling for 5 minutes the liquid was drawn up into a syringe (needle 21G equipped with a cigarette filter), and the amount of the pharmacologically active ingredient contained in the liquid within the syringe was determined via HPLC.

The test was performed on the extrudates as such but not on capsules or tablets containing such extrudates, as this test more relevant with respect to drug abuse. The other constituents of dosage forms (e.g. capsules or tablets) typically make it even more difficult for the abuser to tamper with the dosage form, e.g. by blocking the filters of syringes and the like. Thus, in the course of tampering, abusers frequently initially separate the drug containing subunits of dosage forms (here extrudates) from the remainder of the dosage forms in order to facilitate subsequent abuse, e.g. by extraction. Accordingly, it is more significant to evaluate tamper-resistance of the extrudates instead of the overall dosage forms.

### A) Comparative Examples

The following comparative examples 1 to 4 are not in accordance with the invention, because the weight average molecular weight of the polyethylene oxide is 7,000,000 g/mol, i.e. far above the upper limit of 2,000,000 g/mol, and because the content of disintegrant is below the lower limit of more than 20 wt.-% relative to the total weight of the particles.

### Comparative Example 1:

Powder mixtures of the following ingredients were manufactured and subsequently hot-melt extruded under the following extrusion conditions:

| | 1-1 | 1-2 | 1-3 | 1-4 | 1-5 |
|---|---|---|---|---|---|
| per dosis | mg/ wt.-% | mg/ wt.-% | mg/ wt.-% | mg/ wt.-% | mg/ wt.-% |
| Oxycodone HCl | 10.00/5.56 | 10.00/5.56 | 10.00/5.56 | 10.00/5.56 | 10.00/5.56 |
| Citric acid | 1.44/0.80 | 1.44/0.80 | 1.44/0.80 | 1.44/0.80 | 1.44/0.80 |
| Macrogol 6000 | 25.20/14.00 | 25.20/14.00 | 25.20/14.00 | 25.20/14.00 | 25.20/14.00 |
| alpha-tocopherol | 0.36/0.20 | 0.36/0.20 | 0.36/0.20 | 0.36/0.20 | 0.36/0.20 |
| Xanthan Gum Type 602 | 9.00/5.00 | 9.00/5.00 | 9.00/5.00 | 9.00/5.00 | 9.00/5.00 |
| Polyethylene oxide 7 Mio. | 98.00/54.44 | 98.00/54.44 | 98.00/54.44 | 98.00/54.44 | 95.22/52.20 |
| Sodium bicarbonate | - | - | - | - | 2.78/1.54 |
| Sodium starch glycolate | 36.00/20.00 | - | - | - | - |
| Croscarmellose sodium | - | 36.00/20.00 | - | - | - |
| Starch 1500 | - | - | 36.00/20.00 | - | - |
| Maize starch | - | - | - | 36.00/20.00 | - |
| Carbomer Carbopol 71G | - | - | - | - | 36.00/20.00 |
| Sum | 180.00/ | 180.00/ | 180.00/ | 180.00/ | 180.00/ |
| | 100.00 | 100.00 | 100.00 | 100.00 | 100.00 |
| | | | | | |
| Speed screw [rpm] | 100 | 100 | 100 | 100 | 120 |
| Feed rate [g/min] | 16.66 | 16.66 | 16.66 | 16.66 | 16.66 |
| Melt pressure [bar] | 119 | 141 | 136 | 135 | 116 |
| melt temperature discharge [°C] | 140 | 143 | 142 | 143 | 145 |

The *in vitro* dissolution test revealed the following release profiles:

| Dissolution Oxycodone % | 1-1 | 1-2 | 1-3 | 1-4 | 1-5 |
|---|---|---|---|---|---|
| after 5 min | 70 | 74 | 66 | 78 | 58 |
| after 15 min | 88 | 91 | 88 | 94 | 83 |
| after 30 min | 94 | 94 | 95 | 100 | 92 |
| after 60 min | 96 | 96 | 97 | 102 | 96 |

The test for tamper-resistance provided the following results (where all tested pellets remained intact after the breaking strength tester had reached its upper force limit):

| test battery | 1-1 | 1-2 | 1-3 | 1-4 | 1-5 |
|---|---|---|---|---|---|
| 1 | 0.00* | 1.34 | 0.00* | 22.40 | 0.00* |
| 2 | 0.00* | 3.07 | 20.20 | 30.32 | 0.00* |
| 3 | 0.00* | 1.26 | 6.03 | 18.67 | 0.00* |
| mean [%] | 0.00* | 1.89 | 8.74 | 28.80 | 0.00* |
| SD [%] | 0.00* | 1.02 | 10.37 | 5.95 | 0.00* |

| | | | | | |
|---|---|---|---|---|---|
| *not tested, sample too jelly and could not be drawn into syringe | | | | | |

It becomes clear from the above experimental data that as far as tamper-resistant dosage forms providing immediate release are concerned, the tested disintegrants provide different performance. Under the given experimental conditions, cellulose derivatives (e.g. croscarmellose sodium) provided the best performance, followed by starch derivatives (e.g. sodium starch glycolate) and gas releasing substances (here sodium bicarbonate), followed by pregelatinized starch (e.g. starch 1500) and standard starch (e.g. native maize starch).

### Comparative Example 2:

The influence of the presence and absence of gelling agent as well as the influence of the presence and absence of disintegrant was investigated in analogy to Comparative Example 1. The following compositions A to F were prepared:

| | 2-A | | 2-B | | 2-C | | 2-D | | 2-E | | 2-F | |
|---|---|---|---|---|---|---|---|---|---|---|---|---|
| Substance | mg | wt.-% | mg | wt.-% | mg | wt.-% | mg | wt.-% | mg | wt.-% | mg | wt.-% |
| Oxycodone HCl | 10.00 | 5.56 | 10.00 | 5.56 | 10.00 | 5.56 | 10.00 | 5.56 | 10.00 | 5.56 | 10.00 | 5.56 |
| Citric acid | 1.44 | 0.80 | 1.44 | 0.80 | 1.44 | 0.80 | 1.44 | 0.80 | 1.44 | 0.80 | 1.44 | 0.80 |
| PEG | 25.20 | 14.00 | 25.20 | 14.00 | 25.20 | 14.00 | 25.20 | 14.00 | 25.20 | 14.00 | 25.20 | 14.00 |
| α-Toc. | 0.36 | 0.20 | 0.36 | 0.20 | 0.36 | 0.20 | 0.36 | 0.20 | 0.36 | 0.20 | 0.36 | 0.20 |
| PEO 7 Mio. | 143.0 | 79.44 | 107.0 | 59.44 | 107.0 | 59.44 | 134.0 | 74.44 | 98.00 | 54.44 | 98.00 | 54.44 |
| Carbopol | - | - | 36.00 | 20.00 | 27.00 | 15.00 | - | - | - | - | - | - |
| Xanthan | - | - | - | - | 9.00 | 5.00 | 9.00 | 5.00 | 9.00 | 5.00 | 9.00 | 5.00 |
| Carb.MS | - | - | - | - | - | - | - | - | 36.00 | 20.00 | - | - |
| Na-CMC | - | - | - | - | - | - | - | - | - | - | 36.00 | 20.00 |
| Sum | 180 | 100 | 180 | 100 | 180 | 100 | 180 | 100 | 180 | 100 | 180 | 100 |

| | | | | | | | | | | | | |
|---|---|---|---|---|---|---|---|---|---|---|---|---|
| PEG = Polyethylene glycol 6000; α-Toc. = alpha-tocopherol; PEO 7 Mio = polyethylene oxide M_{w} 7 Mio g/mol; Carbopol = Carbopol 71G; Xanthan = Xanthan gum; Carb.MS= Carboxy methyl starch; Na-CMC = Croscarmellose sodium | | | | | | | | | | | | |

*In vitro* release as well as resistance against solvent extraction were determined in accordance with the invention. The results for the different pharmacologically active ingredients are shown in the table here below:

| Formulation | extract. | diss. |
|---|---|---|
| 2-A | 50 % | 73 % |
| 2-B | 40 % | 90 % |
| 2-C | 28 % | 90 % |
| 2-D | 12 % | 91 % |
| 2-E | 0 % | 94 % |
| 2-F | 2 % | 94 % |

| | | |
|---|---|---|
| extract. = extracted in solvent; diss = dissolution after 30 minutes | | |

It becomes clear from the above comparative data that the disintegrants in formulations E and F provide best performance with respect to immediate drug release and resistance against solvent extraction for all tested pharmacologically active ingredients, whereas the comparative formulations A, B, C and D only provided partial effects for some of the tested pharmacologically active ingredients.

### Comparative Example 3:

The influence of the content of disintegrant was investigated in analogy to Comparative Examples 1 and 2. Compositions 3-1 to 3-3 were prepared and *in vitro* dissolution as well as resistance against solvent extraction were determined.

| | 3-1 | | 3-2 | | 3-3 | |
|---|---|---|---|---|---|---|
| Substance per dose | mg | wt.-% | mg | wt.-% | mg | wt.-% |
| Oxycodone HCl | 10.00 | 5.56 | 10.00 | 5.56 | 10.00 | 5.56 |
| Citric acid | 1.44 | 0.80 | 1.44 | 0.80 | 1.44 | 0.80 |
| PEG 6000 | 27.51 | 15.28 | 25.20 | 14.00 | 27.51 | 15.28 |
| alpha-tocopherol | 0.36 | 0.20 | 0.36 | 0.20 | 0.36 | 0.20 |
| Xanthan Gum Type 602 | 9.00 | 5.00 | 9.00 | 5.00 | 9.00 | 5.00 |
| PEO 7 Mio. | 104.69 | 58.16 | 98.00 | 54.44 | 91.31 | 50.73 |
| Sodium starch glycolate | 27.00 | 15.00 | 36.00 | 20.00 | 45.00 | 25.00 |
| | 180.00 | 100.00 | 180.00 | 100.00 | 180.00 | 100.00 |

| Dissolution (n=3): | | | | | | |
|---|---|---|---|---|---|---|
| 0 | 0.00 | | 0.00 | | 0.00 | |
| 5 | 64.46 | | 69.73 | | 62.04 | |
| 15 | 78.42 | | 87.57 | | 81.83 | |
| 30 | 91.24 | | 94.44 | | 91.76 | |
| 60 | 94.82 | | 96.49 | | 95.12 | |
| extraction without milling: | | | | | | |
| mean [%] | 10.10 | | 0.00* | | 16.37 | |
| SD [%] | 4.67 | | 0.00* | | 12.67 | |

| | | | | | | |
|---|---|---|---|---|---|---|
| * not tested, sample too jelly and could not be drawn into syringe | | | | | | |

It becomes clear from the above comparative data that under the given conditions the best results could be achieved at a content of 20 wt.-% disintegrant (here sodium starch glycolate).

### Comparative Example 4:

Powder mixtures of the following ingredients were manufactured and subsequently hot-melt extruded:

| | 4-1 | |
|---|---|---|
| per dosis | mg | wt.-% |
| Oxycodone HCl | 30.00 | 12.00 |
| Citric acid | 1.25 | 0.50 |
| alpha-tocopherol | 0.5 | 0.20 |
| Polyethylene oxide 7 Mio. | 133.25 | 53.30 |
| Macrogol 6000 | 35.00 | 14.00 |
| Carbopol 71G | 50.00 | 20.00 |
| Sum | 250.00 | 100.00 |

Tablets containing oxycodone pellets were prepared:

| | per tablet | |
|---|---|---|
| Excipients | [mg]/ | [wt-%] |
| Oxycodone pellets | 250.00 | 50.00 |
| Avicel PH101/PVP-CL | 250.00 | 50.00 |
| Aerosil 200 | | |
| Mg stearate | | |
| Sum | 500.00 | 100.00 |

The test for tamper-resistance of intact tablets and intact pellets provided the following results:

| test battery | Oxycodone pellets | Tablet containing Oxvcodone pellets |
|---|---|---|
| 1 | 47.10 | 30.90 |
| 2 | 46.82 | 27.97 |
| 3 | 39.68 | 27.23 |
| mean [%] | 44.53 | 28.70 |

It becomes clear from the above comparative data that under the given conditions tablets containing pellets provide an improved resistance against solvent extraction compared to pellets alone.

### B) Inventive examples

The following examples are in accordance with the invention, because the weight average molecular weight of the polyethylene oxide is 1,000,000 g/mol and the content of disintegrant is more than 20 wt.-%, relative to the total weight of the particles.

### Inventive Example 1:

Powder mixtures of the following ingredients were manufactured and subsequently hot-melt extruded in accordance with Comparative Examples 1 to 4:

| Excipients | [wt.-%] | Amount per pellet unit (180 mg) [mg] | Amount per 3 pellet units (540 mg) [mg] |
|---|---|---|---|
| Oxycodone hydrochloride | 5.56 | 10.0 | 30.0 |
| Citric acid Ph. Eur. | 0.80 | 1.4 | 4.3 |
| Polyethylene glycol 6000 Ph. Eur. | 2.00 | 3.6 | 10.8 |
| alpha Tocopherol | 0.20 | 0.4 | 1.1 |
| Polyethylene oxide 1 Mio NF | 51.44 | 92.6 | 277.8 |
| Croscarmellose sodium Ph. Eur. | 35.00 | 63.0 | 189.0 |
| Hydroxyethylcellulose Ph. Eur. | 5.00 | 9.0 | 27.0 |
| Total | 100.00 | 180.00 | 540.0 |

Comparative tests revealed that when employing polyethylene oxide having a weight average molecular weight of 1,000,000 g/mol, higher contents of disintegrants exceeding a content of 20 wt.-% relative to the total weight of the particles are good processible with hot-melt extrusion.

Pellet tablets were manufactured by blending the pellets with an outer powder phase and compressed into tablets before getting coated in the last process step. The composition of the outer phase of the pellet tablet contains of filler material, disintegrant and lubricant, whereas the film coating is composed of general fast dissolving polymer to maintain the IR dissolution profile.

The composition of a 10 mg oxycodone hydrochloride TRF IR pellet tablet is shown in the following table.

| Excipients | [wt.-%] | Amount per tablet [mg] |
|---|---|---|
| Oxycodone hydrochloride | 1.39 | 10.0 |
| Citric acid Ph. Eur. | 0.20 | 1.4 |
| Polyethylene glycol 6000 Ph. Eur. | 0.50 | 3.6 |
| alpha Tocopherol | 0.05 | 0.4 |
| Polyethylene oxide 1 Mio NF | 12.84 | 92.6 |
| Croscarmellose sodium Ph. Eur. | 8.74 | 63.0 |
| Hydroxyethylcellulose Ph. Eur. | 1.25 | 9.0 |
| Magnesium Stearate Ph. Eur. | 0.15 | 1.1 |
| Crospovidone Ph. Eur. | 7.20 | 51.9 |
| Microcrystalline cellulose Ph. Eur. | 64.78 | 467.0 |
| Opadry® II White (33K58715) | 2.91 | 21.0 |
| Total | 100.00 | 721.00 |

### Inventive Example 2:

The effect of food on the administration of the tablet formulation according to Inventive Example 1 was investigated and compared to a marketed reference formulation. The objective of the trial was to assess the effect of food on both the formulation according to Inventive Example 1 ("Test") and a marketed reference Oxycodone IR formulation (Oxycodon HCl Aristo® film coated tablets; "Reference").

The formulation according to Inventive Example 1 was investigated in an open, randomized, 4-period cross-over, relative bioavailability/food effect trial against the reference. Single oral doses were administered to healthy male subjects under fasted and fed conditions. Serum drug concentrations were determined by a validated LC-MS/MS method. Non-compartmental PK analysis was performed and the usual 80.00 - 125.00 % confidence interval acceptance criteria for bioequivalence were used for comparing the treatments of Test and Reference.

The point estimates for AUC after fed and fasted conditions were 128% and 124% for Test and Reference, respectively, confirming a minimal food effect on the absorption of both compounds. With a median tₘₐₓ of 1.5 h [0.5; 4.0] both Test and Reference showed identical results. This is an important advantage, as a patient switching from a reference marketed formulation to a tamper-resistant dosage form according to the invention will achieve the desired therapeutic effect within the same time window after administration it is used to. Thus, false conclusions leading to an overdose can be prevented, i.e. conclusions that in the absence of the expected therapeutic affect the administered dose of the pharmacologically active ingredient might be too low and that therefore another tamper-resistant dosage form should be taken.

The pharmacokinetic results (PK Set) are compiled in the following tables:

| PK parameter | Statistics | Inventive Example 1/Test | | Comparator/Reference | |
|---|---|---|---|---|---|
| | | fasted (N = 21) | fed (N = 21) | fasted (N = 21) | fed (N = 21) |
| Cₘₐₓ [ng/mL] | Mean (SD) | 20.43 (4.95) | 22.22 (5.44) | 22.55 (5.00) | 24.75 (9.27) |
| | CV (%) | 24.2 | 24.5 | 22.2 | 37.5 |
| AUC₀₋ₜ [h·ng/mL] | Mean (SD) | 105.34 (18.38) | 135.96 (28.28) | 106.00 (19.38) | 132.07 (25.43) |
| | CV (%) | 17.4 | 20.8 | 18.3 | 19.3 |
| AUC [h·ng/mL] | Mean (SD) | 106.33 (18.57) | 137.32 (28.40) | 107.10 (19.46) | 133.40 (25.65) |
| | CV (%) | 17.5 | 20.7 | 18.2 | 19.2 |
| tₘₐₓ [h] | Median | 1.000 | 1.500 | 0.750 | 1.500 |
| | Min - Max | 0.75 -2.50 | 0.50 - 4.00 | 0.50 - 1.50 | 0.50 - 4.00 |

| | | | | | |
|---|---|---|---|---|---|
| Mean = arithmetic mean, SD = standard deviation, CV (%) = overall coefficient of variation | | | | | |

| PK parameter | Inventive Example 1/Test | | | Comparator/Reference | | |
|---|---|---|---|---|---|---|
| | Ratio [%] fasted/fed | 90% CI (lower, upper) | %CV | Ratio [%] fasted/fed | 90% CI (lower, upper) | %CV |
| Cₘₐₓ [ng/mL] | 108.1 | (98.4, 118.8) | 17.8 | 107.2 | (94.1, 122.0) | 24.7 |
| AUC₀₋ₜ [h·ng/mL] | 128.0 | (120.3, 136.2) | 11.7 | 124.2 | (115.6, 133.4) | 13.5 |
| AUC [h·ng/mL] | 128.1 | (120.5, 136.3) | 11.6 | 124.1 | (115.5, 133.3) | 13.5 |

| | | | | | | |
|---|---|---|---|---|---|---|
| CI = Confidence Interval, Ratio [%] = ratio of least square means, %CV = coefficient of intra-subject variation | | | | | | |

The data demonstrate that the formulation according to Inventive Example 1 has comparable in-vivo performance to standard, marketed immediate release formulations. The PK Set of the formulation according to Inventive Example 1 very accurately matches the confidence interval with regard to the PK profile of the standard, marketed immediate release formulation (not tamper-resistant). Both the formulation according to Inventive Example 1 and Comparator showed a similar, clinically not relevant food effect: 28% and 24% higher exposure (AUC), respectively, under fed conditions. The treatment emergent adverse events (TEAEs) in both groups were in line with the known safety profile of oxycodone IR; no serious TEAEs occurred.

Thus, there are no safety issues with the formulation according to Inventive Example 1 because of multiple dosing in view of a delayed onset of action, and the like. Further, there was no or only very marginal difference between fed status and fasted status, i.e. there is no clinically relevant food effect.

### Inventive Example 3:

The formulation according to Inventive Example 1 was tested in a human abuse liability study and compared to a commercial product and to placebo (placebo API and placebo TRF formulation).

A single-site, randomized, double-blind, double-dummy, active-comparator, placebo-controlled, 3-way crossover trial in adult non-dependent recreational opioid users was conducted to compare the intranasal abuse potential of the tablet formulation according to Inventive Example 1 and standard formulations of oxycodone.

The primary objective of the study was to compare the intranasal abuse potential of a manipulated formulation according to Inventive Example 1 and oxycodone active pharmaceutical ingredient (API) powder (representing manipulated oxycodone IR standard formulation). Primary measure: Peak effect (Eₘₐₓ) for Drug Liking "at this moment" on visual analog scale (VAS). The secondary objectives were to compare the pharmacokinetics (PK), further pharmacodynamic (PD) parameters, and assess safety and tolerability.

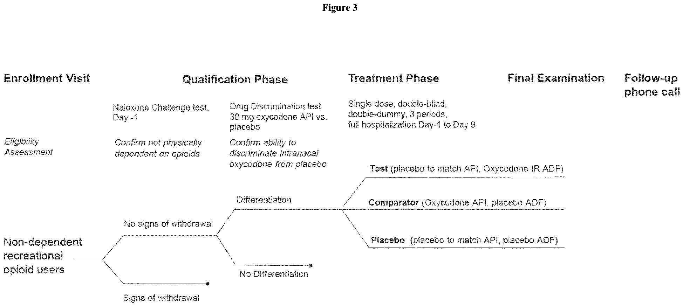

The trial design is illustrated in Figure 3 (Single-site, randomized, double-blind, double-dummy, active-comparator, placebo-controlled, 3-way crossover trial in male and female non-dependent recreational opioid users with recent intranasal use experience).

Subjects received single treatments of Test, Comparator, and Placebo in a randomized, double-blind, double-dummy, 3-way crossover. Each single treatment consisted of two investigational medicinal products (IMPs), to be insufflated (snorted) in quick succession in fixed sequence:

| Test (T) | Comparator (C) | Placebo (P) |
|---|---|---|
| Placebo API, powder (30 mg) | Oxycodone API, powder (30 mg oxycodone HCl) | Placebo API, powder (30 mg) |
| then: | then: | then: |
| manipulated formulation according to Inventive Example 1 (540 mg, containing 30 mg oxycodone HCl) | manipulated placebo tamper-resistant formulation (540 mg) | manipulated placebo tamper-resistant formulation (540 mg) |

Disposition of Subjects and Demographics: 123 subjects were enrolled (i.e. ICF signed). 47 subjects were randomized and completed Qualification Phase. 38 subjects were randomized in Treatment Phase and received IMP. 37 subjects completed trial (Completer Set), 1 subject discontinued due to adverse event (migraine, headache):

| Parameter | Category / Statistics | Treatment Safety Set (N=38) |
|---|---|---|
| Sex | Female / Male, n (%) | 7 (18.4%) / 31 (81.6%) |
| Age [years] | Mean (SD) / Min - Max | 35.7 (9.5) / 22 - 53 |
| BMI [kg/m2] | Mean (SD) / Min - Max | 24.52 (2.91) / 19.6 - 30.8 |

| | | |
|---|---|---|
| BMI = body mass index; ICF = informed consent form; SD = standard deviation | | |

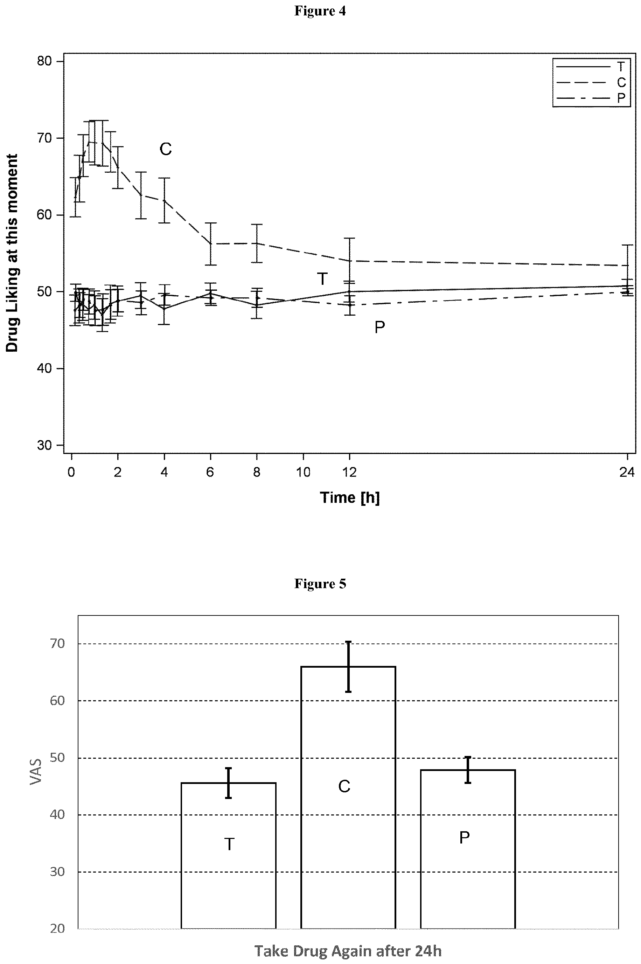

The results of the primary measure are shown in Figure 4.

Pharmacodynamic results:

| Primary endpoint | Treatment | Least Square Mean | 95% Confidence Limits | | |
|---|---|---|---|---|---|
| | | | Lower | - | Upper |
| Peak effect (Emax) of Drug Liking "at this moment" | Test (T) | 54.5 | 49.9 | | 59.2 |
| | Comparator (C) | 75.0 | 70.3 | | 79.6 |
| | Placebo (P) | 52.3 | 47.7 | | 57.0 |
| | Test - Comparator | -20.4 | -25.3 | | -15.6 |
| | Test - Placebo | 2.2 | -2.6 | | 7.0 |
| | Comparator - Placebo | 22.6 | 17.8 | | 27.5 |

Conclusions: Test had significantly lower Peak effect (Eₘₐₓ) of VAS Drug Liking "at this moment" than Comparator (p<0.0001). Comparator had significantly higher Eₘₐₓ than Placebo, thereby confirming the sensitivity of the measures and trial validity. Test and Placebo showed no significant difference in Eₘₐₓ.

Further subjective PD parameters (VAS High, VAS Overall Drug Liking, VAS Take Drug Again) are consistent and support the VAS Drug Liking outcomes that are illustrated in Figure 5, demonstrating that the willingness to take formulation according to Inventive Example 1 again is similar to Placebo and significantly lower than Comparator.

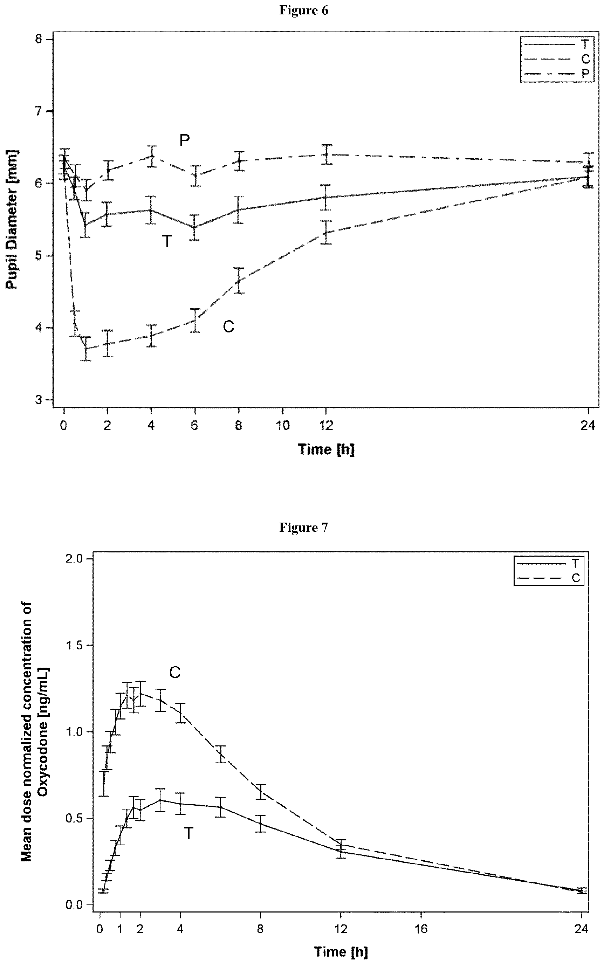

The objective PD measure - pulpillometry, mean pupil diameter - Completer Set is shown in Figure 6, demonstrating less maximum pupil constriction for formulation according to Inventive Example 1 relative to Comparator.

Pharmacokinetics results are presented in Figure 7 relative to the insufflated amount of oxycodone, mean dose-normalized plasma-concentration-time profiles of Oxycodone - PK Set:

It can be concluded that there is delayed and reduced absorption for formulation according to Inventive Example 1 relative to oxycodone API. The median tₘₐₓ is later for formulation according to Inventive Example 1 (Oxycodone API: 1.75 h, ADF: 3.03 h). Cₘₐₓ after formulation according to Inventive Example 1 only ∼40% of that after Comparator. AUC after formulation according to Inventive Example 1 only ∼60% of that after Comparator.

Safety results: No serious treatment emergent adverse events (TEAE) or deaths occurred during the trial. Overall, a higher percentage of subjects reported TEAEs after Comparator treatment (89.2%) than after Test (42.1%) or Placebo (37.8%) treatments:

| | Test | | | | Comparator | | | | Placebo | | | |
|---|---|---|---|---|---|---|---|---|---|---|---|---|
| | N=38 | | E=23 | | N=37 | | E=86 | | N=37 | | E=21 | |
| Primary System Organ Class | n | (%) | e | (%) | n | (%) | e | (%) | n | (%) | e | (%) |
| All SOCs | 16 | (42.1) | 23 | (100) | 33 | (89.2) | 86 | (100) | 14 | (37.8) | 21 | (100) |

Comparator treatment showed a higher incidence of known oxycodone adverse drug reactions (e.g., euphoric mood, pruritus (itch) and various gastrointestinal disorders (e.g. dry mouth, nausea)):

| | Test | | | | Comparator | | | | Placebo | | | |
|---|---|---|---|---|---|---|---|---|---|---|---|---|
| | N=38 | | E=23 | | N=37 | | E=86 | | N=37 | | E=21 | |
| Preferred Term | n | (%) | e | (%) | n | (%) | e | (%) | n | (%) | e | (%) |
| Euphoric mood | 1 | (2.6) | 1 | (4.3) | 18 | (48.6) | 19 | (22.1) | 1 | (2.7) | 1 | (4.8) |
| Pruritus | 0 | (0) | 0 | (0) | 11 | (29.7) | 12 | (14.0) | 0 | (0) | 0 | (0) |
| Drv mouth | 1 | (2.6) | 1 | (4.3) | 4 | (10.8) | 4 | (4.7) | 0 | (0) | 0 | (0) |
| Nausea | 1 | (2.6) | 1 | (4.3) | 6 | (16.2) | 6 | (7.0) | 0 | (0) | 0 | (0) |

| | | | | | | | | | | | | |
|---|---|---|---|---|---|---|---|---|---|---|---|---|
| n = number of subjects with TEAE. E = total number of TEAEs. e = number of TEAEs | | | | | | | | | | | | |

Conclusions: The outcome of the primary measure demonstrates a significantly lower maximal Drug Liking (mean Eₘₐₓ) for insufflated, manipulated formulation according to Inventive Example 1 compared with insufflated oxycodone API in the completer set. Statistically significant differences were observed for Drug Liking "at this moment" VAS Eₘₐₓ between Comparator and Placebo, confirming assay sensitivity and trial validity. Primary and secondary pharmacodynamic measures consistently indicate that the formulation according to Inventive Example 1 separates from oxycodone API, showing significantly lower Positive Effects and Overall Effects in a similar range to Placebo. The pharmacodynamic results are consistent with pharmacokinetic data indicating a delayed and reduced absorption of the formulation according to Inventive Example 1 relative to oxycodone API. Based on adverse event data, treatments were safe and well tolerated. The frequency of TEAEs after Test treatment was significantly lower relative to Comparator treatment, including a lower incidence of euphoric mood. The formulation according to Inventive Example 1 can be expected to have a lower potential of intranasal abuse compared to oxycodone API.

### Example 4 (inventive as well as comparative):

Powder mixtures of the following ingredients were manufactured and subsequently hot-melt extruded in accordance with Comparative Examples 1 to 4 and Inventive Example 1:

| | 4-1 comp. | | 4-2 comp. | | 4-3 comp. | | 4-4 comp. | | 4-5 comp. | |
|---|---|---|---|---|---|---|---|---|---|---|
| | mg | wt% | mg | wt% | mg | wt% | mg | wt% | mg | wt% |
| Oxycodone HCl | 10.00 | 5.56 | 10.00 | 5.56 | 10.00 | 5.56 | 10.00 | 5.56 | 10.00 | 5.56 |
| Citric acid | 1.44 | 0.80 | 1.44 | 0.80 | 1.44 | 0.80 | 1.44 | 0.80 | 1.44 | 0.80 |
| alpha-Tocopherol | 0.36 | 0.20 | 0.36 | 0.20 | 0.36 | 0.20 | 0.36 | 0.20 | 0.36 | 0.20 |
| PEG6000 | 27.00 | 15.00 | 34.20 | 19.00 | 23.40 | 13.00 | 39.60 | 22.00 | 3.60 | 2.00 |
| PEO M_{w} 7 Mio | 96.19 | 53.44 | 97.99 | 54.44 | 90.79 | 50.44 | 101.59 | 56.44 | - | - |
| PEO M_{w} 1 Mio | - | - | - | - | - | - | - | - | 101.59 | 56.44 |
| Xanthan gum | 9.00 | 5.00 | 9.00 | 5.00 | - | - | - | - | - | - |
| HEC | - | - | - | - | 18.00 | 10.00 | - | - | 27.00 | 15.00 |
| NaCMC | 36.00 | 20.00 | 27.00 | 15.00 | 36.00 | 20.00 | - | - | 36.00 | 20.00 |
| Starch 1500 | - | - | - | - | - | - | 27.00 | 15.00 | - | - |
| | | | | | | | | | | |

| | 4-6 inv. | | 4-7 comp. | | 4-8 inv. | | 4-9 comp. | | 4-10 comp. | |
|---|---|---|---|---|---|---|---|---|---|---|
| | mg | wt% | mg | wt% | mg | wt% | mg | wt% | mg | wt% |
| Oxycodone HCl | 10.00 | 5.56 | 10.00 | 5.56 | 10.00 | 5.56 | 10.00 | 5.56 | 10.00 | 5.56 |
| Citric acid | 1.44 | 0.80 | 1.44 | 0.80 | 1.44 | 0.80 | 1.44 | 0.80 | 1.44 | 0.80 |
| alpha-Tocopherol | 0.36 | 0.20 | 0.36 | 0.20 | 0.36 | 0.20 | 0.36 | 0.20 | 0.36 | 0.20 |
| PEG6000 | 3.60 | 2.00 | 3.60 | 2.00 | 3.60 | 2.00 | 3.60 | 2.00 | 3.60 | 2.00 |
| PEO M_{w} 7 Mio | - | - | - | - | - | - | - | - | - | - |
| PEO M_{w} 1 Mio | 92.59 | 51.44 | 110.59 | 61.44 | 92.59 | 51.44 | 110.59 | 61.44 | 110.59 | 61.44 |
| Xanthan gum | - | - | - | - | - | - | - | - | - | - |
| HEC | 18.00 | 10.00 | 27.00 | 15.00 | 9.00 | 5.00 | 36.00 | 20.00 | 18.00 | 10.00 |
| NaCMC | 54.00 | 30.00 | 27.00 | 15.00 | 63.00 | 35.00 | 18.00 | 10.00 | 36.00 | 20.00 |
| Starch 1500 | - | - | - | - | - | - | - | - | - | - |
| | | | | | | | | | | |

| | 4-11 comp. | | 4-12 inv. | | 4-13 comp. | | 4-14 comp. | | 4-15 comp. | |
|---|---|---|---|---|---|---|---|---|---|---|
| | mg | wt% | mg | wt% | mg | wt% | mg | wt% | mg | wt% |
| Oxycodone HCl | 10.00 | 5.56 | 10.00 | 5.56 | 10.00 | 5.56 | 10.00 | 5.56 | 10.00 | 5.56 |
| Citric acid | 1.44 | 0.80 | 1.44 | 0.80 | 1.44 | 0.80 | 1.44 | 0.80 | 1.44 | 0.80 |
| alpha-Tocopherol | 0.36 | 0.20 | 0.36 | 0.20 | 0.36 | 0.20 | 0.36 | 0.20 | 0.36 | 0.20 |
| PEG6000 | 3.60 | 2.00 | 3.60 | 2.00 | 3.60 | 2.00 | 3.60 | 2.00 | 3.60 | 2.00 |
| PEO M_{w} 7 Mio | - | - | - | - | - | - | - | - | - | - |
| PEO M_{w} 1 Mio | 92.59 | 51.44 | 110.59 | 61.44 | 137.59 | 76.44 | 119.59 | 66.44 | 128.59 | 71.44 |
| Xanthan gum | - | - | - | - | - | - | 9.00 | 5.00 | 9.00 | 5.00 |
| HEC | 54.00 | 30.00 | 9.00 | 5.00 | - | - | - | - | - | - |
| NaCMC | 18.00 | 10.00 | 45.00 | 25.00 | - | - | 36.00 | 20.00 | 27.00 | 15.00 |
| Starch 1500 | - | - | - | - | 27.00 | 15.00 | - | - | - | - |

Formulations 4-6, 4-8 and 4-12 are in accordance with the invention (PEO M_{w} 1 Mio and NaCMC > 20 wt.-%). The other formulations are comparative formulations because of the weight average molecular weight of the PEO (M_{w} 7 Mio g/mol) and/or the content of disintegrant (NaCMC, Starch 1500).

All formulations were analyzed regarding their dissolution profile as well as their tamper-resistance such as solvent extraction rates and particle size distribution after physical manipulation of the pellets.

### Dissolution testing:

The dissolution tests were performed in 600 ml 0.1 N hydrochloric acid. The temperature was set to 37°C and the paddle rotation speed was set to 75 rpm. As a target, a drug release of 80% was set at the specification point of 30 minutes.

The drug release after 15 minutes of all formulations was more than 85% and therefore immediate release characteristics were confirmed for all tested formulations.

### Solvent extraction - intravenous abuse:

The test for the intravenous abuse was performed with intact samples. One sample was extracted in 5 ml of boiling water for 5 minutes. The extract was directly filtered through a cigarette filter and then drew in a syringe using a 21 gauge needle. The weight of the empty syringe and the weight of the syringe filled with the extract were measured. The weight of the extract was calculated assuming a density of the extract as equal to water (ρ = 1.0 g/mL). The result of the assay was calculated with reference to the weight of the extract. For this report the results were calculated to mg API in the syringed extract and reported in percent of the label claim. The test was performed in triplicate.

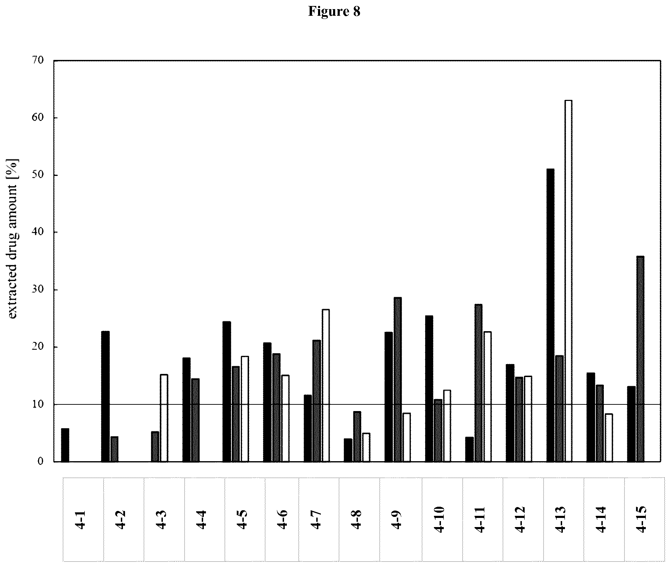

Solvent extraction test revealed that in general, comparative formulations prepared with polyethylene oxide M_{w} 7 Mio. g/mol (4-1 to 4-4) showed lower solvent extraction rates than formulations based on polyethylene oxide M_{w} 1 Mio. g/mol (4-5 to 4-15) as shown in Figure 8. Especially the pellet formulation containing polyethylene oxide M_{w} 7 Mio. g/mol, xanthan gum and croscarmellose sodium (4-1) showed very low extraction rates of less than 5%. Therefore, this comparative pellet formulation was used as a reference to compare with the performance of the subsequent formulations. Beside formulation 4-1, the only formulation showing a solvent extraction rate below 10% for all three repetitions was inventive formulation 4-8. This formulation is based on polyethylene oxide 1 Mio. and contains 35% croscarmellose sodium and 5% hydroxyethylcellulose.

### Physical manipulation of pellets and particle size distribution:

Physical manipulation of the pellets was conducted according to "Chef's Knife Physical Manipulation protocol" prepared by Drug Scan (DS-GR-AD002). Therefore, the knife was sharpened previous to the manipulation of each pellet unit. The pellets were placed on the cutting board and the blade of the knife was placed in the center of the material. The material was cut holding one hand on the handle and the other on the tip of the knife. The pellets were cut for 6 minutes 40 seconds and during this time the material was gathered together if it spread out across the cutting board.

The particle size distribution of the manipulated pellets was analyzed by Camsizer® (Retsch Technology). The pellets were theoretically classified into nine particle size fractions between 0 and 2.8 mm (each fraction has a range of 0.313 mm). For the measurements, 10 g of pellets were used. The cumulative mass distribution (Q3) was calculated, dividing the mass of a specific fraction (x mm) by the total mass of the sample. This value represents the percentage of particles finer than the given particle size (x mm). By doing so, values of Q3 for 50% were calculated, meaning that the certain percentage of the particles are finer than the depicted particle size.

Particle size distribution of manipulated pellets showed significant differences between formulations based on polyethylene oxide M_{w} 7 Mio. g/mol and polyethylene oxide M_{w} 1 Mio. g/mol. Particle sizes of more than 0.5 mm are required to minimize intranasal abuse, which was achieved by all tested manipulated pellet formulations.

The results for the measurement of the particle size distribution x [mm] at Q3 = 50% are compiled in the following table:

| Example | x [mm] | | Example | x [mm] | | Example | x [mm] |
|---|---|---|---|---|---|---|---|
| 4-1 comp. | 0.463 | | 4-6 inv. | 1.555 | | 4-11 comp. | 1.160 |
| 4-2 comp. | 0.467 | | 4-7 comp. | 1.574 | | 4-12 inv. | 2.327 |
| 4-3 comp. | 0.559 | | 4-8 inv. | 2.574 | | 4-13 comp. | 1.673 |
| 4-4 comp. | 0.432 | | 4-9 comp. | 1.943 | | 4-14 comp. | 1.614 |
| 4-5 comp. | 1.827 | | 4-10 comp. | 2.282 | | 4-15 comp. | 3.719 |

The x values in the above table mean that 50 wt.-% of the particles had a particle size below given x value. The greater the given x value the greater the fraction of large particles providing tamper-resistance. Inventive formulations 4-8 and 4-12 provided the best results (i.e. largest x values). In contrast, the comparative formulations containing polyethylene oxide M_{w} 7 Mio. g/mol (4-1 to 4-4) had a significantly greater fraction with smaller particles.

It may therefore be concluded that particle size distribution of formulations containing polyethylene oxide M_{w} 7 Mio. g/mol (4-1 to 4-4) tend to smaller particle sizes than particle size distribution of formulations based on polyethylene oxide M_{w} 1 Mio. g/mol (4-5 to 4-15). Due to the lower molecular weight of the polymer, the pellet matrix is softer and sticky. During physical manipulation of the pellets, the particles tend to agglomerate and will not be distributed within the airflow during particle size analysis. The pellet formulations showing the largest particles were 4-8, 4-12, and 4-15. Pellet formulations of formulations 4-12 and 4-8 were comparable to each other and differed only in the amount of croscarmellose sodium and polyethylene oxide. Even though solvent extraction rates of 4-12 was higher than 10%, this formulation showed low variances in solvent extraction with values of approximately 15%.

## Claims

1. A tamper-resistant pharmaceutical dosage form comprising a multitude of particles which comprise
- a pharmacologically active compound selected from opioids;
- a polyalkylene oxide having a weight average molecular weight within the range of from 200,000 g/mol to 2,000,000 g/mol, and
- a disintegrant;
wherein the pharmacologically active compound is dispersed in a matrix comprising the polyalkylene oxide and the disintegrant;
wherein the content of the disintegrant is more than 20 wt.-%, based on the total weight of the particles; wherein the content of the polyalkylene oxide is at least 25 wt.-%, based on the total weight of the particles; and
wherein the dosage form provides under *in vitro* conditions immediate release of the pharmacologically active compound in accordance with Ph. Eur.

2. The pharmaceutical dosage form according to claim 1, which has a breaking strength of at least 300 N.

3. The pharmaceutical dosage form according to claim 1 or 2, which exhibits resistance against solvent extraction such that when
(i) dispensing the pharmaceutical dosage form that is either intact or has been manually comminuted by means of two spoons in 5 ml of purified water,
(ii) heating the liquid up to its boiling point,
(iii) boiling the liquid in a covered vessel for 5 min without the addition of further purified water,
(iv) drawing up the hot liquid into a syringe, and
(v) determining the amount of the pharmacologically active compound contained in the liquid within the syringe,
the liquid part of the formulation that can be separated from the remainder by means of the syringe is not more than 10 wt.-% of the pharmacologically active compound originally contained in the dosage form.

4. The pharmaceutical dosage form according to any of the preceding claims, wherein the disintegrant is or comprises
(a) a polysaccharide; preferably a polysaccharide mixture obtained from soybeans or sodium alginate;
(b) a starch; preferably standard starch or pregelatinized starch;
(c) a starch derivative; preferably sodium starch glycolate or sodium carboxymethyl starch;
(d) a cellulose derivative; preferably croscarmellose sodium;
(e) an acrylate; preferably carbopol;
(f) a polyvinylpyrrolidone; preferably crospovidone;
(g) a gas releasing substance; preferably sodium bicarbonate; or
(h) a protein or protein derivative; preferably crosslinked casein.

5. The pharmaceutical dosage form according to any of the preceding claims, wherein the content of the disintegrant is at least 25 wt.-%; preferably at least 30 wt.-%; in each case based on the total weight of the particles.

6. The pharmaceutical dosage form according to any of the preceding claims, wherein the content of the disintegrant is within the range of 35±15 wt.-%; preferably 35±10 wt.-%; more preferably 35±5.0 wt.-%; in each case based on the total weight of the particles.

7. The pharmaceutical dosage form according to any of the preceding claims, wherein the polyalkylene oxide has a weight average molecular weight within the range of 1,000,000±750,000 g/mol; preferably 1,000,000±500,000 g/mol; more preferably 1,000,000±250,000 g/mol.

8. The pharmaceutical dosage form according to any of the preceding claims, wherein the content of the polyalkylene oxide is at least 40 wt.-%; preferably at least 45 wt.-%; more preferably at least 50 wt.-%; in each case based on the total weight of the particles.

9. The pharmaceutical dosage form according to any of the preceding claims which provides a release profile such that under *in vitro* conditions in 600 ml 0.1 M HCl (pH 1) at 75 rpm after 30 min at least 90 wt.-% of the pharmacologically active ingredient that was originally contained in the dosage form have been released.

10. The pharmaceutical dosage form according to any of the preceding claims, which additionally comprises
(I) a gelling agent; preferably a polysaccharide; more preferably a cellulose ether; still more preferably a cellulose ether selected from the group consisting of methylcellulose, hydroxyethylcellulose, hydroxypropylcellulose, and hydroxypropylmethylcellulose; in particular hydroxyethylcellulose; and/or
(II) a plasticizer; preferably selected from the group consisting of is polyalkylene glycols, triacetin, fatty acids, fatty acid esters, waxes, and microcrystalline waxes; more preferably a polyethylene glycol; in particular a polyethylene glycol having a weight average molecular weight within the range of from 4000 to 10,000 g/mol, preferably from 5400 to 6600 g/mol;
(III) an acid; preferably an organic carboxylic acid; more preferably a dicarboxylic acid or a tricarboxylic acid; still more preferably a hydroxy dicarboxylic acid or a hydroxy tricarboxylic acid; in particular citric acid; and/or
(IV) an antioxidant; preferably selected from the group consisting of ascorbic acid, salts of ascorbic acid, butylated hydroxyanisole (BHA), butylated hydroxytoluene (BHT), monothioglycerol, phosphorous acid, vitamin E and the derivatives thereof, coniferyl benzoate, nordihydroguajaretic acid, gallus acid esters, sodium bisulfite; more preferably butylated hydroxytoluene, butylated hydroxyanisole or alpha-tocopherol; in particular alpha-tocopherol.

11. The pharmaceutical dosage form according to claim 10, wherein
(I) the content of the gelling agent is within the range of 7.5±7.0 wt.-%, preferably 7.5±5.0 wt.-%, in each case based on the total weight of the particles; and/or
(II) the content of the plasticizer is within the range of 2.0±1.5 wt.-%, preferably 2.0±1.0 wt.-%, in each case based on the total weight of the particles; and/or
(III) the content of the acid is within the range of 0.8±0.7 wt.-%, preferably 0.8±0.5 wt.-%, in each case based on the total weight of the particles; and/or
(IV) the content of the antioxidant is within the range of 0.20±0.15 wt.-%, preferably 0.20±0.10 wt.-%, in each case on the total weight of the particles.

12. The pharmaceutical dosage form according to any of the preceding claims, wherein the opioid is selected from the group consisting of oxycodone, hydrocodone, oxymorphone, hydromorphone, morphine, tramadol, tapentadol, and the physiologically acceptable salts thereof; preferably oxycodone or a physiologically acceptable salt thereof; in particular oxycodone hydrochloride.

13. The pharmaceutical dosage form according to any of the preceding claims, wherein the content of the pharmacologically active compound is at least 3.0 wt.-%, based on the total weight of the particles.

14. The pharmaceutical dosage form according to any of the preceding claims, wherein the particles
- are hot-melt extruded; and/or
- have an individual weight of at lost 5.0 mg, more preferably at most 4.5 mg, still more preferably at most 4.0 mg, yet more preferably at most 3.5 mg, even more preferably at most 3.0 mg, most preferably at most 2.5 mg, and in particular at most 2.0 mg.

15. The pharmaceutical dosage form according to any of the preceding claims, which is a tablet or capsule.
